# EUROPEAN PATENT SPECIFICATION

(11) **EP 3 153 515 B1**
(45) Date of publication and mention of the grant of the patent: **12.08.2020**
(21) Application number: 14893934.1
(22) Date of filing: 03.12.2014
(51) Int. Cl.: C07D 491/056, C07D 519/00, C07D 403/14, C07D 409/14, C07D 401/14, C07D 413/14, C07D 495/04, C07D 491/113, A61K 31/4178, A61K 31/498, A61K 31/4439, A61K 31/5377, A61K 31/4184, A61P 31/14, C07D 491/048, C07D 498/04

(54) **COMPOUNDS FOR INHIBITING HEPATITIS C VIRUS, PHARMACEUTICAL COMPOSITIONS AND USES THEREOF**
VERBINDUNGEN ZUR HEMMUNG DES HEPATITIS-C-VIRUS, PHARMAZEUTISCHE ZUSAMMENSETZUNGEN UND VERWENDUNGEN DAVON
COMPOSÉS POUR INHIBER LE VIRUS DE L'HÉPATITE C, COMPOSITIONS PHARMACEUTIQUES ET LEURS UTILISATIONS

(30) Priority: 06.06.2014 WO PCT/CN2014/079386
(43) Date of publication of application: 12.04.2017
(73) Proprietor: AB Pharma Ltd., Minhang District, Shanghai 201108 (CN)
(72) Inventor: ZHAN, Zheng-yun James, Shanghai (CN)
(74) Representative: Biggi, Cristina
(86) International application number: PCT/CN2014/092902
(87) International publication number: WO 2015/184753

(56) References cited:
- WO-A1-2010/062821
- WO-A2-2011/156543
- WO-A2-2012/068234
- CN-A- 101 558 059
- CN-A- 102 596 936
- CN-A- 104 230 946
- D. BHATTACHARYA ET AL: "Pharmacological disruption of hepatitis C NS5A protein intra- and intermolecular conformations", JOURNAL OF GENERAL VIROLOGY., vol. 95, no. Pt_2, 30 August 2013 (2013-08-30), pages 363-372, XP055485273, GB ISSN: 0022-1317, DOI: 10.1099/vir.0.054569-0

## Description

### Technical Field

This invention relates to HCV inhibitory chemical compounds, pharmaceutical compositions and their applications.

### Background Art

Hepatitis C virus (HCV) is the major pathogen that causes non-A non-B hepatitis. HCV infection may result in chronic liver diseases, such as hepatic cirrhosis and hepatic carcinoma. Since it is estimated that 3-5% of the world population have been infected with HCV, HCV infection is deemed an urgent human health problem (Lavanchy et al, J. Viral Hepatitis, 1999, 6, 35-47; Alter et al, J. Hepatology 1999, 31, 88-91; Alberti et al, J. Hepatology 1999, 31, 17-24.).

HCV is a single strand RNA virus in the Flaviviridae family. It is comprised of a nucleocapsid protein (C), envelope proteins (E1 and E2), and some non-structural proteins (NS1, NS2, NS3, NS4a, NS5a and NS5b). A number of enzymes and protein domains of the virus can be the target of new drugs. NS5a of HCV is among the latest and most promising target. NS5A structurally consists of 3 independent characteristic fragments and the functions of these fragments are still under investigation. At present, researches on the application of numerous NS5A inhibitors have been carried out by pharmaceutical companies rapidly and extensively.

It is found a group of chemical compounds can effectively inhibit the replication of HCV RNA by targeting at NS5A. Biological chemistry studies indicate that NS5A molecular inhibitors can directly bind to NS5A polypeptide. This has been proven by the drug-resistant mutant in fragment I of NS5A polypeptide chain.

WO2010/062821 and D. Bhattacharya et al.: "Pharmacological disruption of hepatitis C NS5A protein intra- and intermolecular conformations", J. General Virology, vol. 95, No. Pt_2, 30 August 2013, p.363-372 disclose HCV inhibitors structurally similar to the compounds Ia described below, differeing for the presence of double bonds in specific position and in certain terminal groups.

NS5A protein is a multifunctional protein in the forms of phosphorylated(p56) and hyperphosphorylated(p58) exposed groups. NS5A phosphorylation is involved a multiple aspects of the regulation of HCV replication. Even though the exact inhibitory mechanism of these chemical compounds is still not clear, it has been confirmed that they can inhibit the hyperphosphorylation of NS5A. NS5A inhibitors break the hyperphosphorylation without affecting the fundamental phosphorylation at C-terminal region of NS5A. The activity of these inhibitors is independent of the characteristic fragments II and III of NS5A and totally different from that of inhibitors that block the hyperphorylated kinases of NS5A; their activity is consistent with that of NS5A inhibitors whose binding site is the N-terminal region.

Moreover, NS5A inhibitors can promote the accumulation of intermediate polyproteins, suggesting that the binding of these inhibitors with NS5A has priority over polyprotein complex. Experiments demonstrated that NS5A inhibitors did change the subcellular localization, separation mode and biochemical fractionation result of NS5A proteins. NS5A inhibitors may affect the expression and regulation of HCV in many aspects. These findings may be helpful to the explanation of the special efficacy of these HCV replication complex inhibitors. Since the year 2000, many European and American research institutes and pharmaceutical companies have been extensively and thoroughly developing a variety of micromolecular HCV NS5A inhibitors, but so far non of these NS5A inhibitors has been approved to be marketed. All NS5A inhibitors currently in clinical stage suffer from a variety of shortcomings such as side effects of different degrees, therefore it is necessary to further develop new NS5A inhibitor of better therapeutic effect and lower side effects.

### Summary of the invention

This invention, aiming to solve the existing technical problems and overcome the defect of the lack of effective HCV inhibiting drugs, proposes compounds, pharmaceutical compositions completely difference from existing ones and applications thereof. Capable of effectively inhibiting HCV NS5A, the compounds of this invention are used to prepare pharmaceutical drugs for the prevention and/or treatment of HCV-NS5A infection and showing a great market prospects.

The present inventors have, through extensive R & D, designed and synthesized a group of chemical compounds which, being novel HCV-NS5A protein inhibitors, can be used to effectively inhibit HCV NS5A and treat HCV infections. It offers more and better options in the further optimization and clinical application of linear chain polypeptides polycyclic compounds for effective inhibition of HCV by introducing a variety of linear chain polypeptides based structures and structural optimization of the linear chain polypeptides polycyclic compounds for enhanced biological activities of the linear chain polypeptides heterocyclic compounds in inhibiting HCV NS5A.

This invention relates to compounds Ia-70, Ia-71, Ia-113 and Ia-115 which are representatives of Formula Ia their stereoisomers, tautomers, pharmaceutically acceptable salts, or their isotopic substitutions in which the hydrogen, oxygen or nitrogen atom is replaced by its corresponding isotope,

| | |
|---|---|
| **Ia-70** | 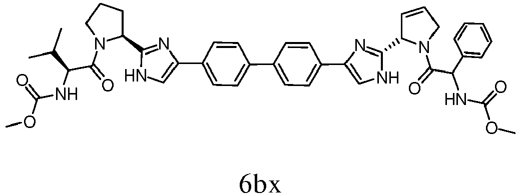 |
| **Ia-71** | 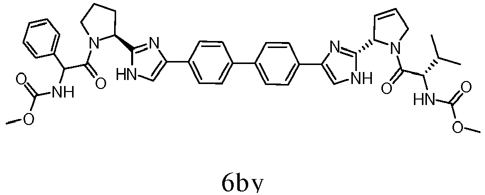 |
| **Ia-113** | 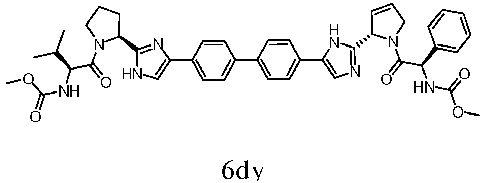 |
| **Ia-115** | 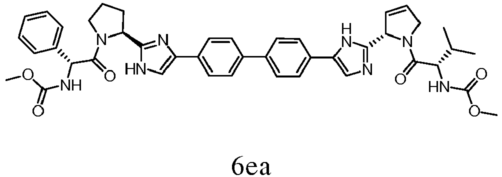 |

The invention is defined by the claims. Any subject-matter falling outside the scope of the claims is provided for information purposes only. Any references in the description to methods of treatment refer to the compounds, pharmaceutical compositions and medicaments of the present invention for use in a method for treatment of the human or animal body by therapy.

In this invention, heterocyclic compounds for inhibiting HCV are designed and synthesized, their inhibitory action against HCV activity further studied, the relation between novel heterocyclic compounds of various structures and their inhibitory action against HCV activity thoroughly explorered, and the novel heterocyclic compounds and their preparation method are further developed and optimized for effective treatment of HCV infection.

The key to abbreviations of the chemical reagents and solvents used in the systhesis of heterocyclic compounds in this invention is summarized in the explanations of apparatuses and raw materials section of embodiments.

It should be understood in the art that knowledge of the structure of the compounds of this invention can be used in conjunction with well-known methods, such as chemically synthetic methods or plant extraction methods, and well recognized raw materials, to get the compounds of this invention, and these methods have been included in this invention.

The key innovation point of this invention lies in the preparation of compound 6 (see structural formula series 3) through coupling reaction or amidation of intermediate **4 or 5,** obtained by by deprotection (removal of PG and or PG1 group) of intermediate **3**, which is synthesized by amidation or coupling reaction of compound **SM1** of the structure formula series 1 and compound **SM2** of the structure formula series 2 as shown below. Methods for their preparation are shown in the following reaction schemes 1-3; wherein the "X" in compounds SM1 and SM3 in reaction schemes 1 and 2 is bromine (Br), "Y" in compounds SM2 and SM4 is boric acid or a borate.

This invention also discloses the preparation of the described compounds as represented by Formula **Ia**, their stereoisomers, tautomers, esterified or amidated prodrugs, pharmaceutically acceptable salts, by any of the following methods (see embodiments for specific synthetic methods and reaction conditions):
Method1: Compound SM1 and compound SM2 were carried out by Suzuki catalytic coupling reaction in organic solvent to get compound 3 (**IIa**); 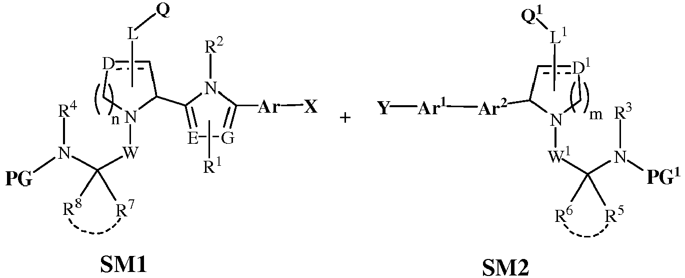 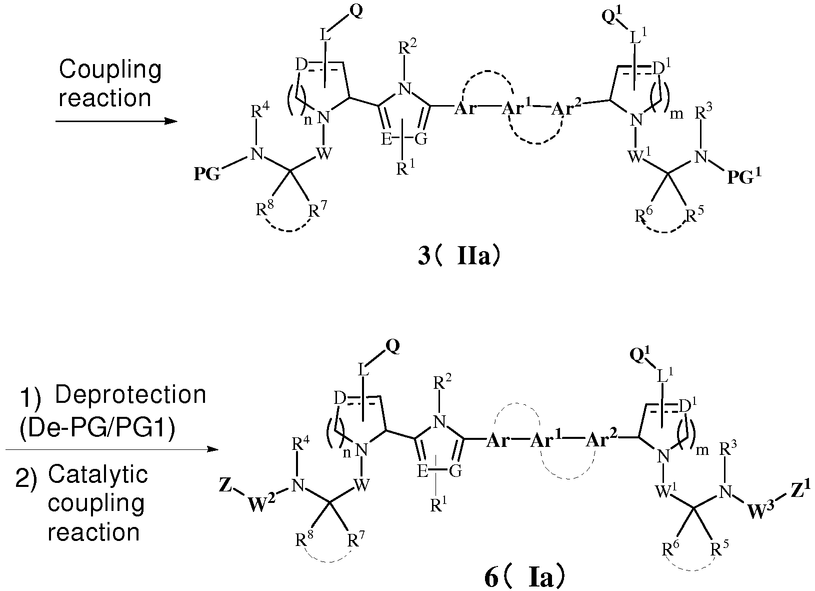
Method 2: Compound SM3 and compound SM4 were carried out by a catalytic coupling reaction to get compound **6** (**Ia**); 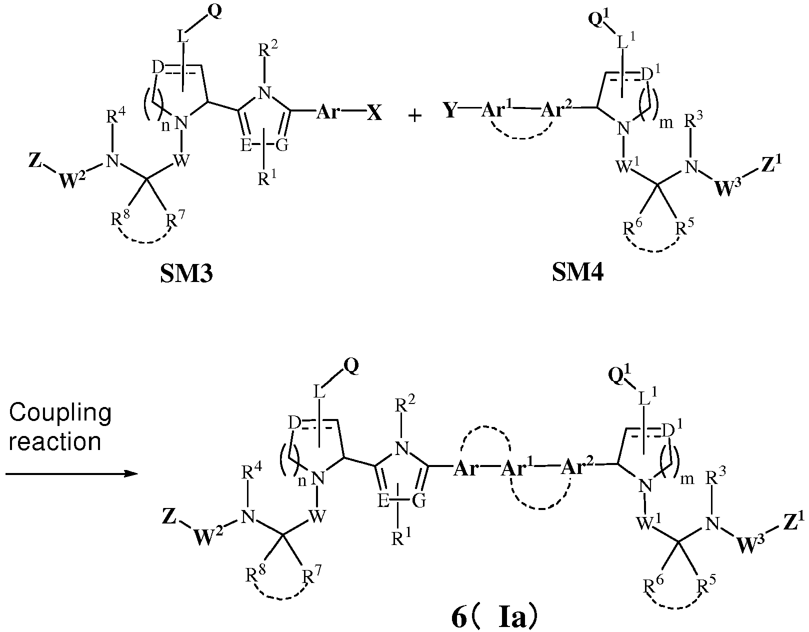
   In the following examples, heterocyclic functional group containing compounds **SM3** (**SM-3a to SM-3cw**) of structural formula series 1 and heterocyclic functional group containing compounds **SM4** (**SM-4a to SM-4bw**) of structural formula series 2 were carried out by a catalytic coupling reaction (see reaction scheme 3) by a composition of chemical preparation techniques to synthesize a series of novel compounds **6** of formula **Ia and Ib** (**6a-6ep** and **6fa-6gq**, refer to structural formula series 3 as shown below for more details).
Method 3:
   Compounds **6fa-6gq (Ib)** are synthesized by the catalytic coupling reaction of compounds SM3 and compounds SM4:

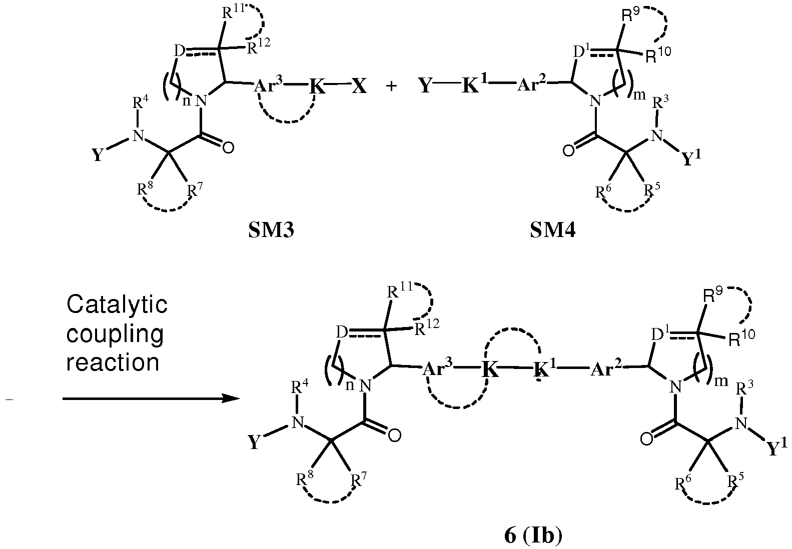

In the following examples, heterocyclic functional group containing compounds **SM3** (**SM-3a to SM-3cw**) of structural formula series **1** and heterocyclic functional group containing compounds **SM4** (**SM-4a** to **SM-4bw**) of structural formula series **2** were carried out by a catalytic coupling reaction (see reaction scheme 3) to synthesize a series of novel compounds **6a-6gq** of formulae **Ia-Ib** (see structural formula series3).

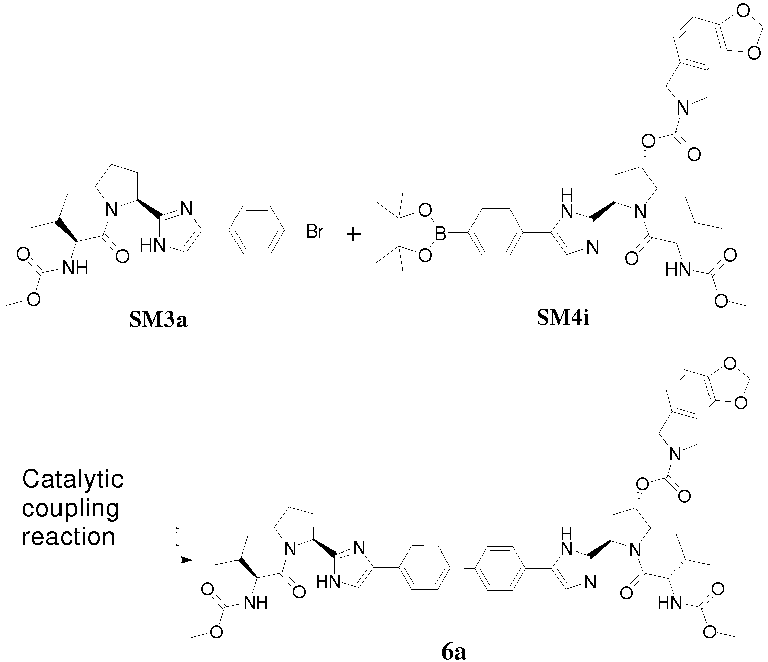

Structural formula series **1** and **2** correspond to the material compounds **SM3** and **SM4**, respectively. Both are required for the synthesis of target compound **Ia** of this invention, and their structural formulae are as follows:
Material compounds **SM3** (**SM-3a to SM-3cw**) of structural formula series **1**:

| No. | **Structural formula of material compounds SM3** |
|---|---|
| 1 | 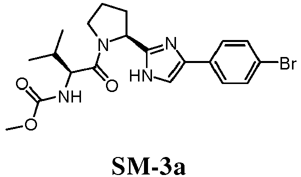 |
| 2 | 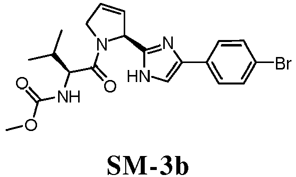 |
| 3 | 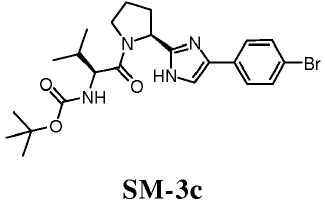 |
| 4 | 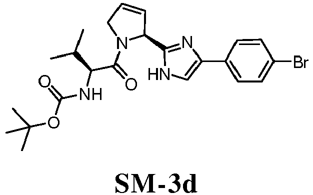 |
| 5 | 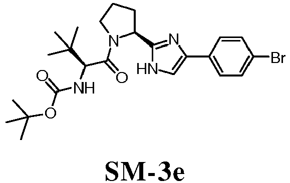 |
| 6 | 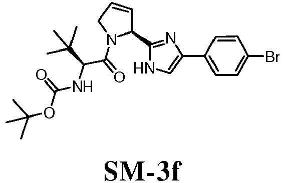 |
| 7 | 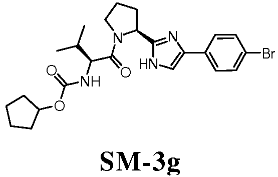 |
| 8 | 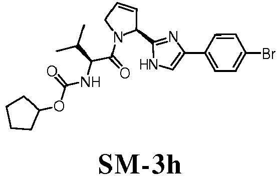 |
| 9 | 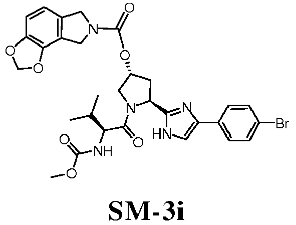 |
| 10 | 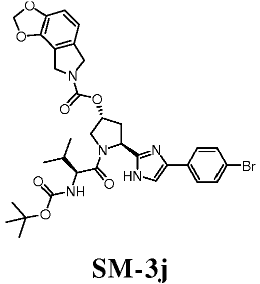 |
| 11 | 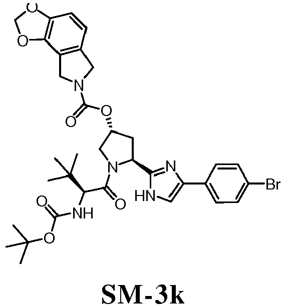 |
| 12 | 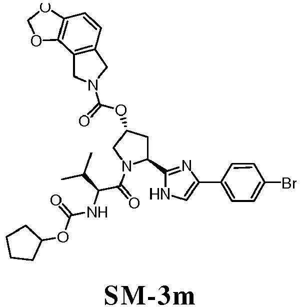 |
| 13 | 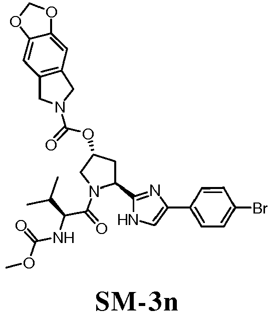 |
| 14 | 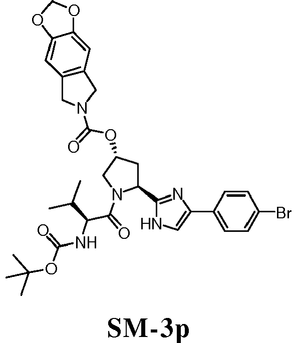 |
| 15 | 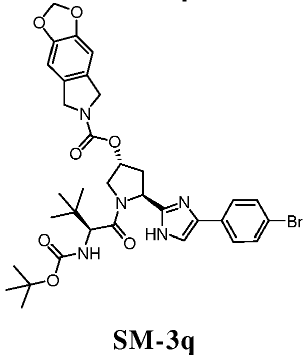 |
| 16 | 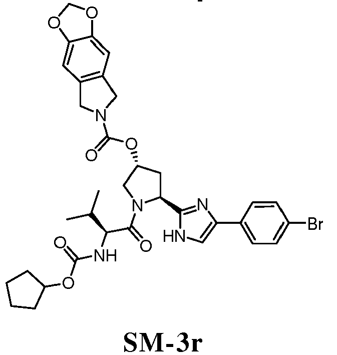 |
| 17 | 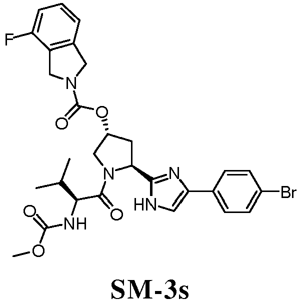 |
| 18 | 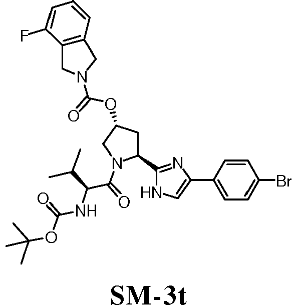 |
| 19 | 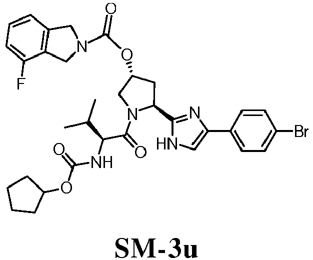 |
| 20 | 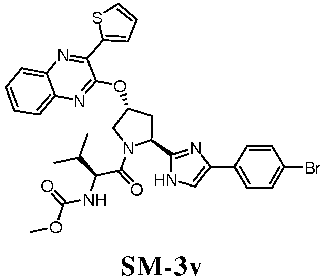 |
| 21 | 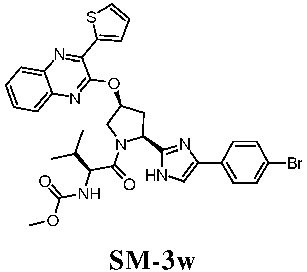 |
| 22 | 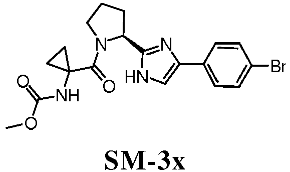 |
| 23 | 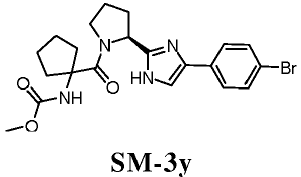 |
| 24 | 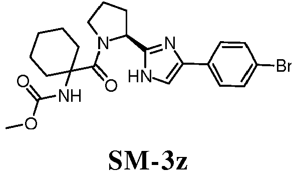 |
| 25 | 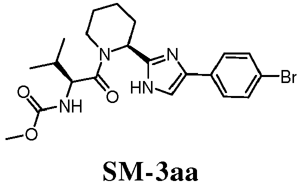 |
| 26 | 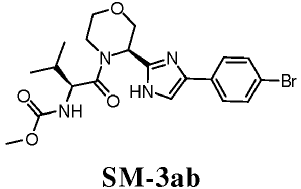 |
| 27 | 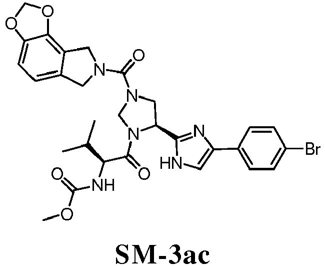 |
| 28 | 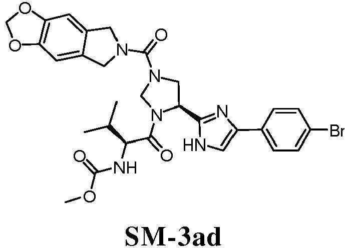 |
| 29 | 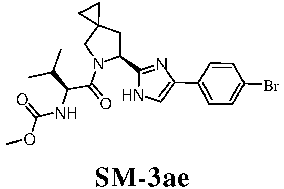 |
| 30 | 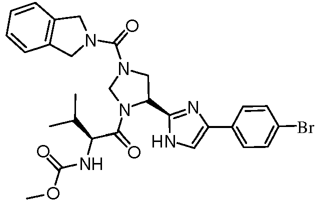 |
| | 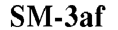 |
| 31 | 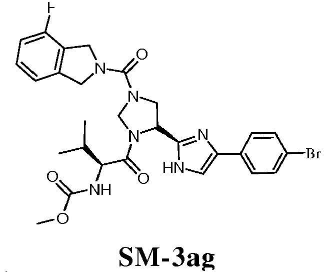 |
| 32 | 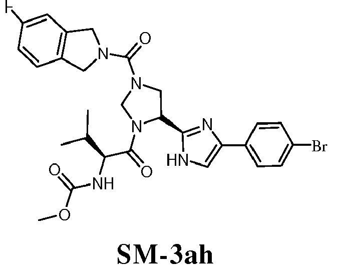 |
| 33 | 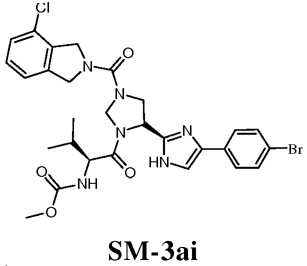 |
| 34 | 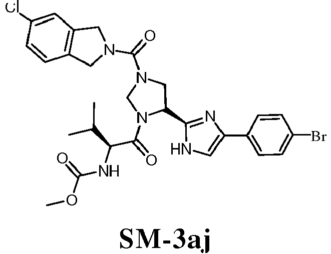 |
| 35 | 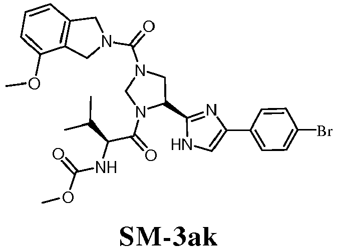 |
| 36 | 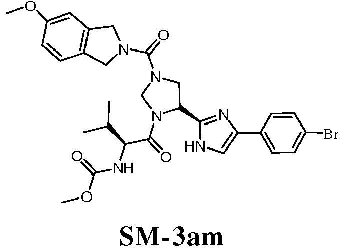 |
| 37 | 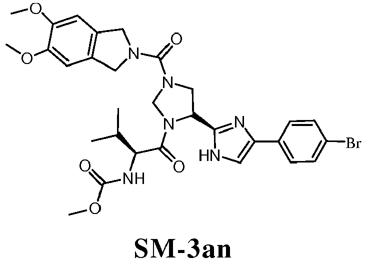 |
| 38 | 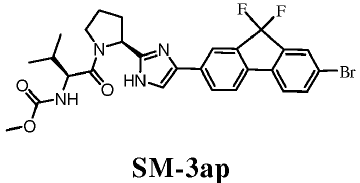 |
| 39 | 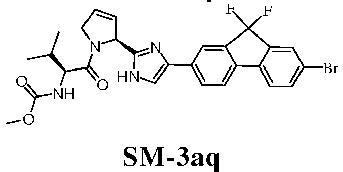 |
| 40 | 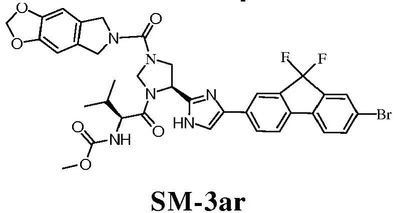 |
| 41 | 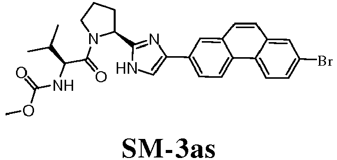 |
| 42 | 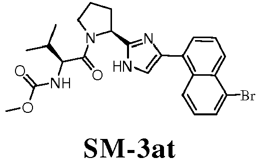 |
| 43 | 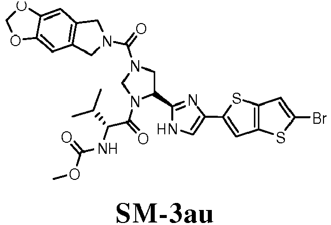 |
| 44 | 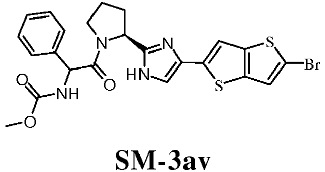 |
| 45 | 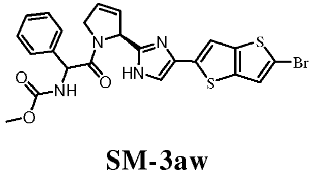 |
| 46 | 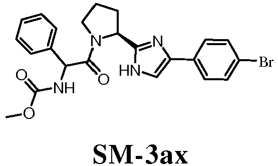 |
| 47 | 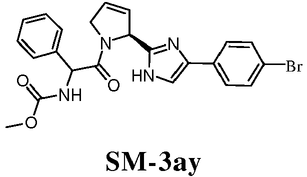 |
| 48 | 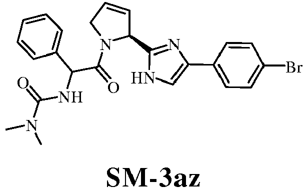 |
| 49 | 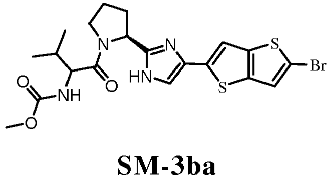 |
| 50 | 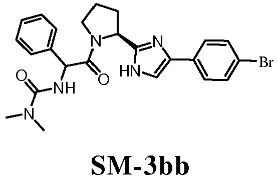 |
| 51 | 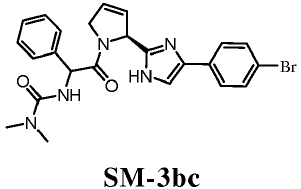 |
| 52 | 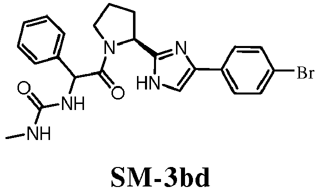 |
| 53 | 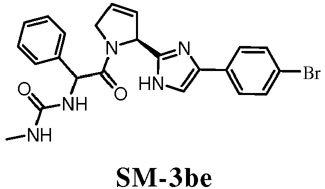 |
| 54 | 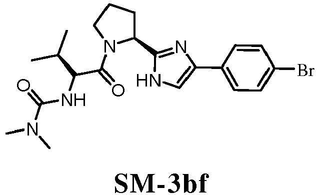 |
| 55 | 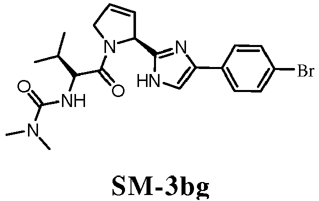 |
| 56 | 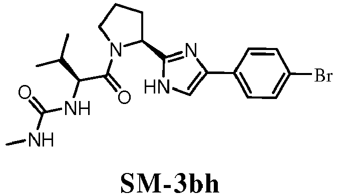 |
| 57 | 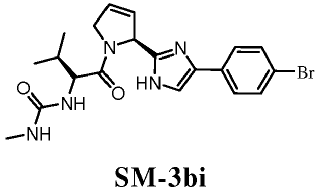 |
| 58 | 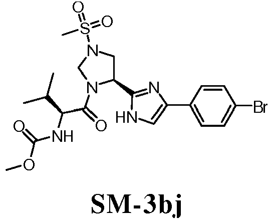 |
| 59 | 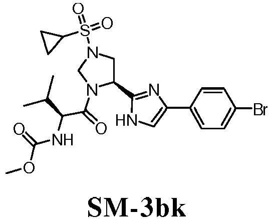 |
| 60 | 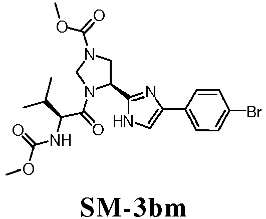 |
| 61 | 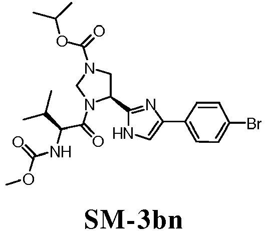 |
| 62 | 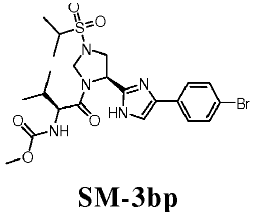 |
| 63 | 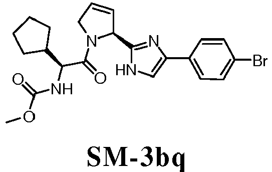 |
| 64 | 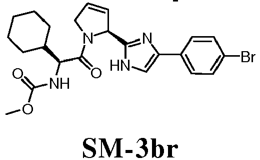 |
| 65 | 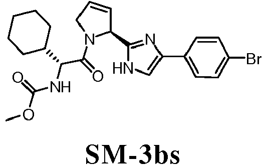 |
| 66 | 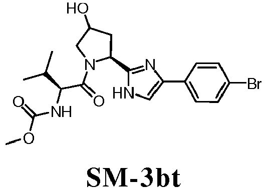 |
| 67 | 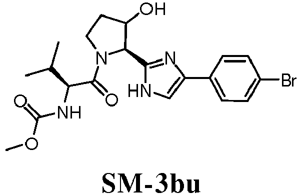 |
| 68 | 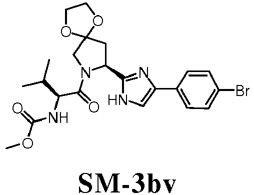 |
| 69 | 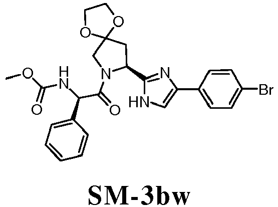 |
| 70 | 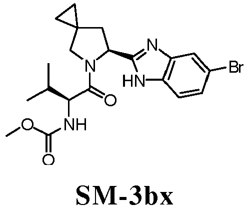 |
| 71 | 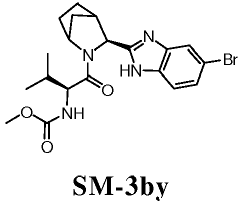 |
| 72 | 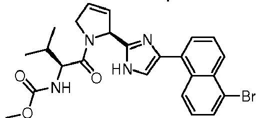 |
| | 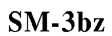 |
| 73 | 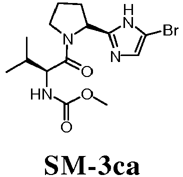 |
| 74 | 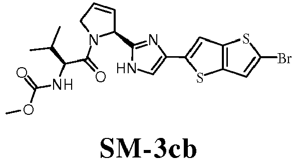 |
| 75 | 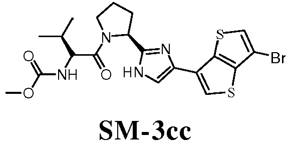 |
| 76 | 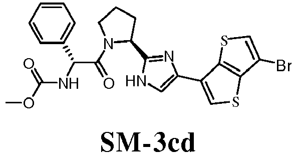 |
| 77 | 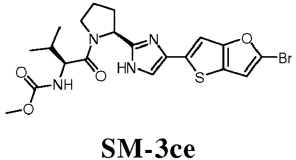 |
| 78 | 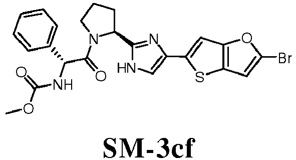 |
| 79 | 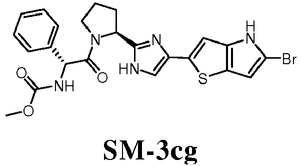 |
| 80 | 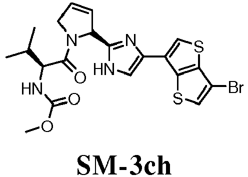 |
| 81 | 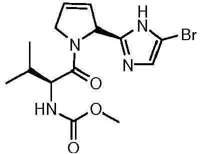 |
| | 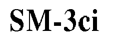 |
| 82 | 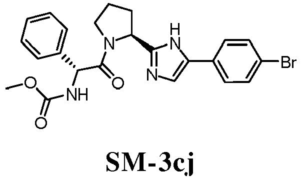 |
| 83 | 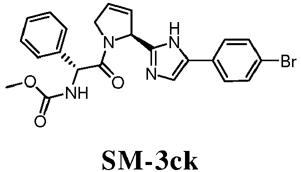 |
| 84 | 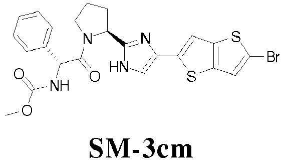 |
| 85 | 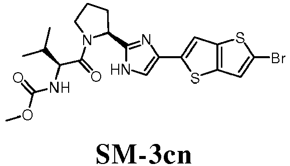 |
| 86 | 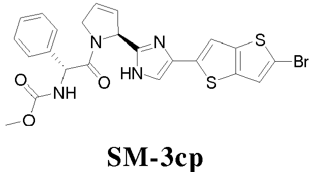 |
| 87 | 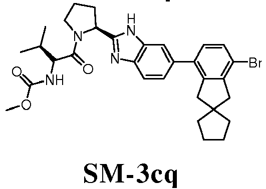 |
| 88 | 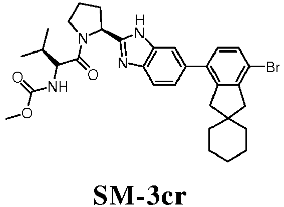 |
| 89 | 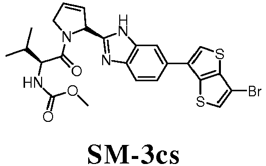 |
| 90 | 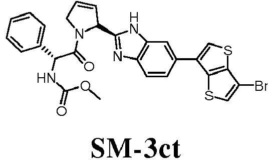 |
| 91 | 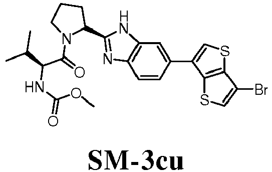 |
| 92 | 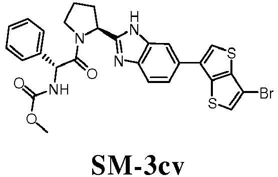 |
| 93 | 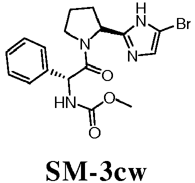 |

The following structural formula series **2** is a concrete example of the materials **SM4** (**SM-4a to SM-4bw**) for synthesis of key structure of compounds of this invention, their structure formula is as shown below:
Material compounds **SM4** (**SM-4a to SM-4bw**) of structural formula series **2**:

| **NO.** | **Structural formula of material compounds SM4** |
|---|---|
| 1 | 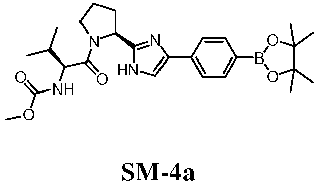 |
| 2 | 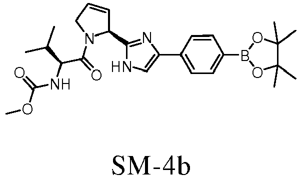 |
| 3 | 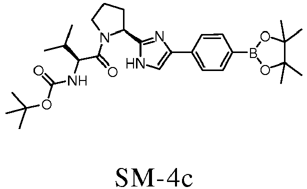 |
| 4 | 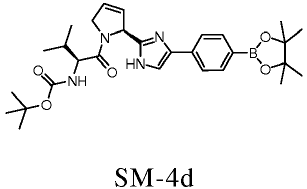 |
| 5 | 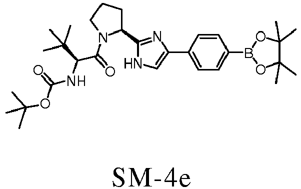 |
| 6 | 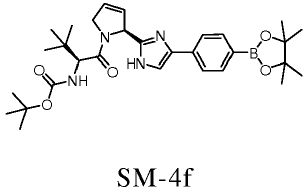 |
| 7 | 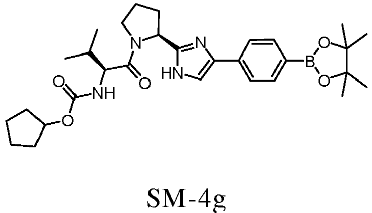 |
| 8 | 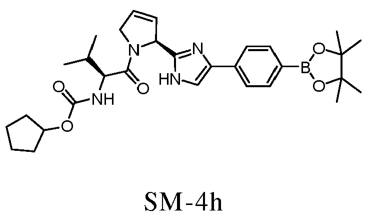 |
| 9 | 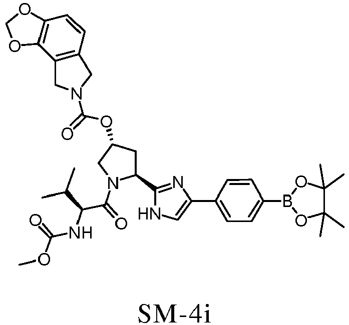 |
| 10 | 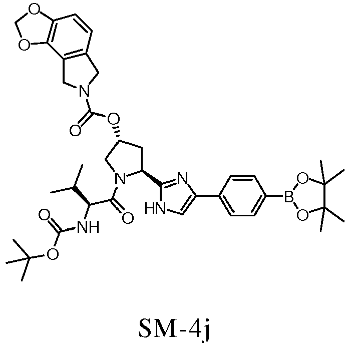 |
| 11 | 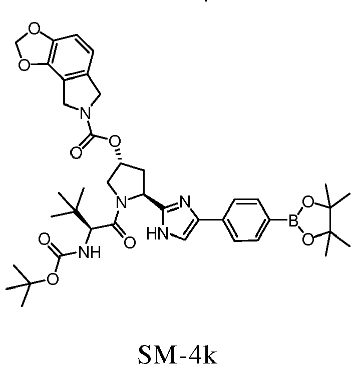 |
| 12 | 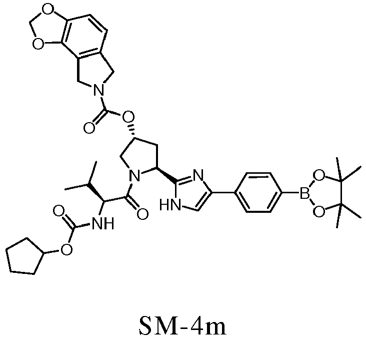 |
| 13 | 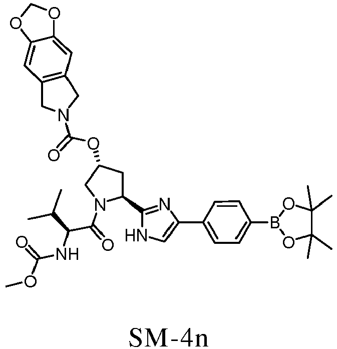 |
| 14 | 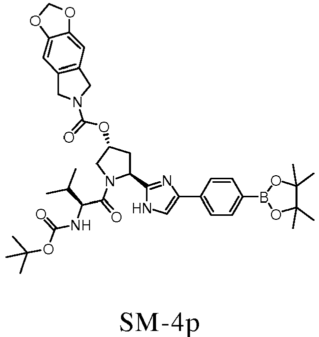 |
| 15 | 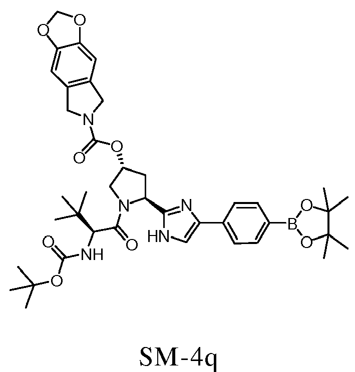 |
| 16 | 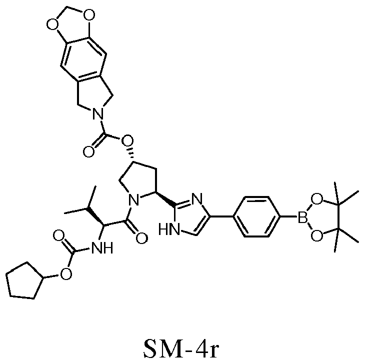 |
| 17 | 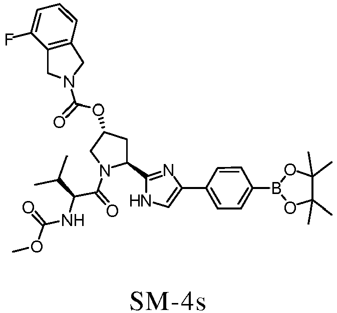 |
| 18 | 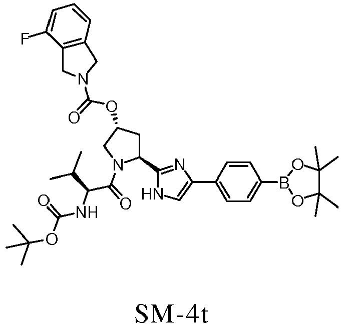 |
| 19 | 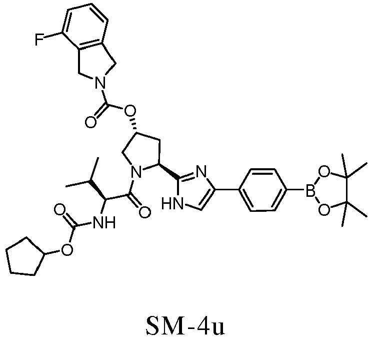 |
| 20 | 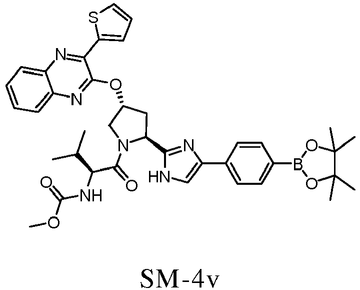 |
| 21 | 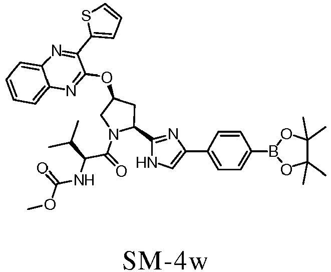 |
| 22 | 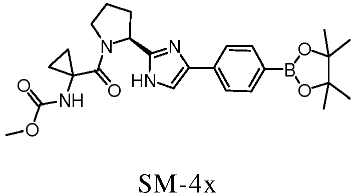 |
| 23 | 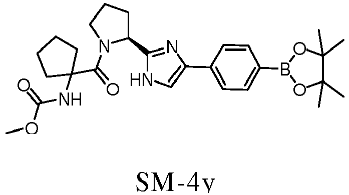 |
| 24 | 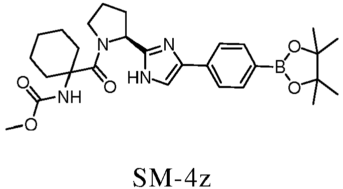 |
| 25 | 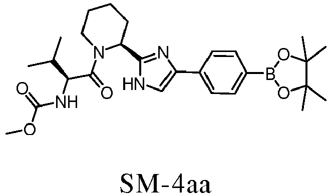 |
| 26 | 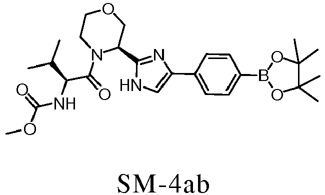 |
| 27 | 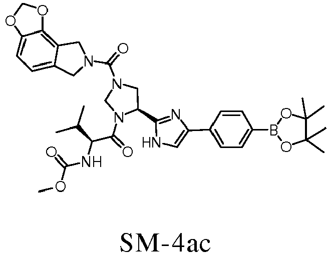 |
| 28 | 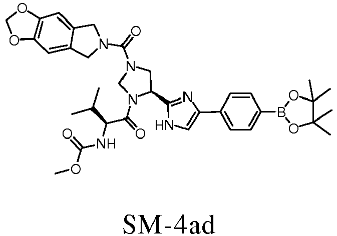 |
| 29 | 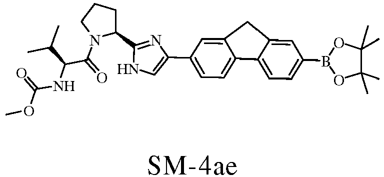 |
| 30 | 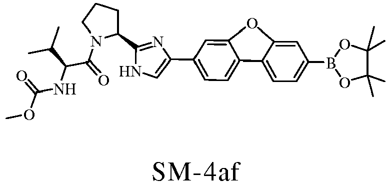 |
| 31 | 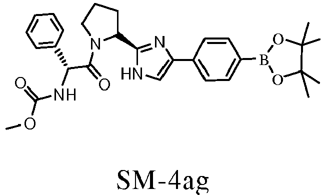 |
| 32 | 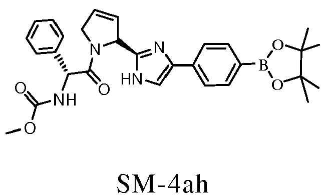 |
| 33 | 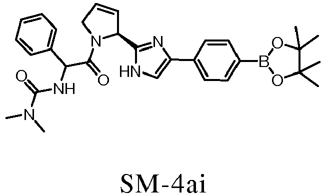 |
| 34 | 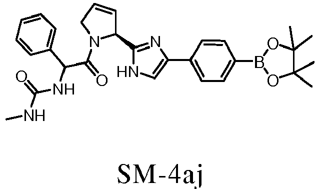 |
| 35 | 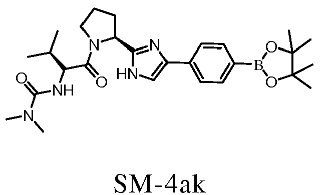 |
| 36 | 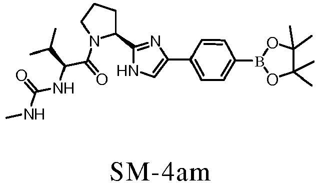 |
| 37 | 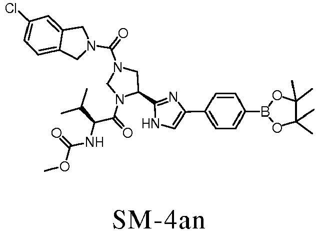 |
| 38 | 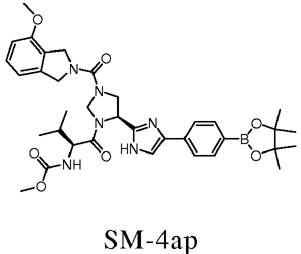 |
| 39 | 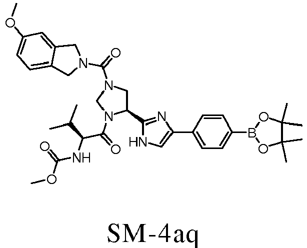 |
| 40 | 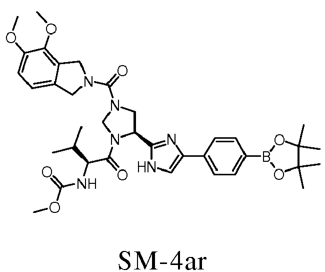 |
| 41 | 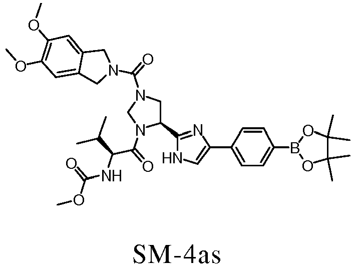 |
| 42 | 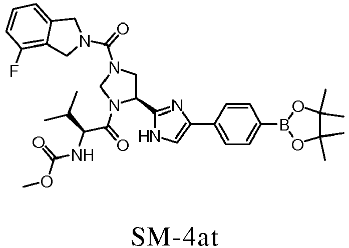 |
| 43 | 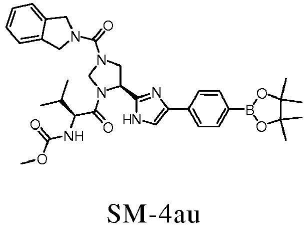 |
| 44 | 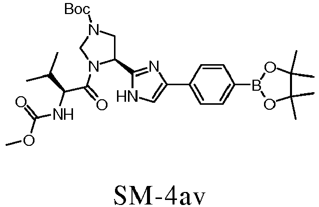 |
| 45 | 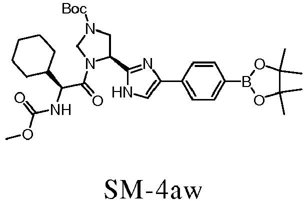 |
| 46 | 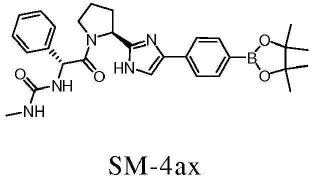 |
| 47 | 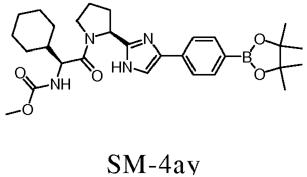 |
| 48 | 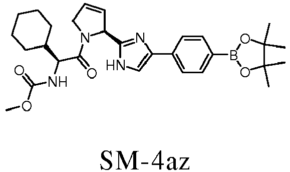 |
| 49 | 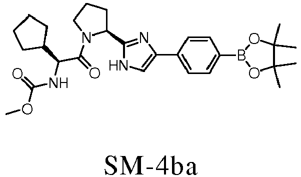 |
| 50 | 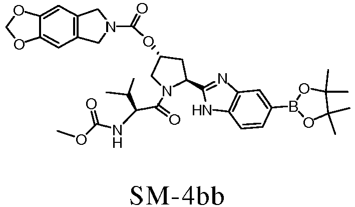 |
| 51 | 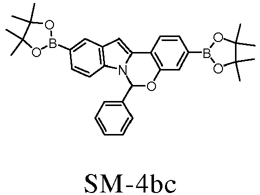 |
| 52 | 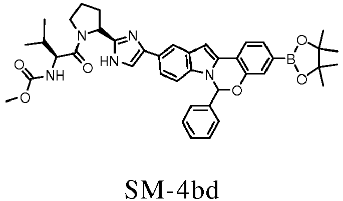 |
| 53 | 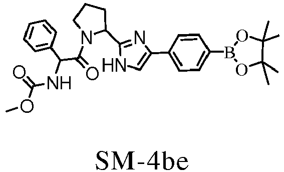 |
| 54 | 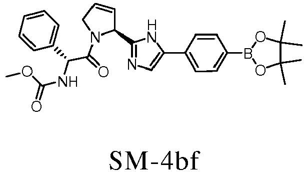 |
| 55 | 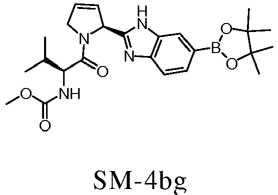 |
| 56 | 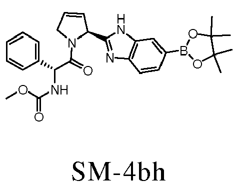 |
| 57 | 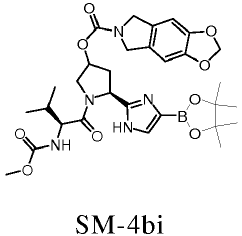 |
| 58 | 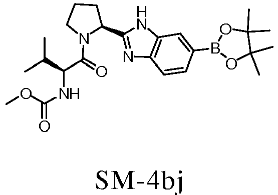 |
| 59 | 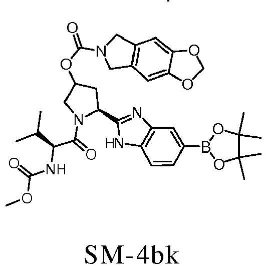 |
| 60 | 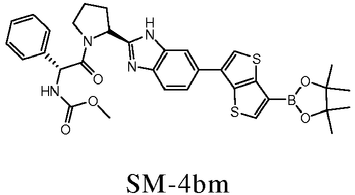 |
| 61 | 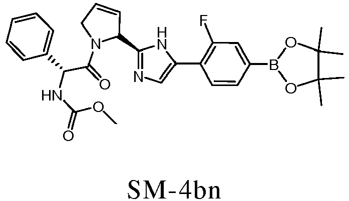 |
| 62 | 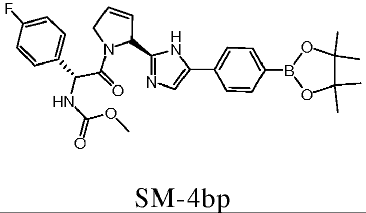 |
| 63 | 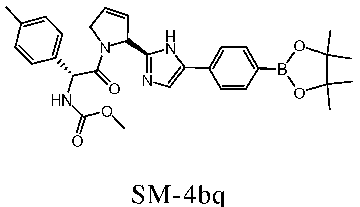 |
| 64 | 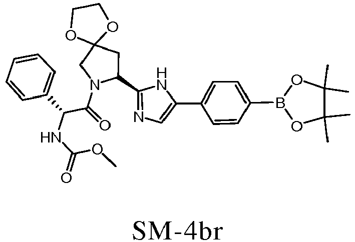 |
| 65 | 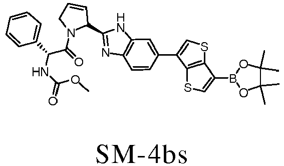 |
| 66 | 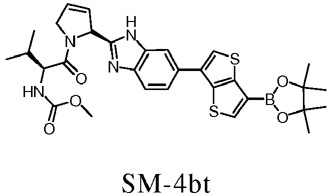 |
| 67 | 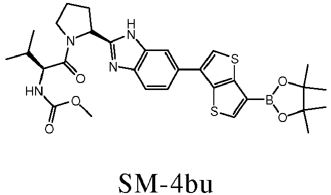 |
| 68 | 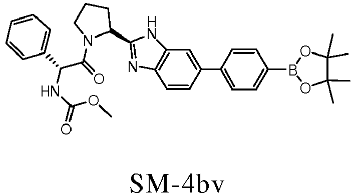 |
| 69 | 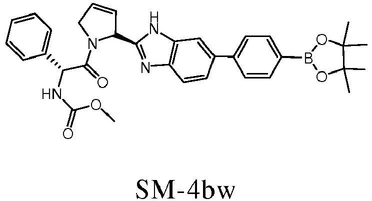 |

The following are concrete examples of target compounds **6a-6ep** (**Ia**) and target compounds **6fa-6gq** (**Ib**) of structural formula series 3 synthesized in accordance with the above-mentioned reaction scheme 3.
Below are compounds **6a-6ep** as represented by Formula **Ia**:

| NO. | Compounds as represented by Formula **Ia** |
|---|---|
| **Ia-1** | 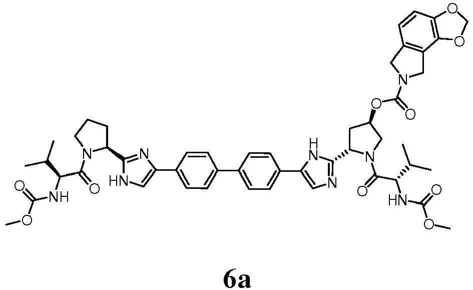 |
| **Ia-2** | 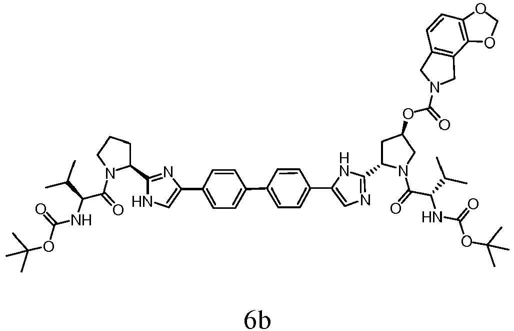 |
| **Ia-3** | 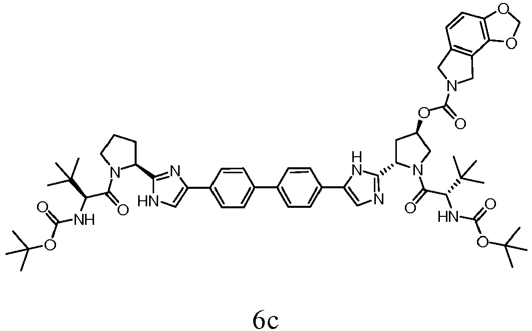 |
| **Ia-4** | 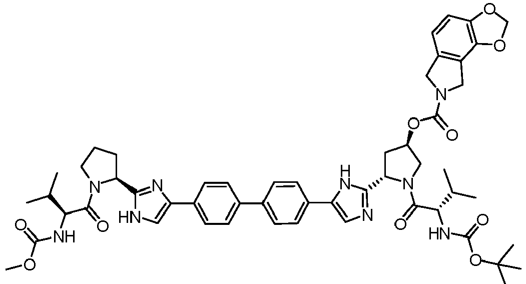 |
| | 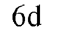 |
| **Ia-5** | 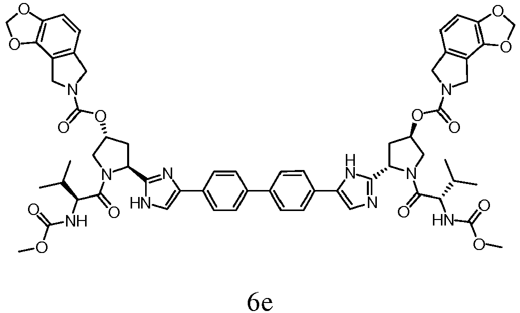 |
| **Ia-6** | 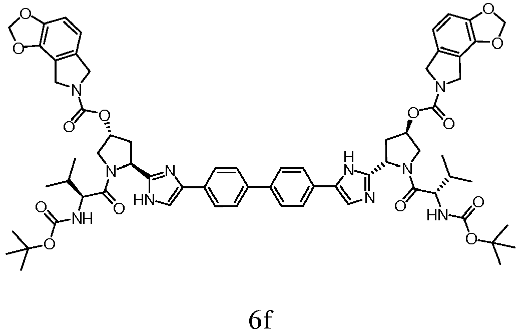 |
| **Ia-7** | 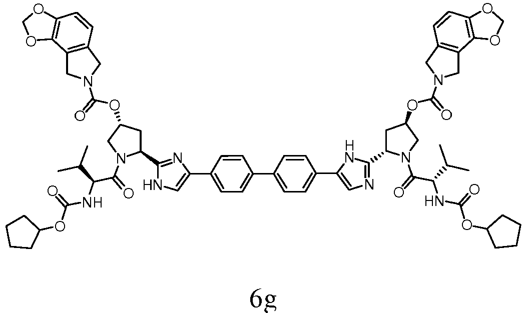 |
| **Ia-8** | 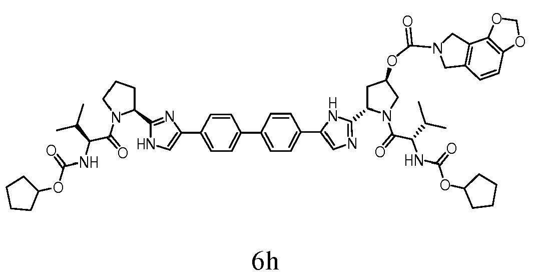 |
| **Ia-9** | 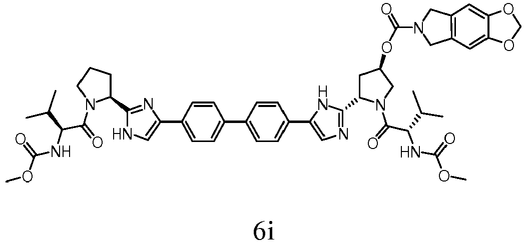 |
| **Ia-10** | 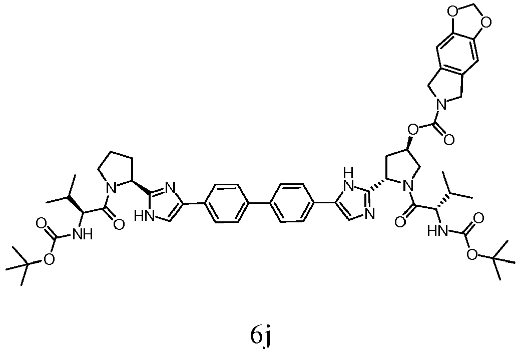 |
| **Ia-11** | 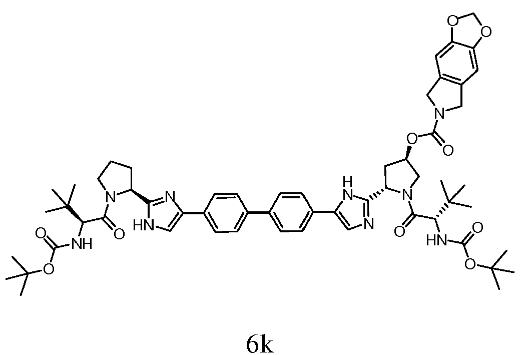 |
| **Ia-12** | 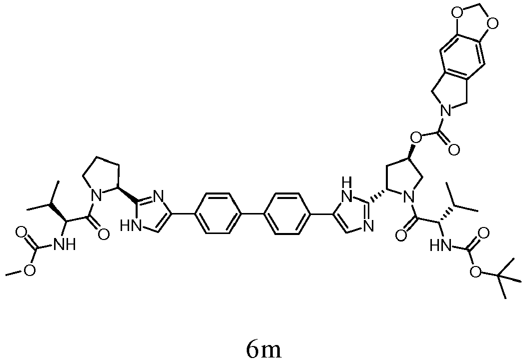 |
| **Ia-13** | 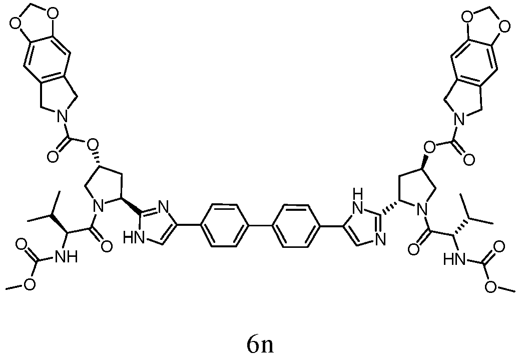 |
| **Ia-14** | 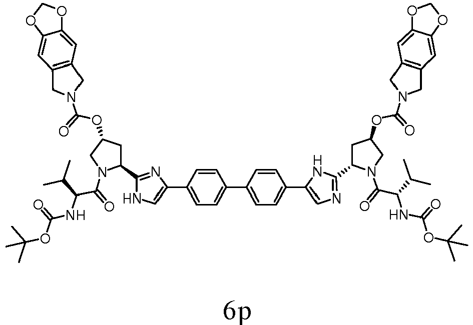 |
| **Ia-15** | 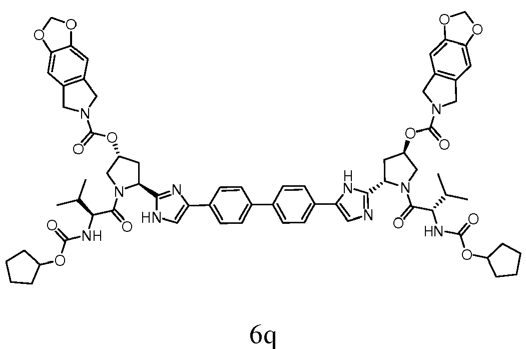 |
| **Ia-16** | 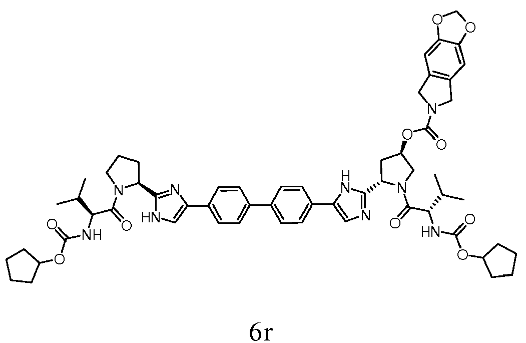 |
| **Ia-17** | 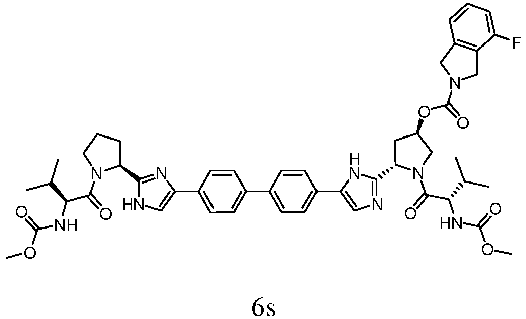 |
| **Ia-18** | 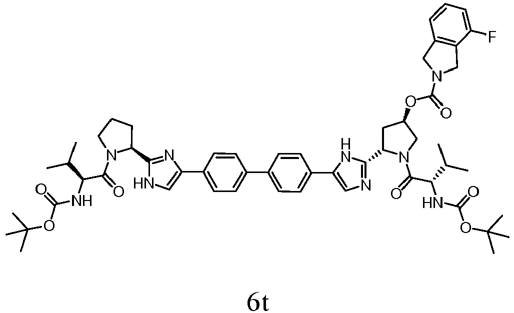 |
| **Ia-19** | 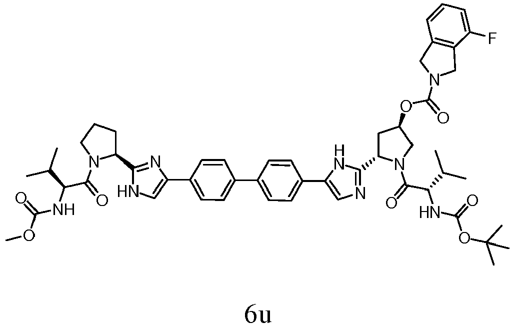 |
| **Ia-20** | 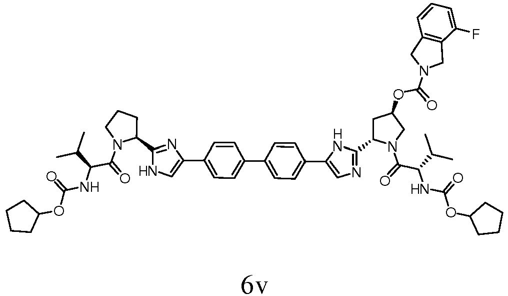 |
| **Ia-21** | 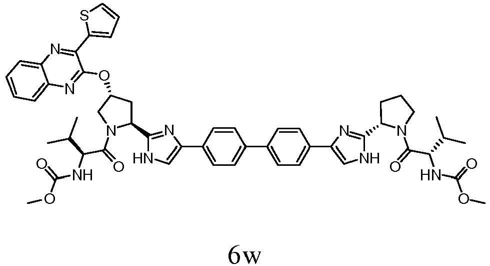 |
| **Ia-22** | 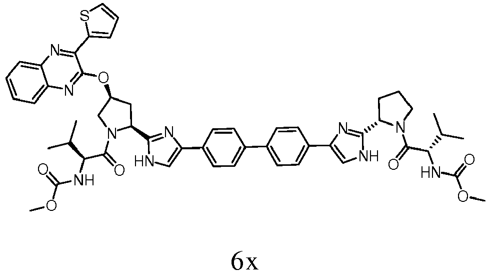 |
| **Ia-23** | 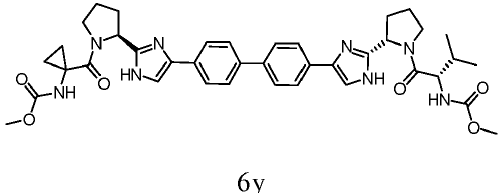 |
| **Ia-24** | 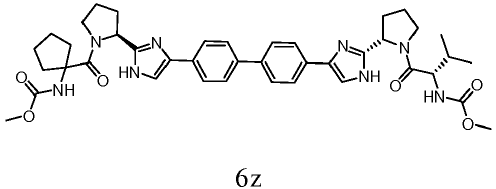 |
| **Ia-25** | 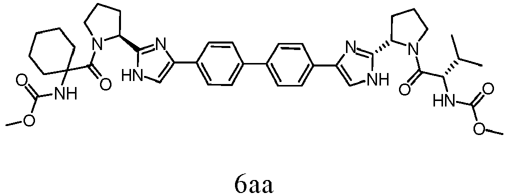 |
| **Ia-26** | 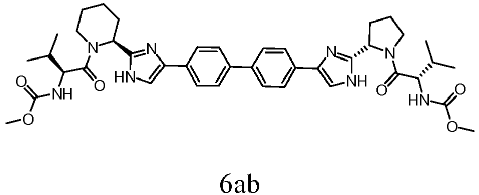 |
| **Ia-27** | 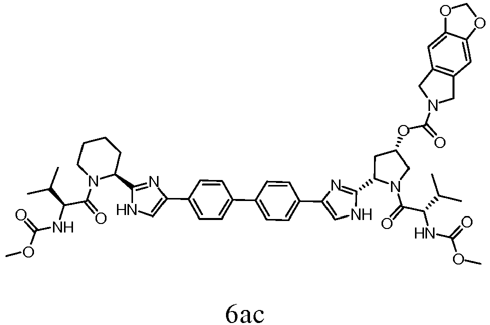 |
| **Ia-28** | 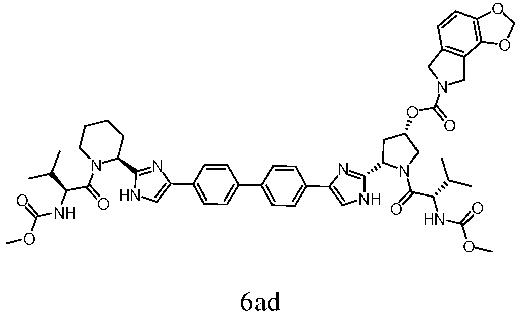 |
| **Ia-29** | 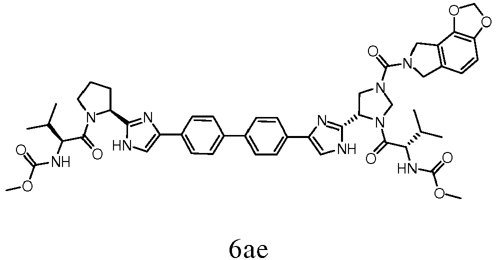 |
| **Ia-30** | 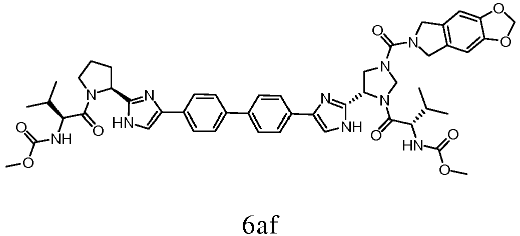 |
| **Ia-31** | 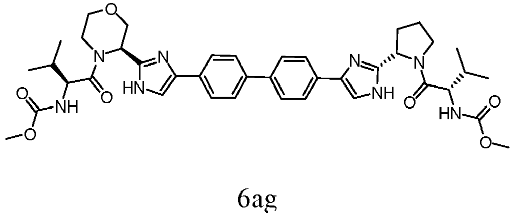 |
| **Ia-32** | 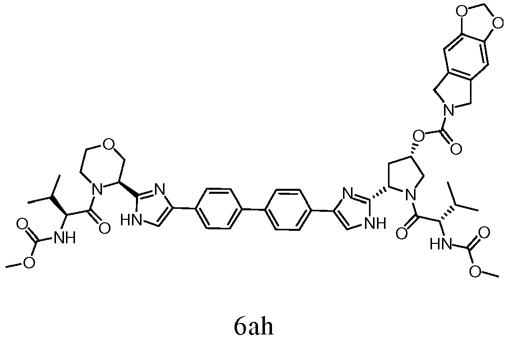 |
| **Ia-33** | 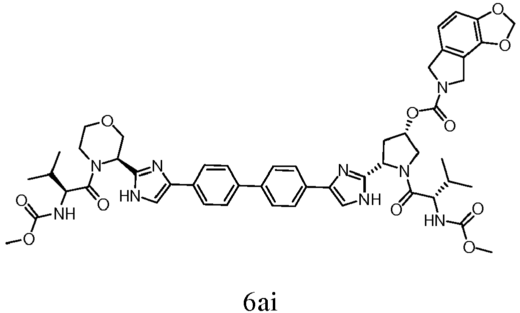 |
| **Ia-34** | 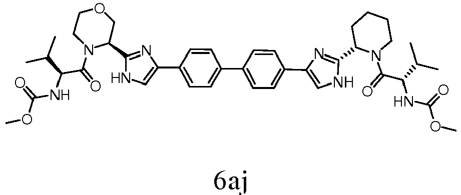 |
| **Ia-35** | 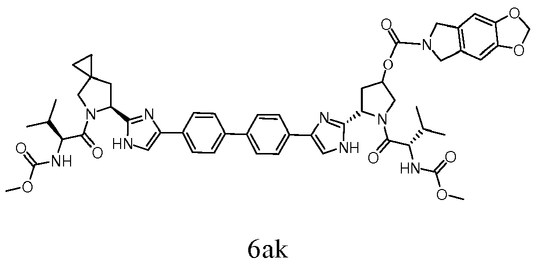 |
| **Ia-36** | 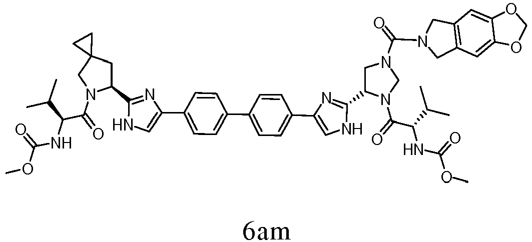 |
| **Ia-37** | 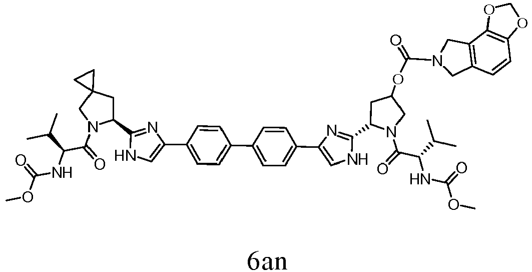 |
| **Ia-38** | 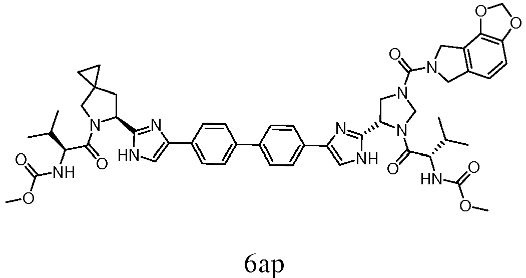 |
| **Ia-39** | 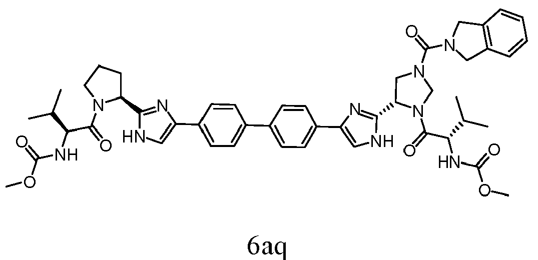 |
| **Ia-40** | 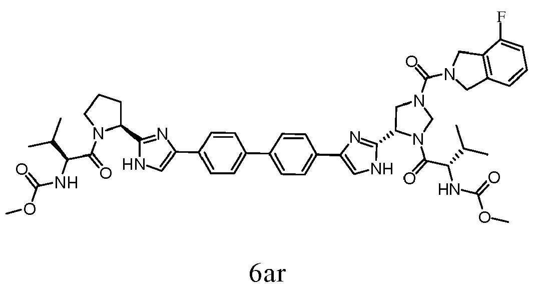 |
| **Ia-41** | 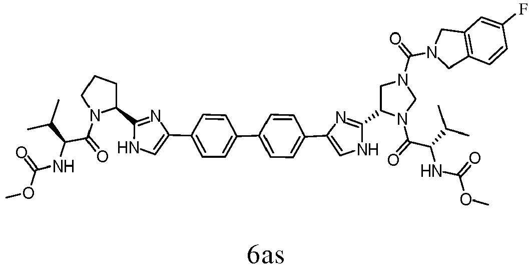 |
| **Ia-42** | 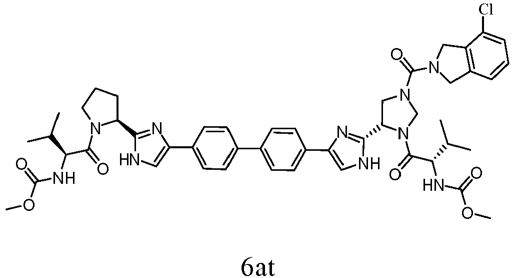 |
| **Ia-43** | 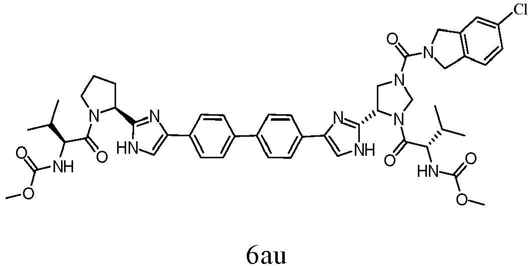 |
| **Ia-44** | 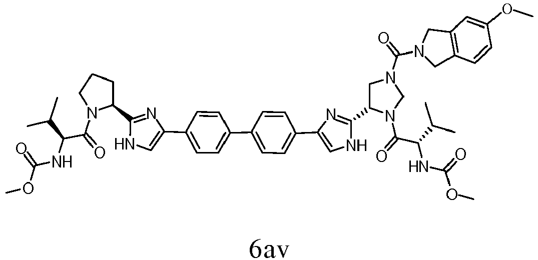 |
| **Ia-45** | 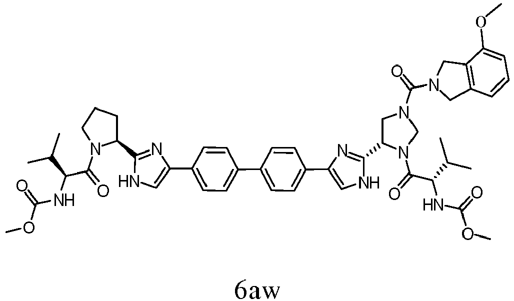 |
| **Ia-46** | 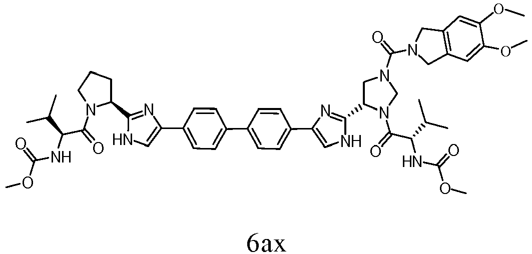 |
| **Ia-47** | 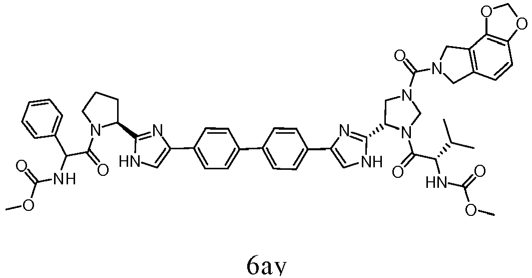 |
| **Ia-48** | 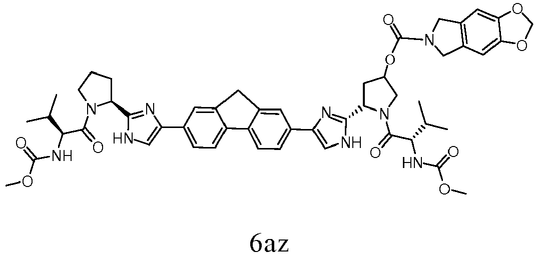 |
| **Ia-49** | 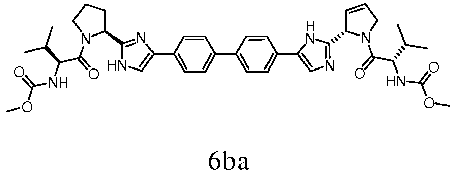 |
| **Ia-50** | 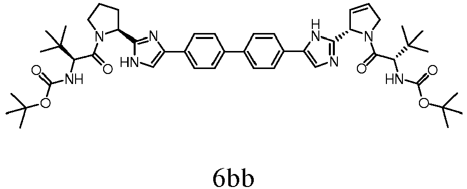 |
| **Ia-51** | 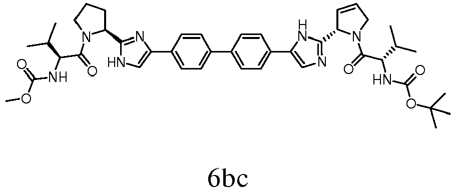 |
| **Ia-52** | 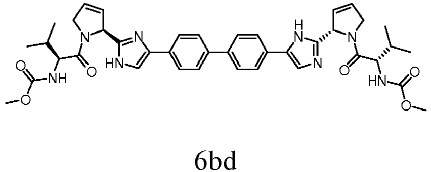 |
| **Ia-53** | 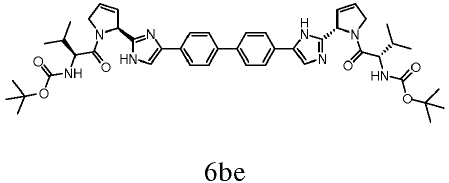 |
| **Ia-54** | 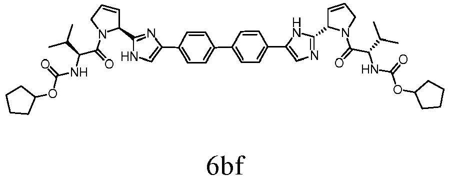 |
| **Ia-55** | 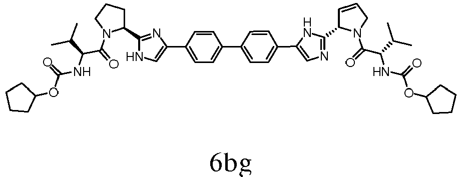 |
| **Ia-56** | 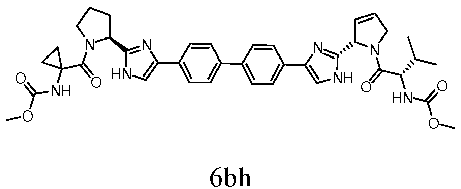 |
| **Ia-57** | 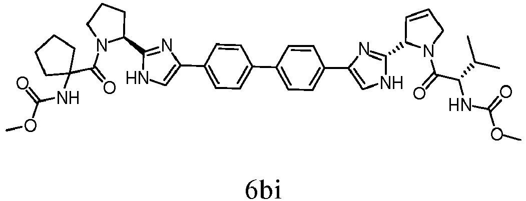 |
| **Ia-58** | 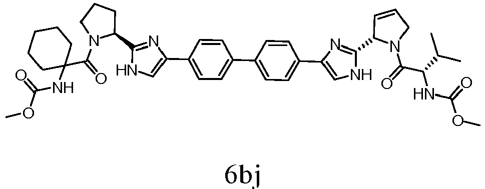 |
| **Ia-59** | 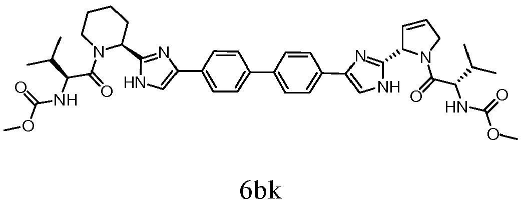 |
| **Ia-60** | 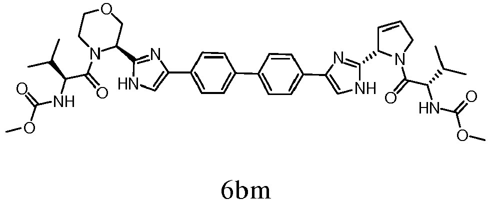 |
| **Ia-61** | 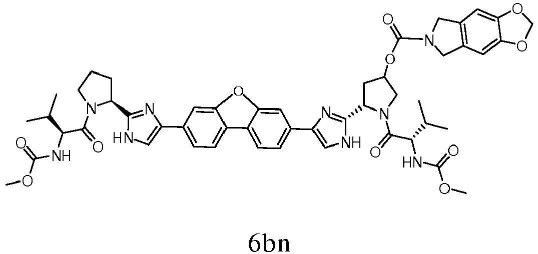 |
| **Ia-62** | 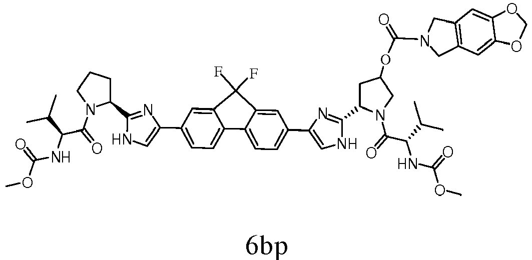 |
| **Ia-63** | 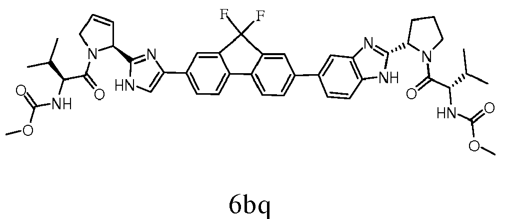 |
| **Ia-64** | 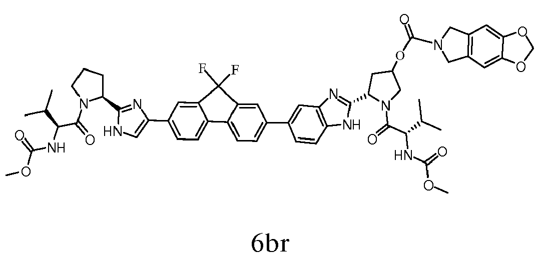 |
| **Ia-65** | 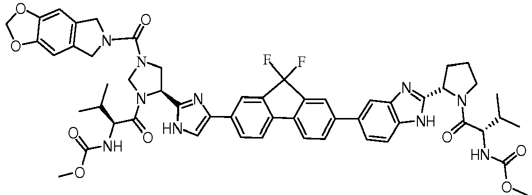 |
| | 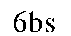 |
| **Ia-66** | 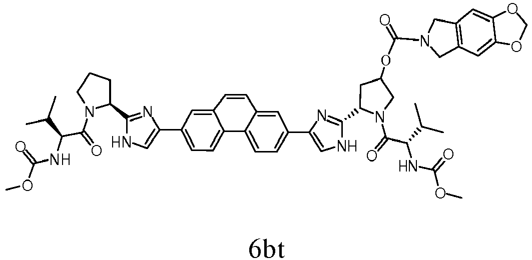 |
| **Ia-67** | 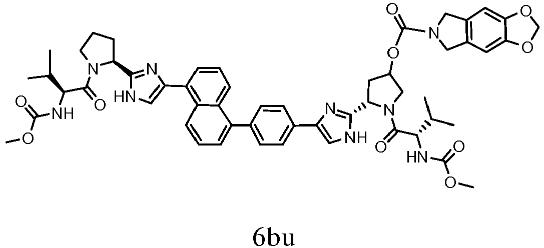 |
| **Ia-68** | 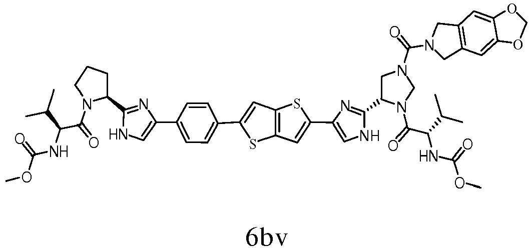 |
| **Ia-69** | 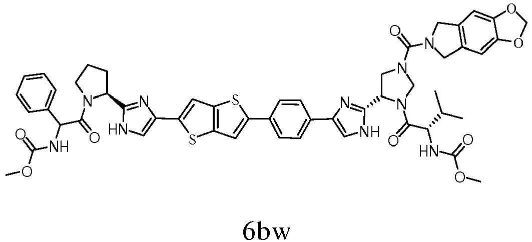 |
| **Ia-70** | 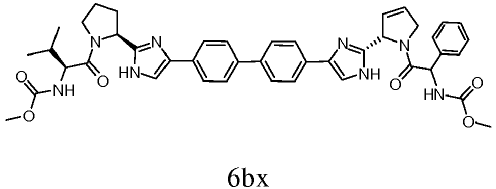 |
| **Ia-71** | 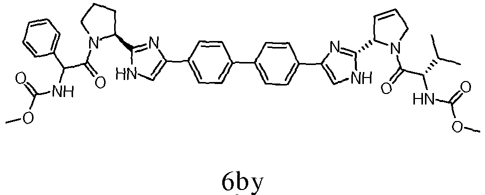 |
| **Ia-72** | 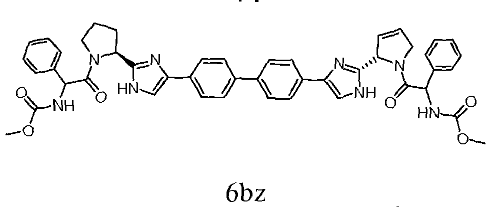 |
| **Ia-73** | 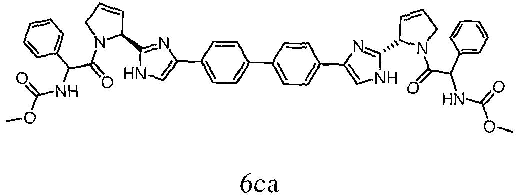 |
| **Ia-74** | 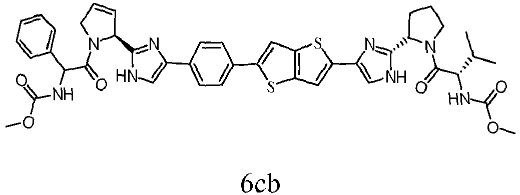 |
| **Ia-75** | 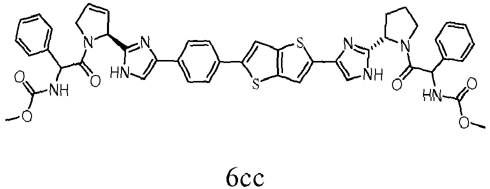 |
| **Ia-76** | 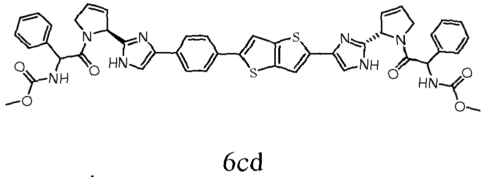 |
| **Ia-77** | 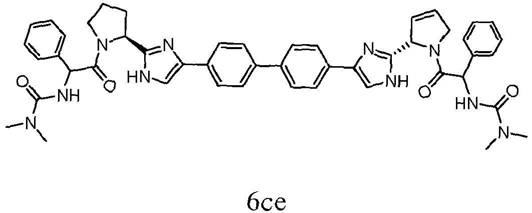 |
| **Ia-78** | 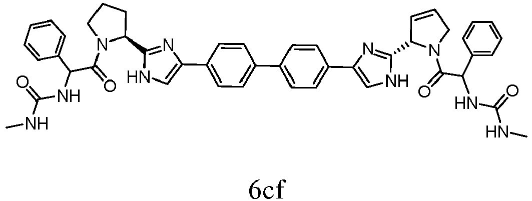 |
| **Ia-79** | 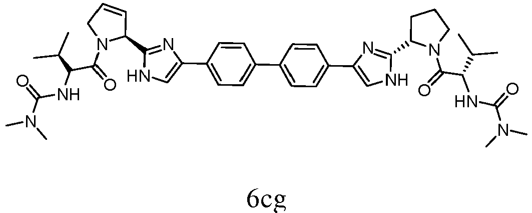 |
| **Ia-80** | 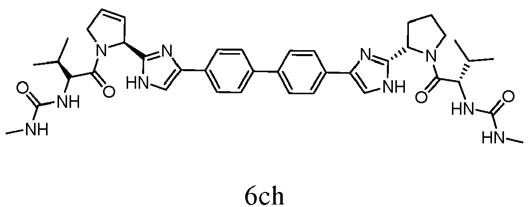 |
| **Ia-81** | 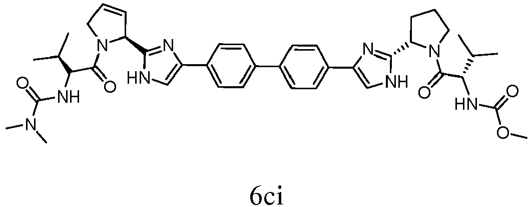 |
| **Ia-82** | 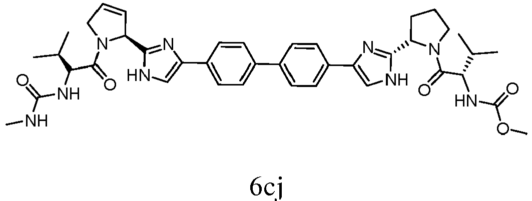 |
| **Ia-83** | 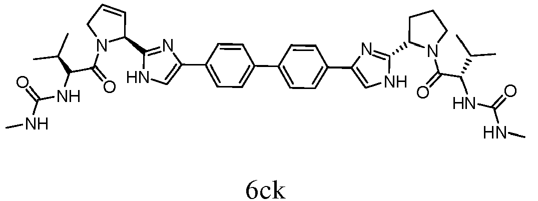 |
| **Ia-84** | 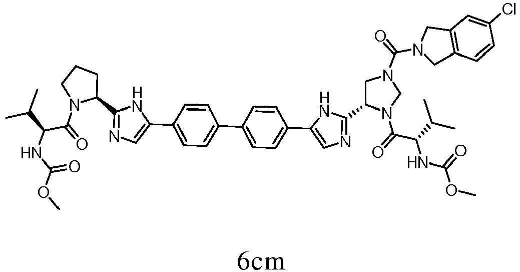 |
| **Ia-85** | 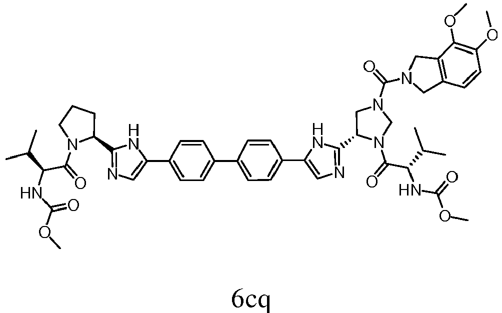 |
| **Ia-86** | 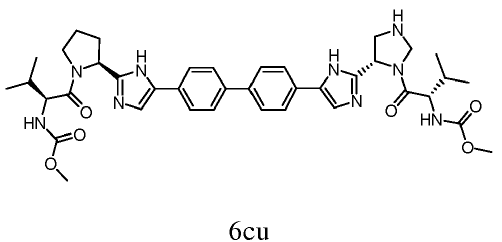 |
| **Ia-87** | 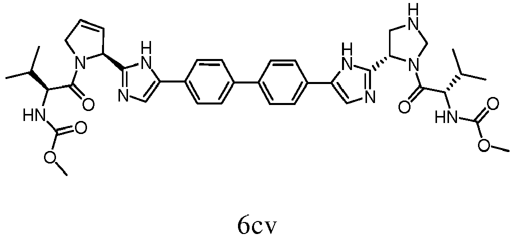 |
| **Ia-88** | 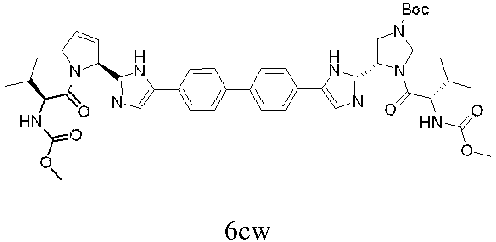 |
| **Ia-89** | 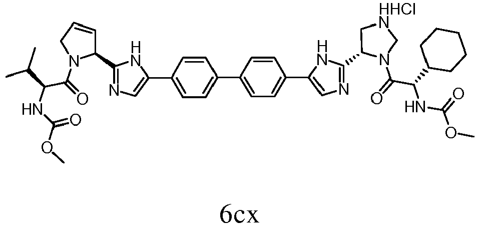 |
| **Ia-90** | 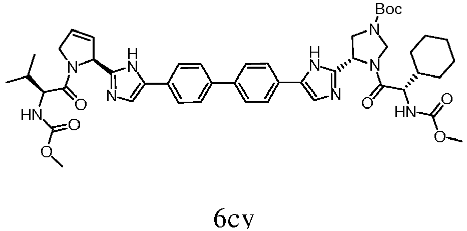 |
| **Ia-91** | 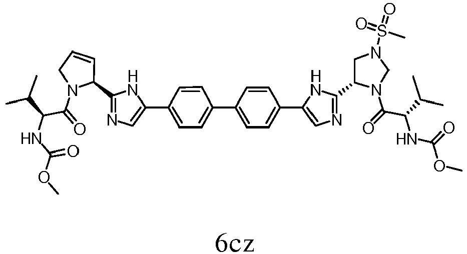 |
| **Ia-92** | 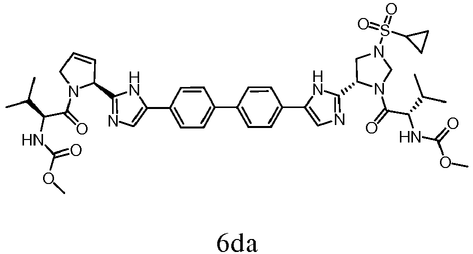 |
| **Ia-93** | 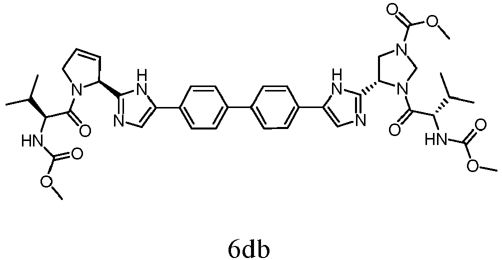 |
| **Ia-94** | 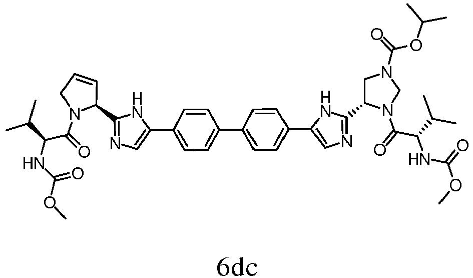 |
| **Ia-95** | 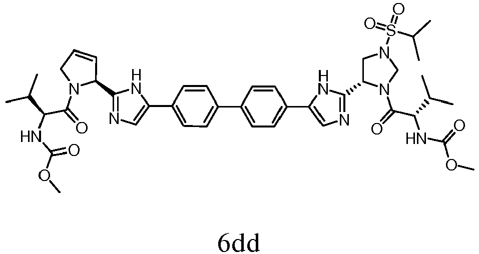 |
| **Ia-96** | 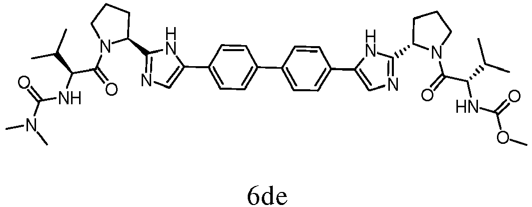 |
| **Ia-97** | 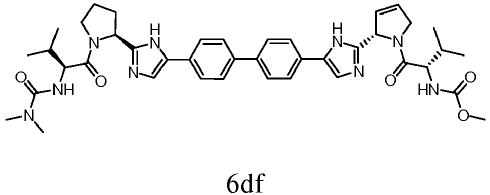 |
| **Ia-98** | 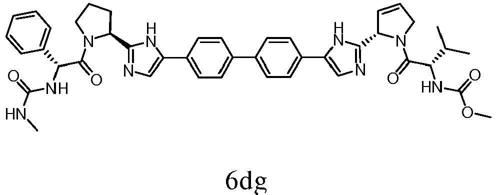 |
| **Ia-99** | 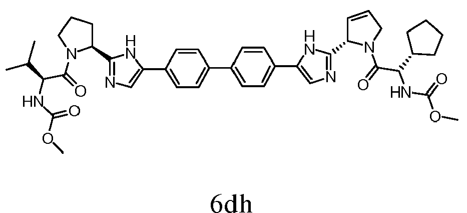 |
| **Ia-100** | 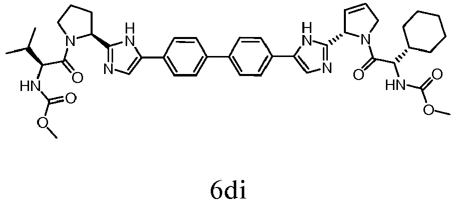 |
| **Ia-101** | 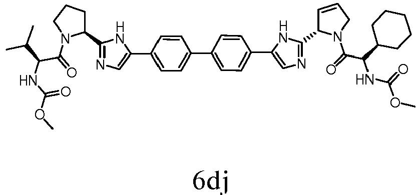 |
| **Ia-102** | 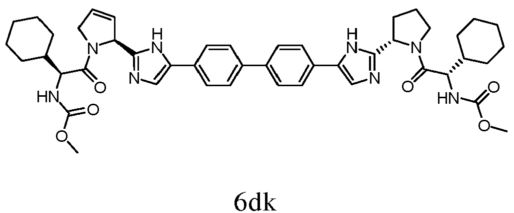 |
| **Ia-103** | 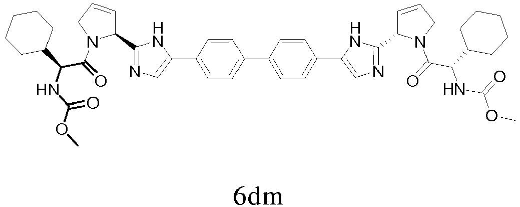 |
| **Ia-104** | 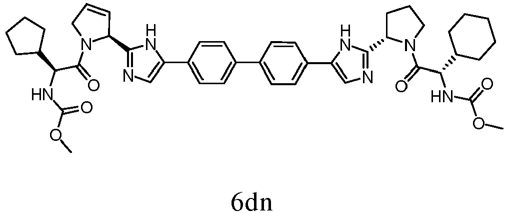 |
| **Ia-105** | 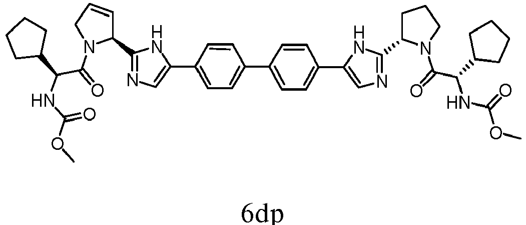 |
| **Ia-106** | 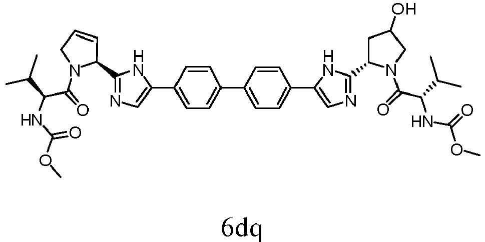 |
| **Ia-107** | 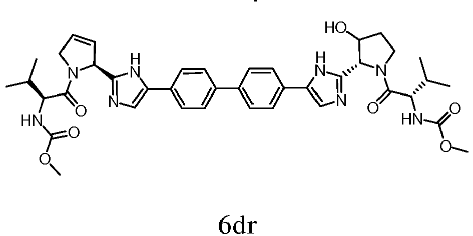 |
| **Ia-108** | 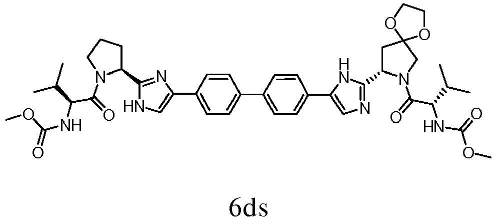 |
| **Ia-109** | 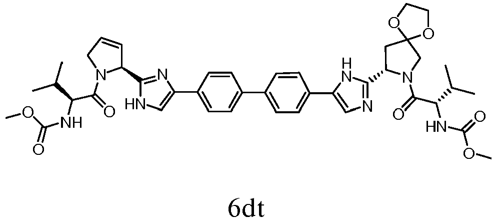 |
| **Ia-110** | 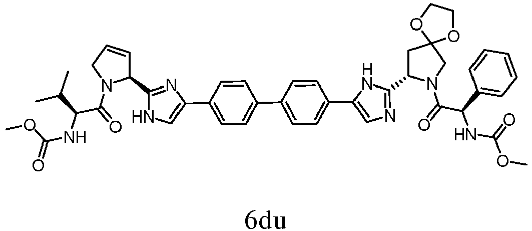 |
| **Ia-111** | 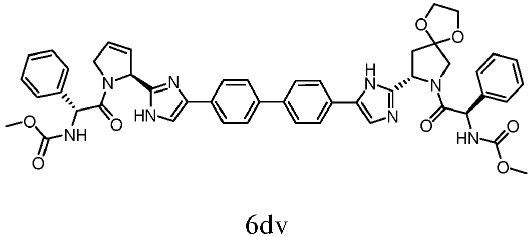 |
| **Ia-112** | 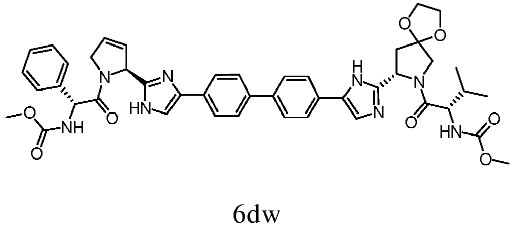 |
| **Ia-113** | 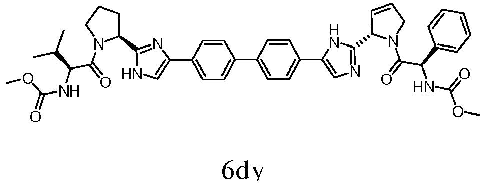 |
| **Ia-114** | 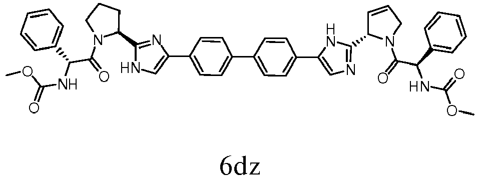 |
| **Ia-115** | 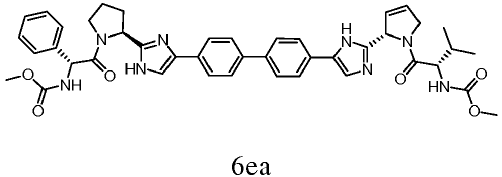 |
| **Ia-116** | 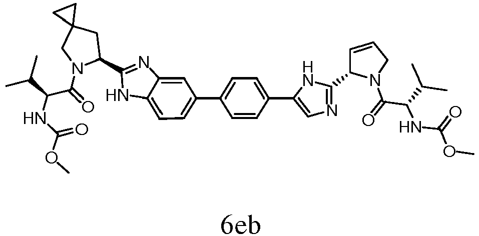 |
| **Ia-117** | 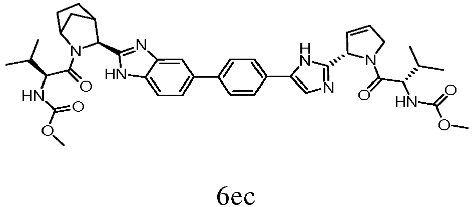 |
| **Ia-118** | 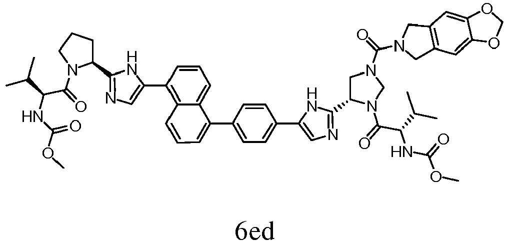 |
| **Ia-119** | 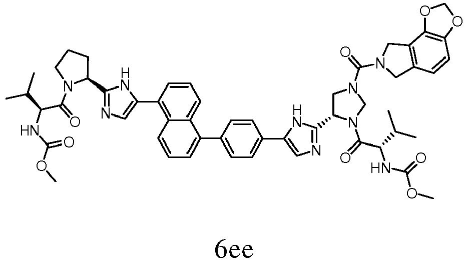 |
| **Ia-120** | 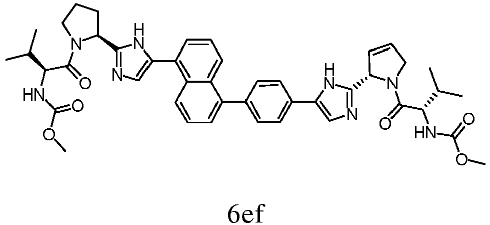 |
| **Ia-121** | 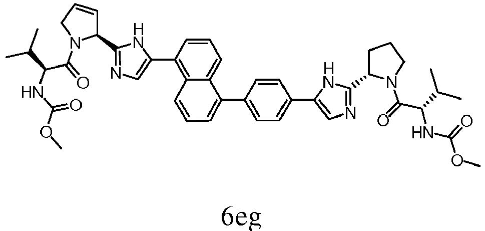 |
| **Ia-122** | 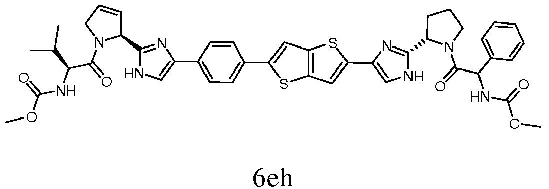 |
| **Ia-123** | 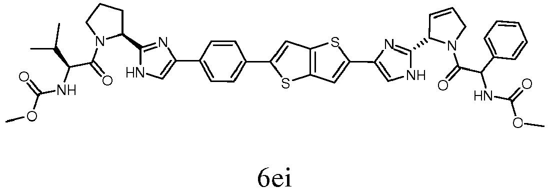 |
| **Ia-124** | 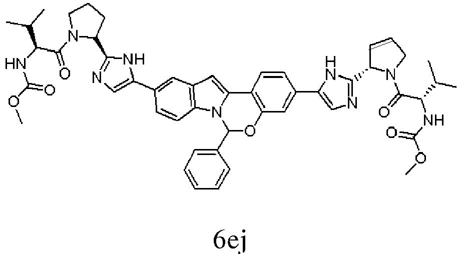 |
| **Ia-125** | 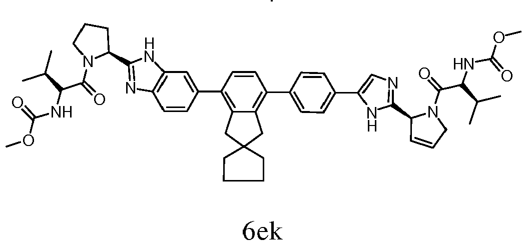 |
| **Ia-126** | 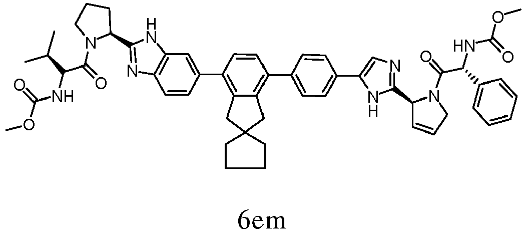 |
| **Ia-127** | 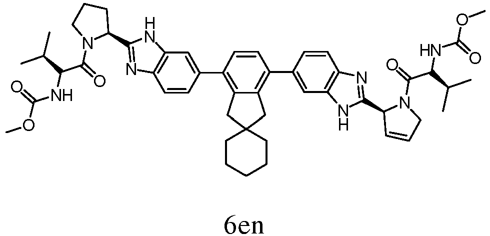 |
| **Ia-128** | 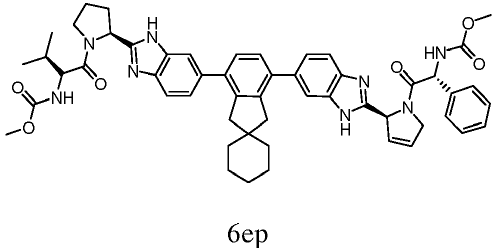 |

Compounds **6fa-6gq** as represented by Formula **Ib** are shown below:

| NO. | Compounds as represented by Formula **Ib** |
|---|---|
| **Ib-1** | 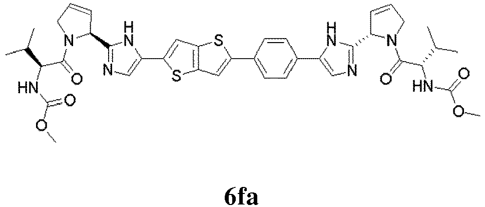 |
| **Ib-2** | 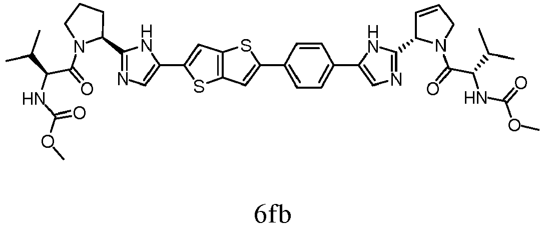 |
| **Ib-3** | 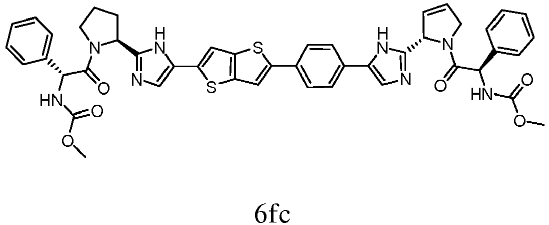 |
| **Ib-4** | 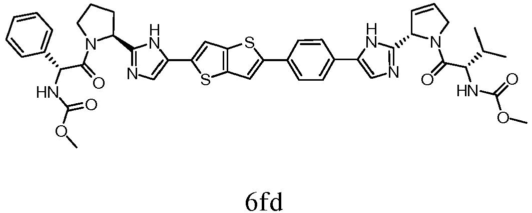 |
| **Ib-5** | 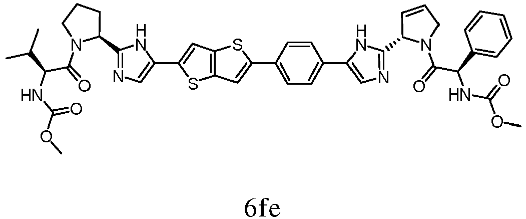 |
| **Ib-6** | 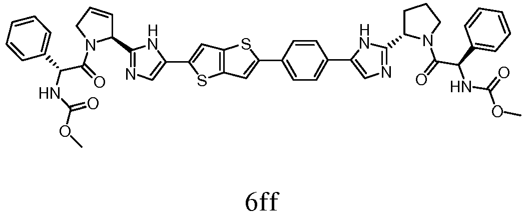 |
| **Ib-7** | 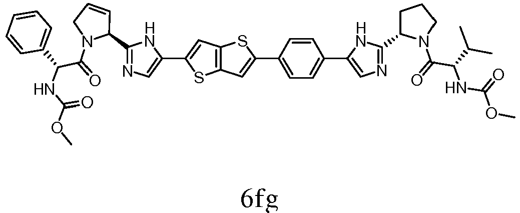 |
| **Ib-8** | 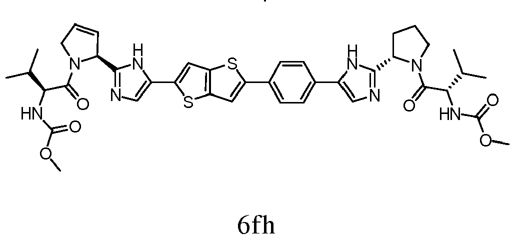 |
| **Ib-9** | 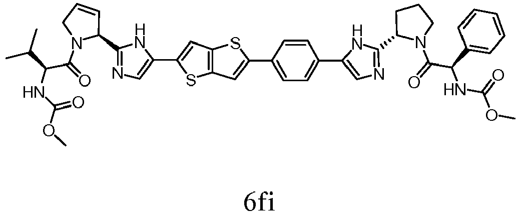 |
| **Ib-10** | 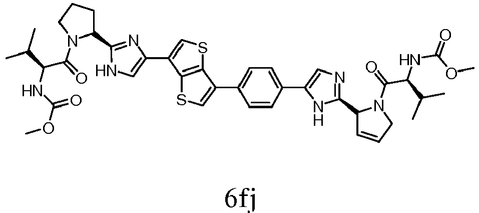 |
| **Ib-11** | 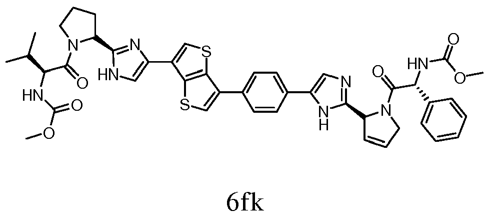 |
| **Ib-12** | 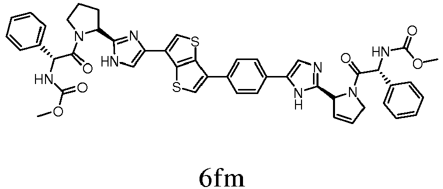 |
| **Ib-13** | 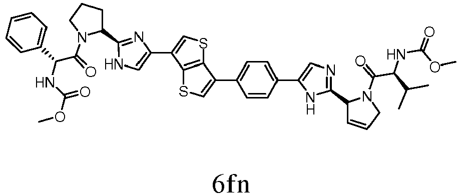 |
| **Ib-14** | 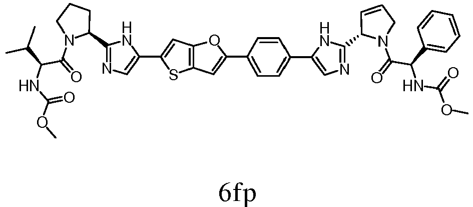 |
| **Ib-15** | 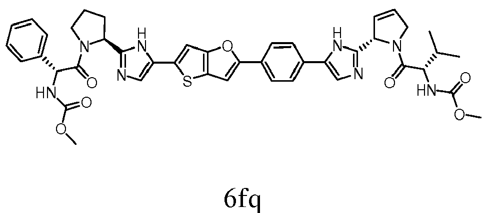 |
| **Ib-16** | 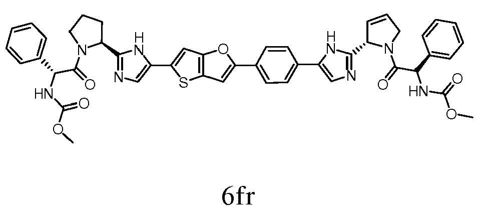 |
| **Ib-17** | 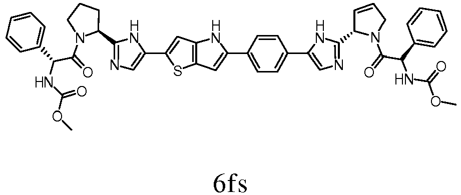 |
| **Ib-18** | 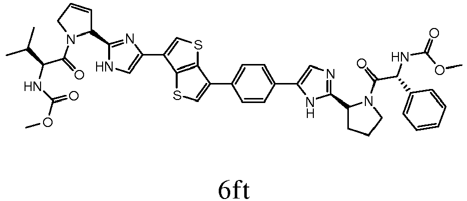 |
| **Ib-19** | 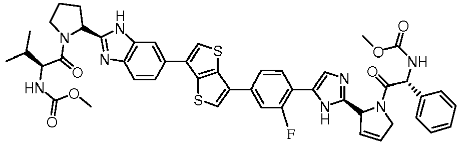 |
| | 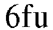 |
| **Ib-20** | 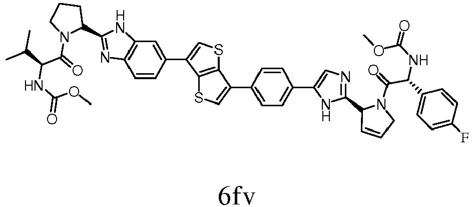 |
| **Ib-21** | 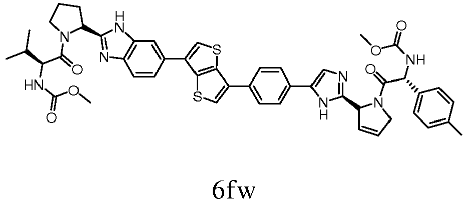 |
| **Ib-22** | 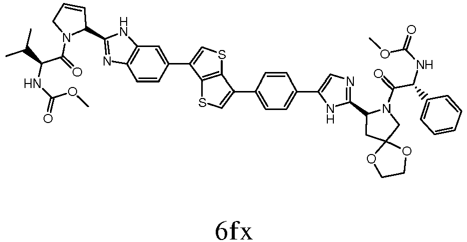 |
| **Ib-23** | 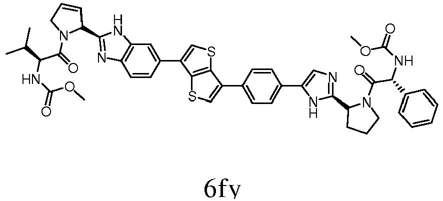 |
| **Ib-24** | 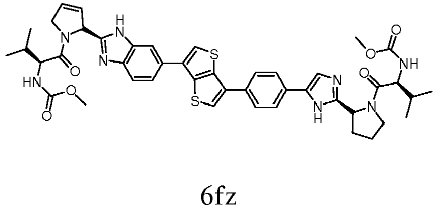 |
| **Ib-25** | 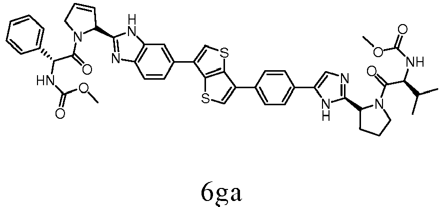 |
| **Ib-26** | 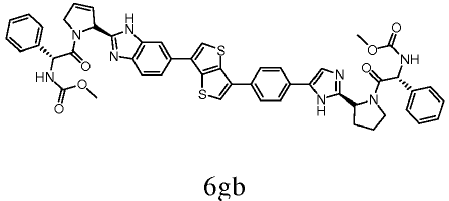 |
| **Ib-27** | 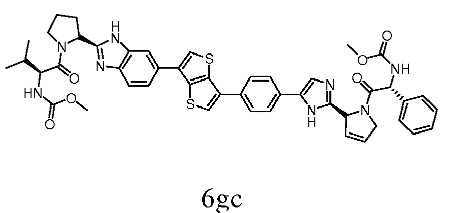 |
| **Ib-28** | 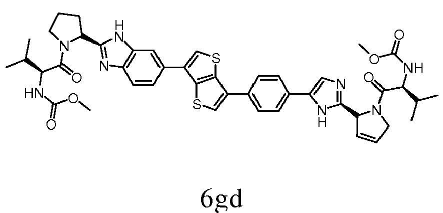 |
| **Ib-29** | 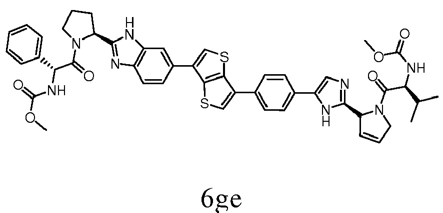 |
| **Ib-30** | 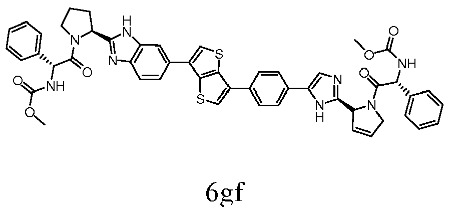 |
| **Ib-31** | 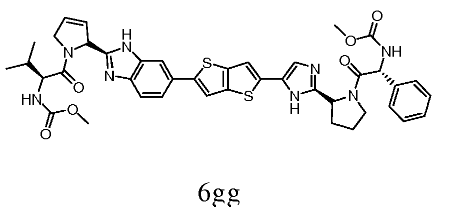 |
| **Ib-32** | 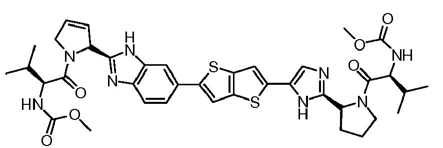 |
| | 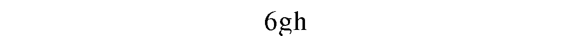 |
| **Ib-33** | 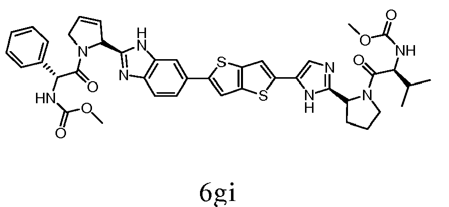 |
| **Ib-34** | 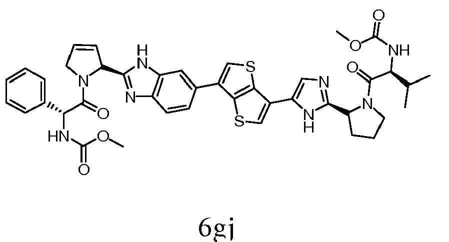 |
| **Ib-35** | 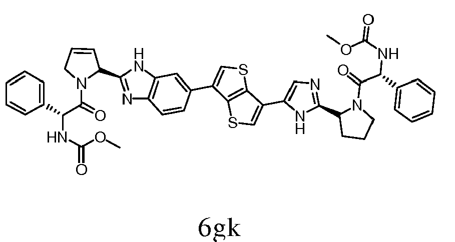 |
| **Ib-36** | 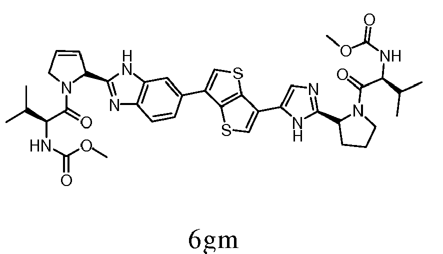 |
| **Ib-37** | 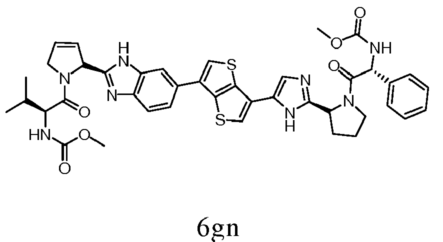 |
| **Ib-38** | 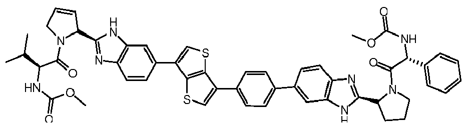 |
| | 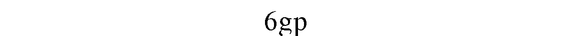 |
| **Ib-39** | 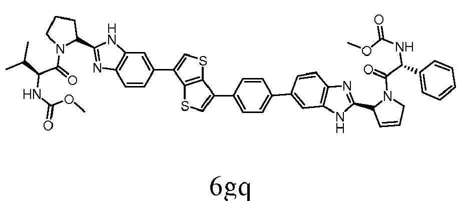 |

This invention also discloses the application of the described compounds as represented by Formula Ia-70, Ia-71, Ia113 or Ia-115, their stereoisomers, tautomers, pharmaceutically acceptable salts in the preparation of HCV inhibiting drugs.

This invention also discloses the application of the mixture of one or more compositions selected from the described compounds as represented by Formula Ia-70, Ia-71, Ia113 or Ia-115, their stereoisomers, tautomers, and pharmaceutically acceptable salts in the preparation of HCV inhibiting drugs.

This invention also discloses a pharmaceutical composition comprising the described compounds as represented by Formula Ia-70, Ia-71, Ia113 or Ia-115, their stereoisomers, tautomers, or pharmaceutically acceptable salts, and pharmaceutically acceptable excipient(s).

The described pharmaceutical composition in this invention may also contain one or more ingredients selected from immunoregulants, HCV-NS3/4A inhibitors, HCV-NS5B inhibitors, HCV inhibitors in the categories of nucleosides, nucleoside derivatives and non-nucleosides, HBV inhibitors, HIV inhibitors, anti-cancer drugs and anti-inflammatory drugs. Wherein, the described immunoregulants are preferably interferon or interferon derivatives; wherein the described interferon is preferably pegylated interferon; the described HIV inhibitors include ritonavir and/or ribavirin; the described HBV inhibitors include lamivudine, telbivudine, adefovir, emtricitabine, entecavir, tenofovir and clevudine; the described HIV inhibitors include ritonavir and/or ribavirin; the described HCV protease inhibitor is preferably VX-950, ZN2007, ABT-450, RG-7227, TMC-435, MK-5172, MK-7009, ACH-1625, GS-9256, TG2349, BMS-650032, IDX320, yimitasvir phosphate, or seraprevir potassium; the described HCV polymerase inhibitor is preferably GS-5885, TMC647055, ABT-267, BMS-791325, PPI-383, or ALS-002158.

In the described pharmaceutical composition of this invention, the content of the described compounds as represented by Formula Ia-70, Ia-71, Ia113 or Ia-115, their stereoisomers, tautomers, and pharmaceutically acceptable salts is preferably 0.01%-99.9% (mass percent); the described mass percent means the percentage of the mass of compounds as represented by Formula **Ia,** their stereoisomers, tautomers, and pharmaceutically acceptable salts in total mass of the pharmaceutical composition. This invention also discloses the application of the described pharmaceutical compositions in the preparation of HCV-inhibiting medicine.

Unless otherwise specifically provided herein, the described alkyl group refers to branched chain and linear chain saturated fatty hydrocarbonyl containing 1∼20 carbon atoms, preferably 1∼10 carbon atoms, and more preferably 1∼8 carbon atoms, e.g. methyl, ethyl, n-propyl, isopropyl, n-butyl, tert-butyl, isobutyl, pentyl, hexyl, heptyl, octyl, nonyl, decyl, 4,4-dimethyl pentyl, 2,2,4-trimethyl pentyl, hendecyl, lauryl, and their isomers; and any of the above-mentioned alkyls that has 1∼4 substituents selected from aryl, heterocyclic aryl, cycloalkyl, cycloalkenyl, epoxyl, heterocyclic radical, alkoxyl carbonyl, aryloxycarbonyl, heterocyclic oxyl, alkyl amino, alkylaminocarbonyl, arylamino, heterocyclic amino, aryl sulfonyl, alkylaminosulfonyl, heterocyclic aminosulfonyl, alkylsulfonamino, heterocyclic sulfonamino, arylsulfonamino, alkyl aminosulfonamino, alkylcarbonylamino, fused aryl, fused alkylaryl, fused alkyl, fused epoxyl, alkylureido, alkyl group, alkyl sulphide, alkylthioureido, ureido or thioureido.

Unless otherwise specifically provided herein, the described alkoxyl refers to a radical formed by the connection of alkyl and oxigen atom, i.e., 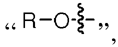 R represents alkyl radical.

Unless otherwise specifically provided herein, the described aryl refers to any stable monocyclic or bicyclic radical, with each nucleus consisting of up to 7 atoms, wherein at least one ring is aromatic; in the case of bicyclic nucleus, fused nucleus is excluded but spiro nucleus is included. For example, phenyl or 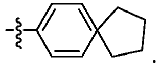 and any of the aryl radicals having one or more of the following radicals as substituents: aryl, heterocyclic aryl, cycloalkyl, cycloalkenyl, epoxyl, heterocyclic radical, alkoxyl carbonyl, aryloxycarbonyl, heterocyclic oxyl, alkyl amino, alkylaminocarbonyl, arylamino, heterocyclic amino, aryl sulfonyl, alkylaminosulfonyl, heterocyclic aminosulfonyl, alkylsulfonamino, heterocyclic sulfonamino, arylsulfonamino, alkyl aminosulfonamino, alkylcarbonylamino, fused aryl, fused cyclic alkylaryl, fused cyclic alkyl, fused epoxyl, alkylureido, alkyl, alkyl sulphide, alkylthioureido, ureido or thioureido, for example, biphenylyl.

Unless otherwise specifically provided herein, the described heterocyclic aryl refers to a stable monocyclic or bicyclic ring whose nucleus consists of up to 7 atoms, wherein at least one ring is an aromatic one containing 1-4 hetero-atoms selected from O, N, and S; and the above-mentioned heterocyclic aryl containing one or more substituents selected from the undermentioned radicals defined in this invention: aryl, heterocyclic aryl, cycloalkyl, cycloalkenyl, epoxyl, heterocyclic radical, alkoxyl carbonyl, aryloxycarbonyl, heterocyclic oxyl, alkyl amino, alkylaminocarbonyl, arylamino, heterocyclic amino, aryl sulfonyl, aalkylaminosulfonyl, heterocyclic aminosulfonyl, alkylsulfonamino, heterocyclic sulfonamino, arylsulfonamino, alkyl aminosulfonamino, alkylcarbonylamino, fused aryl, fused alkylaryl, fused alkyl, fused epoxyl, alkylureido, alkyl, alkyl sulphide, alkylthioureido, ureido or thioureido. Heterocyclic aryl radicals that fall into the scope of this definition include but are not limited to acridinyl, carbazolyl, cinnolinyl, quinoxalyl, pyrazolyl, indyl, benzotriazolyl, furyl, thiophene thiofuryl, benzothiazolyl, benzothiophenyl, benzofuranyl, quinolinyl, isoquinolyl, oxazolyl, isoxazolyl, indyl, pyrazinyl, pyridazinyl, pyridyl, pyrimidinyl, pyrrolyl, tetrahydroquinolinyl. According to the following definition of heterocyclic nucleus, heterocyclic aryls are deemed to include the N-oxide derivatives of any nitrogen-containing heterocyclic aryls. When a heterocyclic aryl substituent is a bicyclic substituent and one of the nucleus is a non-aromatic one or hetero-atom free, it is understandable that the two nucleus are connected vial the aromatic ring or the heteroatom-containing ring.

Unless otherwise specifically provided herein, the described alkyl sulphide refers to a radical formed by connecting the alkyl radical to sulfur atom, i.e., 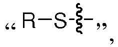 wherein R represents an alkyl radical.

Unless otherwise specifically provided herein, the described aryloxyl refers to a radical formed by connecting the aryl group to an oxigen atom, i.e., 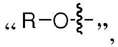 wherein R represents an aryl radical.

Unless otherwise specifically provided herein, the described arylamino radical refers to a radical formed by substituting a hydrogen in "NH₃" with an aryl radical.

Unless otherwise specifically provided herein, the described cycloalkyl radical refers to a total-carbon monocyclic or polycyclic radical that is free of any double bond on its necleus. It is preferably a cycloalkyl radical consisting of 1∼3 rings of 3∼20 carbons, more preferably, 3∼10 carbons, for example: cyclopropyl, cyclobutyl, cyclopentyl, cyclohexyl, cycloheptyl, cyclooctyl, cyclodecanyl and cyclolauryl; the cycloalkyl radical can be substituted by 1∼4 substituents as defined herein, i.e. deuterium, halogen, alkyl, alkoxyl, hydroxyl, aryl, aryloxyl, arylalkyl, cycloalky, alkylamino, amido, oxygen, acyl, arylcarbonylamino, amino, nitrile, mercapto, alkyl sulphide and alkyl.

Unless otherwise specifically provided herein, the described cycloalkenyl refers to a total-carbon monocyclic or polycyclic radical, wherein every nucleus can contain one or more double bonds but none of such necleus should be with a conjugated π electric system. It is preferably a cycloalkenyl whose necleus is consisted of 3∼20 carbons, more preferably of 3∼10 carbons, e.g., cyclopropenyl, cyclobutenyl, cyclopentenyl, cyclohexenyl, cycloheptenyl, cyclooctenyl, cyclodecenyl and cyclododecenyl; the cycloalkenyl can be substituted by one or more substituent defined in this invention, including deuterium, halogen, alkyl, alkoxyl, hydroxyl, aryl, aryloxyl, arylalkyl, cycloalkyl, alkylamino, amido, oxygen, carbonyl, arylcarbonylamino, amino, nitrile, mercapto, alkyl sulphide and alkyl. When substitution of cycloalkenyl take place on a carbon-carbon double bond and the double bond is satuared, cycloalkyl will form.

Unless otherwise specifically provided herein, the described epoxyl refers to a cycloalkyl connected to an etheryl-containing radical.

Unless otherwise specifically provided herein, the described heterocyclic radical refers to an aromatic or non-aromatic heterocyclic ring that contain one or more hetero-atoms selected from O, N and S, and may include bicyclic radicals. Therefore, heterocyclic radicas include the above-mentioned heterocyclic aryls and their dihydro or tetrahydro analogs. Other examples of heterocyclic radicals include but are not limited to the following: benzimidazolyl, benzofuranyl, benzopyrazolyl, benzotriazolyl, benzothiazolyl, benzothiophenyl, benzoxazolyl, carbolinyl, furyl, imidazolyl, dihydroindyl, indyl, indazolyl, isobenzofuranyl, isoazaindenyl, isoquinolyl, isothiazolyl, isoxazolyl, oxazolyl, oxazolinyl, isooxazolinyl, oxy-cyclobutyl, pyranyl, pyrazinyl, pyrazolyl, pyridazinyl, pyridinopyridyl, pyridazinyl, pyridyl, pyrimidinyl, pyrrolyl, quinazolinyl, quinolinyl, quinoxalyl, tetrahydropyranyl, thiadiazolyl, thiazolyl, thiophene thiofuryl, triazolyl, azetidinyl, 1,4-dioxanyl, hexahydroazepinyl, piperazinyl, piperidyl, pyrrolealkyl group, morpholinyl, thio-morpholinyl, dihydrobenzimidazolyl, dihydrobenzofuranyl, dihydrobenzothiophenyl, dihydrobenzoxazolyl, dihydrofuryl, dihydroimidazolyl, dihydroindyl, dihydroisoxazolyl, dihydroisothiazolyl, dihydrooxdiazolyl, dihydrooxazolyl, dihydropyrazinyl, dihydropyrazolyl, dihydropyridyl, dihydropyrimidinyl, dihydropyrrolyl, dihydroquinolinyl, dihydrotetrazolyl, dihydrothiadiazolyl, dihydrothiazolyl, dihydrothiophene thiofuryl, dihydrotriazolyl, dihydroazetidinyl, methylenedioxybenzoyl, tetrahydrofuryl and tetrahydrothiophene thiofuryl and their N-oxides. Heterocyclic radicals can be connected via carbon atom or hetero-atom to nucleus molecule.

Unless otherwise specifically provided herein, the described fused aryl refers to a polycyclic organic compound formed by the fused connection of two or more aryl radicals and/or heterocyclic aryl radicals, the described fused aryl radical may have substituents defined in this invention such as alkyl, alkoxyl, alkyl sulphide, aryloxyl, arylamino, heterocyclic radical, cycloalkyl, cycloalkenyl, epoxyl, aryl, halogen, carbonyl, hydroxyl, heterocyclic aryl in a reasonable way. For example, naphthalenyl, anthracenyl, quinonyl, phenanthrenyl, fluorenyl, Benzimidazolyl, furofuryl, thienothienyl, acenaphthyl,

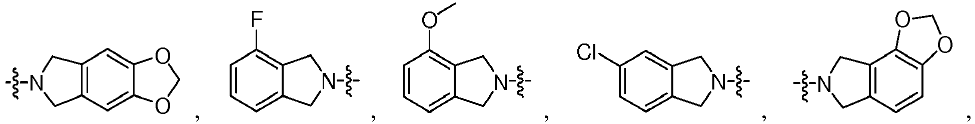

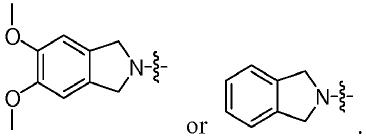

Unless otherwise specifically provided herein, the described fused ring alkylaryl radical refers to an aryl radical whose aromatic nucleus has hydrogen(s) substituted by fused ring alkyl radicals.

Unless otherwise specifically provided herein, the described fused ring alkyl radical refers to a non-aromatic polycyclic system formed by the reduction of one or more double bonds on the fused aryl nucleus.

Unless otherwise specifically provided herein, the described fused ring ether radical refers to a radical formed by connecting an oxygen to a fused aryl or fused alkyl radical, i.e., 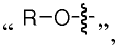 wherein R represents a fused aryl or a fused alkyl radical.

Unless otherwise specifically provided herein, the described alkoxyl carbonyl refers to a radical formed by connecting an alkoxyl radical to a carbonyl radical, i.e., 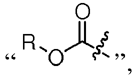 wherein R represents an alkyl radical.

Unless otherwise specifically provided herein, the described aryloxycarbonyl radical refers to a radical formed by connecting an aryloxyl radical to a carbonyl radical, i.e., 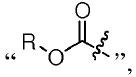 wherein R represents an aryl radical.

Unless otherwise specifically provided herein, the described heterocyclic oxyl radical refers to a radical formed by connecting a heterocyclic radical to an oxygen atom, i.e., 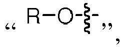 wherein R represents a heterocyclic radical.

Unless otherwise specifically provided herein, the described alkyl amino radical refers to a radical formed by connecting an alkyl radical to an amino radical, i.e., 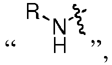 wherein R represents an alkyl radical.

Unless otherwise specifically provided herein, the described alkylaminocarbonyl radical refers to a radical formed by connecting an alkyl amino radical to a carbonyl radical, i.e., 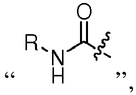 wherein R represents an alkyl radical.

Unless otherwise specifically provided herein, the described arylamino radical refers to a radical formed by connecting an aryl radical to an amino radical, i.e., 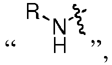 wherein R represents an aryl group.

Unless otherwise specifically provided herein, the described heterocyclic amino radical refers to a radical formed by connecting a heterocyclic radical to an amino radical, i.e., 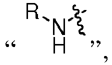 wherein R represents a heterocyclic radical.

Unless otherwise specifically provided herein, the described arylaminosulfonyl radical refers to a radical formed by connecting an arylamino radical to a sulfonyl radical, i.e., 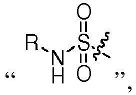 wherein R represents an aryl group.

Unless otherwise specifically provided herein, the described alkylaminosulfonyl radical refers to a radical formed by connecting an alkyl amino radical to a sulfonyl radical, i.e., 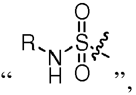 wherein R represents an alkyl group.

Unless otherwise specifically provided herein, the described heterocyclic aminosulfonyl radical refers to a radical formed by connecting a heterocyclic amino radical to a sulfonyl group, i.e., 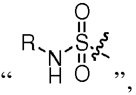 wherein R represents a heterocyclic radical.

Unless otherwise specifically provided herein, the described alkylsulfonamino refers to a radical formed by connecting an alkyl radical to Sulfonaminoa group formed by connecting, i.e., 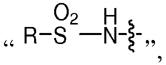 wherein R represents an alkyl radical.

Unless otherwise specifically provided herein, the described Heterocyclic ringSulfonamino refers to a radical formed by connecting a heterocyclic radical to a sulfonaminoa radical, i.e., 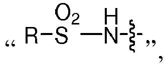 wherein R represents a heterocyclic radical.

Unless otherwise specifically provided herein, the described arylsulfonamino radical refers to a radical formed by connecting an aryl radical to a sulfonamino radical, i.e., 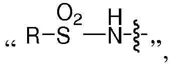 wherein R represents an aryl group.

Unless otherwise specifically provided herein, the described alkyl aminosulfonamino radical refers to a radical formed by connecting an alkyl amino radical to a sulfonaminoa radical, i.e., 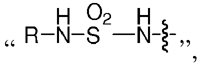 wherein R represents an alkyl group.

Unless otherwise specifically provided herein, the described alkylcarbonylamino radical refers to a radical formed by connecting an alkyl radical to a carbonyl radical and an amino radical in succession, i.e., 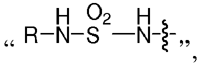 wherein R represents an alkyl group.

Unless otherwise specifically provided herein, the described alkylureido radical refers to a radical formed by connecting an alkyl radical to a ureido radical and an amino radical in succession, i.e., 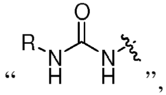 wherein R represents an alkyl group.

Unless otherwise specifically provided herein, the described alkylthioureido radical refers to a radical formed by connecting an alkyl radical to a thioureido radical in succession, i.e., 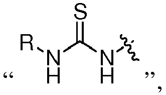 wherein R represents an alkyl group.
In this invention, the term halogen refers to fluorine, chlorine, bromine, iodine or astatine.
In this invention, the term hydroxyl radical refers to 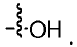 In this invention, the term amino radical refers to 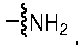 In this invention, the term nitrile radical refers to 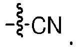 In this invention, the term carboxyl refers to 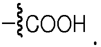 In this invention, the term sulfonyl refers to 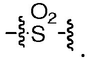 In this invention, the term sulfonamino radical refers to 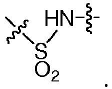 In this invention, the term carbonyl refers to 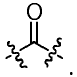 In this invention, the term ureido radical refers to 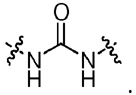 In this invention, the term thioureido radical refers to 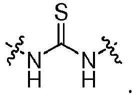 In this invention, the substituent radicals may be preceded by a Cₓ₁-_{yl} (x1 and y1 are integers), e.g. "Cₓ₁-_{y1}" alkyl, "Cₓ₁-_{y1}" alkoxyl, "Cₓ₁-_{y1}" alkyl sulphide, "Cₓ₁-_{y1}" aryl, "Cₓ₁-_{y1}" heterocyclic aryl, "Cₓ₁-_{y1}" cycloalkyl, "Cₓ₁-_{y1}" cycloalkenyl, "Cₓ₁-_{y1}" epoxyl, "Cₓ₁-_{y1}" heterocyclic radical, "Cₓ₁-_{y1}" alkoxyl carbonyl, "Cₓ₁-_{y1}" aryloxycarbonyl, "Cₓ₁-_{y1}" heterocyclic oxyl, "Cₓ₁-_{y1}" alkyl amino, "Cₓ₁-_{y1}" alkylaminocarbonyl, "Cₓ₁-_{y1}" arylamino, "Cₓ₁-_{y1}" heterocyclic amino, "Cₓ₁-_{y1}" aryl sulfonyl, "Cₓ₁-_{y1}" alkylaminosulfonyl, "Cₓ₁-_{y1}" heterocyclic aminosulfonyl, "Cₓ₁-_{y1}" alkylsulfonamino, "Cₓ₁-_{y1}" heterocyclic sulfonamino, "Cₓ₁-_{y1}" arylsulfonamino, "Cₓ₁-_{y1}" alkyl aminosulfonamino, "Cₓ₁-_{y1}" alkylcarbonylamino, "Cₓ₁-_{y1}" fused aryl, "Cₓ₁-_{y1}" fused alkylaryl, "Cₓ₁-_{y1}" fused alkyl, "Cₓ₁-_{y1}" fused epoxyl, "Cₓ₁-_{y1}" alkylureido or "Cₓ₁-_{y1}" alkylthioureido. This Cₓ₁-_{y1} denotes the number of the number of skeleton carbon atoms (carbon atoms in substituent groups are excluded). For example, C₁∼C₂₀ alkyl denotes a C₁∼C₂₀ alkyl radical that has 1∼20 carbon atoms in its skeleton structure (not substituted).

In the art without departing from common sense, the above-mentioned preferred conditions can be combined at discretion to yield preferred embodiments of the invention. The reagents and raw materials used in the invention are all commercially available.

The advantages of this invention are as follows:
1) the design and introduction of novel heterocyclic functional groups that contain the following two substituent groups "L, Q and/or L¹, Q¹" or double bond(s): 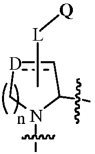 and 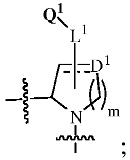 and heterocyclic functional groups of Formula **Ib** that contain no substituent groups "L, Q and L¹, Q¹" : 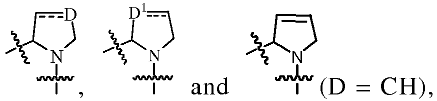 and the synthesis of a group of novel heterocyclic functional group containing linear polypeptide compounds capable of effectively inhibiting HCV, especially novel heterocyclic functional group containing compounds with high selectivity in inhibiting HCV NS5A.
2) The compounds of this invention are advantageous for its obvious HCV NS5A inhibitory activity, this invention also further develops and optimizes the structure of multiple novel heterocyclic rings containing linear compounds that effectively inhibit HCV infection.
3) several compound (**6dy** and **6fm**) which, identified in the study of the correlation between HCV NS5A inhibitors' structure and their activities, demonstrate high HCV NS5A inhibitory activity superior to that of known NCEs in clinical trial (e.g.: BMS790052) and low toxicity at high dosage and no observable side effects, thus laying down a solid foundation for the development of a highly effective anti-HCV new drug.
4) The compounds of this invention are mainly for inhibiting HCV NS5A and can be used in composition with one or more drugs to inhibit HCV and other viruses. They are promising in the development of more and better new drugs for the society.

### Detailed Description of the Preferred Embodiments

It should be understood that these embodiments are merely illustrative of the present invention and are not intended that the invention should be limited thereto. Any of the following embodiments that is not provided with specific experiment method and conditions was carried out by Zannan SciTech or other CROs with routine method under conventional conditions or with a method selected in accordance with instructions book of materials or with methods specified in WO2008/021927 A2, WO2010/132601 A1, WO2011/075615 A1 and other references to get key intermediates SM1, SM2, SM3, and SM4 of this invention.

Compounds of this invention may containing tricyclic functional group(s) and one or more heterocyclic rings with an asymmetric center. Therefore, such compounds may be in the form of a mixture of mesomer and racemate, single antimer, and/or tautomer. Compounds 6a-6ax(**Ia**) prepared in the invention are chiral heterocyclic compounds; the optical purity of natural amino acids and non-natural amino acids in products is determined by polarimeter and/or chromatographic column. The structural characterization of every final products(including compounds **6a-6gq** and the following reference compounds: **Ref-1**(BMS790052), **Ref-2**(GS5885), **Ref-3, Ref-4(DIX-719))** is done by LC-MS and ¹H-NMR analysis.

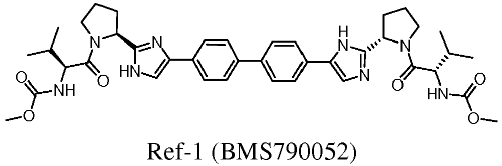

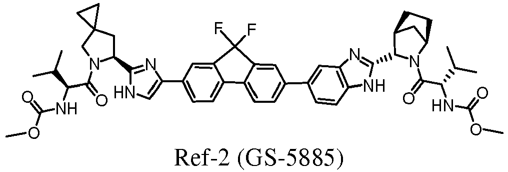

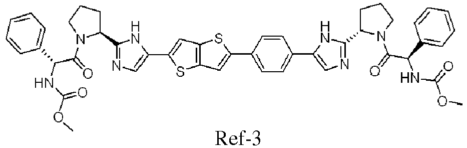

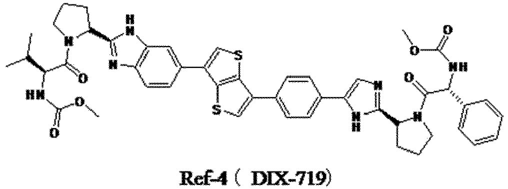

The synthesis and effects of the compounds and intermediates of this invention are illustrated with the following embodiments.
Apparatuses and raw materials used in the embodiments are as follows:
IR spectra data are obtained with Fourier Transform AVATAR™ 360 E.S.P.™ IR spectrometer (Thermo Nicolet) and represented in cm⁻¹.
1HNMR spectra are obtain with Varian Mercury Plus NMR analyzer at 400 or 500 MHz. Chemical shift is recorded in ppm with tetramethylsilane(TMS) as internal standard(CHCl₃: δ= 7.26 ppm). The recorded data include the following: chemical shift and its splitting constants and coupling constants(s: singlet; d : doublet; t: triplet;
q: quartet; br: broad peak; m: multiplet).
Unless otherwise specified, MS data are analyzed with LS-MS (Finnigan LCQ Advantage); all reactions are carried out in argon atmosphere under anhydrous and anaerobic conditions. Solid metal organic compounds are stored in drier under argon atmosphere.
Tetrahydrofuran and ether are added natrium and benzophenone and then distilled. Dichlormethane(DCM), pentane and hexane are subjected to calcium hydride. The special raw materials and intermediates used in the invention are ordered from and provided by Zannan SciTech, all other chemical reagents are purchased from Shanghai Reagent Company, Aldrich, Acros, and/or other reagent suppliers. Where the amount of any intermediate or product is insufficient for the next step experiment during the synthesis process, such intermediate or product will be synthesized repetitively until sufficient amount is obtained. EC₅₀t tests and MTD tests are carried out by WuXi AppTec and or other CROs for the compounds prepared in the invention.
Key to Abbreviations of chemical materials, reagents and solvents used in the invention and its embodiments:
AIBN: Azobisisobutyronitrile
Boc: Butoxylcarbonyl
(BOC)₂O: Di-tert-butyl pyrocarbonate
CDI: N,N'-carbonyldiimidazole
DBU: 1,8-Diazabicyclo[5.4.0]undec-7-ene
EDCI: N-(3-dimethylaminopropyl)-N'-ethylcarbodiimide hydrochloride
HATU: 2-(7-Aza-1H-benzotriazole-1-yl)-N,N,N',N'-tetramethyluronium hexafluorophosphate
NBS: N-bromo-succinimide
DMAP: 4-Dimethylaminopyridine
DIEA: N,N-Diisopropylethylamine
SOCl₂: Thionyl chloride
Pd/C: Palladium carbon
HMTA: Hexamethylenetetramine
HOAc: Glacial acetic acid
HBr: Hydrobromic acid
HCl: Hydrochloric acid
TFA: Trifluoroacetic acid
TsOH: para-Toluenesulfonic acid
K₂CO₃: Potassium carbonate
ACN: Acetonitrile
DCM: Dichlormethane
DMF: N,N-dimethylformamide
DMSO: Dimethyl sulfoxide
Et₂O: Diethyl ether
EA: Acetoacetate
PE: Petroleum ether
THF: Tetrahydrofuran
TBME: tert-Butyl Methyl Ether

### Embodiment 1 (not of the invention)

### Synthesis of compound 6a

**SM-3a** (0.11g, 0.24mmol) and **SM-4i** (0.168g, 0.24mmol, 1.0eq.) were dissolved in 5mL of DMF, added potassium carbonate (0.1g, 0.72mmol, 3.0eq.) and water (3mL) under stirring and nitrogen gas, heated to 100°C, then added tetrakis(triphenylphosphine)palladium (0.01g) in one go, allowed to react thoroughly at 100°C under stirring. When HPLC analysis showed that the reactants had reacted completely, the reaction liquid was filtered, added water and acetoacetate for extraction; the organic phase was combined, rinsed with salt solution, dried with dessicant, and finally separated and purified by column chromatography to get a yellow solid product **6a** (68mg), yield: 30%.
Product **6a**'s ¹HNMR (300MHz, CDC13) spectrum: δ 7.49-7.84 (m, 8H), 7.22-7.24 (m, 2H), 6.65-6.78 (m, 2H), 5.98-5.99 (m, 2H), 5.51-5.55 (m, 2H), 5.43-5.51 (m, 2H), 5.27-5.31 (m, 1H), 4.60-4.72 (m, 4H), 4.12-4.38 (m, 3H), 3.85-3.91 (m, 1H), 3.64-3.74 (m, 4H), 3.49 (s, 3H), 2.54-2.61 (m, 1H), 2.36-2.42 (m, 1H), 1.91-2.28 (m, 5H), 0.85-0.91 (m, 12H). MS analysis confirms that **6a'**s ESI-MS [(M+H)⁺]: theoretical m/z: 944.4; measured value: 944.5.

### Embodiment 2 (not of the invention)

### Synthesis of compound 6b

The synthesis method of compound **6b** was the same with that in Embodiment 1; the product was carried out by one-step catalytic coupling reaction to get product **6b**, wherein compounds **SM-3c** (0.24mmol) and **SM-4j** (0.24mmol) were used in place of compounds **SM-3a** and **SM-4i,** a yellow solid product **6b** (0.062g) was obtained, yield: 25%.
¹H NMR (500 MHz, CDC13) of product **6b**: δ 7.48-7.84 (m, 8H), 6.66-6.77 (m, 2H), 5.98 (m, 2H), 5.14-5.57 (m, 5H), 4.60-4.72 (m, 4H), 4.13-4.32 (m, 3H), 3.84 (m, 2H), 3.71 (m, 1H), 3.37 (m, 1H), 2.58 (m, 1H), 1.93-2.36 (m, 8H), 1.25-1.45 (m, 20H), 0.87-1.13 (m, 12H). MS analysis confirms that **6b**'s ESI-MS [(M+H)⁺]:: theoretical m/z: 1028.5; measured value: 1028.6.

### Embodiment 3 (not of the invention)

### Synthesis of compound 6c

The synthesis method of compound **6c** was the same with that in Embodiment 1; the product was carried out by one-step catalytic coupling reaction to get product **6c**, wherein compounds **SM-3e** (0.24mmol) and **SM-4k** (0.24mmol) were used in place of compounds **SM-3a** and **SM-4i,** a yellow solid product **6c** (0.078g) was obtained, yield: 31%.
MS analysis confirms that **6c'**s ESI-MS [(M+H)⁺]:: theoretical m/z: 1056.6; measured value: 1056.7.

### Embodiment 4 (not of the invention)

### Synthesis of compound 6d

The synthesis method of compound **6d** was the same with that in Embodiment 1; the product was carried out by one-step catalytic coupling reaction to get product **6d**, wherein compounds **SM-3a** (0.29mmol) and **SM-4j** (0.29mmol) were used in place of compounds **SM-3a** and **SM-4i,** a yellow solid product **6d** (0.16g) was obtained, yield: 57%.
¹H NMR (300 MHz, CDC13) of product **6d**: δ 7.31-7.79 (m, 8H), 7.22-7.27 (m, 2H), 6.66-6.78 (m, 2H), 5.98-5.99 (m, 2H), 5.28-5.56 (m, 4H), 4.62-4.69 (m, 4H), 4.20-4.59 (m, 3H), 3.88-3.97 (m, 1H), 3.62-3.75 (m, 4H), 1.78-2.01 (m, 8H), 1.36-1.46 (m, 9H), 0.89-0.94 (m, 12H). MS analysis confirms that **6d'**s ESI-MS [(M+H)⁺]:: theoretical m/z: 986.5; measured value: 986.6.

### Embodiment 5 (not of the invention)

### Synthesis of compound 6e

The synthesis method of compound **6e** was the same with that in Embodiment 1; the product was carried out by one-step catalytic coupling reaction to get product **6e**, wherein compounds **SM-3i** (0.14mmol) and **SM-4j** (0.14mmol) were used in place of compounds **SM-3a** and **SM-4i,** a yellow solid product **6e** (0.048g) was obtained, yield: 30%.
¹H NMR (500 MHz, CDC13) of product **6e**: δ 7.82 (brs, 2H), 7.50-7.61 (m, 6H), 6.66-6.78 (m, 4H), 5.98 (s, 2H), 5.97 (s, 2H), 5.55 (brs, 2H), 5.39-5.46 (m, 4H), 4.60-4.74 (m, 8H), 4.21-4.25 (m, 4H), 3.84-3.85 (m, 2H), 3.49 (s, 6H), 2.57 (m, 2H), 1.93-1.94 (m, 2H), 1.73 (m, 4H), 1.32 (m, 1H), 1.12 (m, 1H), 0.82-0.88 (m, 12H). MS analysis confirms that **6e'**s ESI-MS [(M+H)⁺]:: theoretical m/z: 1149.5; measured value: 1149.6.

### Embodiment 6 (not of the invention)

### Synthesis of compound 6f

The synthesis method of compound **6f** was the same with that in Embodiment 1; the product was carried out by one-step catalytic coupling reaction to get product **6f**, wherein compounds **SM-3j** (0.23mmol) and **SM-4j** (0.23mmol) were used in place of compounds **SM-3a** and **SM-4i,** a yellow solid product **6f** (0.12g) was obtained, yield: 42.3%.
¹H NMR (500 MHz, CDC13) of product **6f**: δ 7.62-7.83 (m, 8H), 6.68-6.78 (m, 4H), 5.96-5.98 (m, 4H), 5.55 (s, 2H), 5.47 (s, 2H), 5.15 (m, 2H), 4.61-4.72 (m, 8H), 4.12-4.22 (m, 4H), 3.85 (m, 2H), 3.49 (s, 6H), 2.58 (m, 2H), 1.74-1.92 (m, 4H), 1.25-1.35 (m, 20H), 1.12 (m, 2H), 0.84 (s, 12H). MS analysis confirms that **6f'**s ESI-MS [(M+H)⁺]:: theoretical m/z: 1233.6; measured value: 1233.6.

### Embodiment 7 (not of the invention)

### Synthesis of compound 6g

The synthesis method of compound **6g** was the same with that in Embodiment 1; the product was carried out by one-step catalytic coupling reaction to get product **6g**, wherein compounds **SM-3m** (0.08mmol) and **SM-4m** (0.08mmol) were used in place of compounds **SM-3a** and **SM-4i**, a yellow solid product **6g** (0.013g) was obtained, yield: 14%.
¹H NMR (CD₃OD, 400 MHz) of product **6g**: δ 7.38-7.34 (m, 1H), 7.00-6.96 (m, 2H), 6.11-6.03 (m, 1H), 5.43-5.39 (m, 1H), 5.29-5.27 (m, 1H), 4.65-4.64 (m, 2H), 4.62 (s, 2H) ,4.57 (s, 2H). MS analysis confirms that **6g'**s ESI-MS [(M+H)⁺]:: theoretical m/z: 1257.6; measured value: 1257.6.

### Embodiment 8 (not of the invention)

### Synthesis of compound 6h

The synthesis method of compound **6h** was the same with that in Embodiment 1; the product was carried out by one-step catalytic coupling reaction to get product **6h**, wherein compounds **SM-3g** (0.05mmol) and **SM-4m** (0.05mmol) were used in place of compounds **SM-3a** and **SM-4i,** a yellow solid product **6h** (0.01g) was obtained, yield: 20%.
¹H NMR (CD₃OD, 400 MHz) of product **6h**: δ 7.38-7.34 (m, 1H), 7.00-6.96 (m, 2H), 6.11-6.03 (m, 1H), 5.43-5.39 (m, 1H), 5.29-5.27 (m, 1H), 4.65-4.64 (m, 2H), 4.62 (s, 2H), 4.57 (s, 2H). MS analysis confirms that **6h'**s ESI-MS [(M+H)⁺]:: theoretical m/z: 1052.5; measured value: 1052.6.

### Embodiment 9 (not of the invention)

### Synthesis of compound 6i

The synthesis method of compound **6i** was the same with that in Embodiment 1; the product was carried out by one-step catalytic coupling reaction to get product **6i**, wherein compounds **SM-3a** (0.19mmol) and **SM-4n** (0.19mmol) were used in place of compounds **SM-3a** and **SM-4i,** a yellow solid product **6i** (0.10g) was obtained, yield: 55%.
¹H NMR (500 MHz, CDC13) of product **6i**: δ 7.62-7.83 (m, 8H), 6.72 (s, 1H), 6.66 (s, 1H), 5.97 (s, 2H), 5.44-5.54 (m, 4H), 5.28 (m, 1H), 4.57-4.69 (m, 4H), 4.34 (m, 1H), 4.25 (m, 1H), 4.17 (m, 1H), 3.83-3.86 (m, 2H), 3.74-3.76 (m, 1H), 3.70 (s, 3H), 3.65 (m, 1H), 3.50 (s, 3H), 2.57 (m, 1H), 2.36 (m, 1H), 2.20 (m, 1H), 2.09-2.10 (m, 1H), 1.79-1.98 (m, 5H), 1.04-1.16 (m, 2H), 0.84-0.89 (m, 12H). MS analysis confirms that **6i'**s ESI-MS [(M+H)⁺]:: theoretical m/z: 944.4; measured value: 944.5.

### Embodiment 10 (not of the invention)

### Synthesis of compound 6j

The synthesis method of compound **6j** was the same with that in Embodiment 1; the product was carried out by one-step catalytic coupling reaction to get product **6j**, wherein compounds **SM-3c** (0.19mmol) and **SM-4p** (0.19mmol) were used in place of compounds **SM-3a** and **SM-4i**, a yellow solid product **6j** (0.04g) was obtained, yield: 20%.
¹H NMR (500 MHz, CDCl₃) of product **6j**: δ 7.83 (m, 2H), 7.51-7.64 (m, 6H), 6.72 (s, 1H), 6.64 (s, 1H), 5.97 (m, 2H), 5.14-5.56 (m, 5H), 4.55-4.67 (m, 4H), 4.13-4.31 (m, 3H), 3.82 (m, 2H), 3.48-3.60 (m, 2H), 2.57 (m, 1H), 2.32 (m, 1H), 1.72-2.07 (m, 7H), 1.08-1.32 (m, 20H), 0.84-0.90 (m, 12H). MS analysis confirms that **6j'**s ESI-MS [(M+H)⁺]: theoretical m/z: 1028.5; measured value: 1028.6.

### Embodiment 11 (not of the invention)

### Synthesis of compound 6k

The synthesis method of compound **6k** was the same with that in Embodiment 1; the product was carried out by one-step catalytic coupling reaction to get product **6k**, wherein compounds **SM-3e** (0.21mmol) and **SM-4q** (0.21mmol) were used in place of compounds **SM-3a** and **SM-4i,** a yellow solid product **6k** (0.045g) was obtained, yield: 20%.
¹H NMR (500 MHz, CDCl₃) of product **6k**: δ 7.62-7.81 (m, 8H), 6.71 (s, 1H), 6.62 (s, 1H), 5.97 (s, 2H), 5.16-5.50 (m, 5H), 4.58-4.66 (m, 4H), 4.28-4.35 (m, 2H), 4.21-4.23 (d, J = 9.5 Hz, 1H), 3.90 (m, 1H), 3.78 (m, 1H), 3.66 (m, 1H), 3.42 (m, 1H), 2.58 (m, 1H), 2.34 (m, 1H), 2.01-2.09 (m, 2H), 1.49-1.64 (m, 5H), 1.32 (s, 9H), 1.26 (s, 9H), 0.82-0.93 (m, 18H). MS analysis confirms that **6k'**s ESI-MS [(M+H)⁺]: theoretical m/z: 1056.6; measured value: 1056.7.

### Embodiment 12 (not of the invention)

### Synthesis of compound 6m

The synthesis method of compound **6m** was the same with that in Embodiment 1; the product was carried out by one-step catalytic coupling reaction to get product **6m**, wherein compounds **SM-3a** (0.38mmol) and **SM-4p** (0.38mmol) were used in place of compounds **SM-3a** and **SM-4i**, a yellow solid product **6m** (0.3g) was obtained, yield: 79%.
¹H NMR (500 MHz, CDCl₃) of product **6m**: δ 7.58-7.82 (m, 8H), 6.71 (s, 1H), 6.64 (s, 1H), 5.97 (s, 2H), 5.46-5.55 (m, 3H), 5.18-5.28 (m, 2H), 4.56-4.66 (m, 4H), 4.35 (m, 1H), 4.15-4.24 (m, 2H), 3.84-3.89 (m, 2H), 3.67-3.75 (m, 5H), 2.58 (m, 1H), 2.37 (m, 1H), 2.22 (m, 1H), 2.10 (m, 1H), 1.91-2.05 (m, 3H), 1.36 (s, 9H), 1.07-1.13 (m, 4H), 0.84-0.90 (m, 12H)). MS analysis confirms that **6m'**s ESI-MS [(M+H)⁺]: theoretical m/z: 986.5; measured value: 986.6.

### Embodiment 13 (not of the invention)

### Synthesis of compound 6n

The synthesis method of compound **6n** was the same with that in Embodiment 1; the product was carried out by one-step catalytic coupling reaction to get product **6n** wherein compounds **SM-3n** (0.24mmol) and **SM-4n** (0.24mmol) were used in place of compounds **SM-3a** and **SM-4i**, a yellow solid product **6n** (0.054 obtained, yield: 19.3
¹H NMR (500 MHz, CDCl₃) of product **6n**: δ 7.83 (brs, 2H), 7.50-7.63 (m, 6H), 6.72 (s, 2H), 6.66 (s, 2H), 5.97 (s, 4H), 5.36-5.54 (m, 6H), 4.57-4.68 (m, 8H), 4.24-4.27 (m, 2H), 4.16-4.19 (m, 2H), 3.84-3.85 (m, 2H), 3.51 (s, 6H), 2.55-2.59 (m, 2H), 1.92-1.94 (m, 2H), 1.66-1.68 (m, 4H), 1.32 (m, 1H), 1.12 (m, 1H), 0.84-0.88 (m, 12H). MS analysis confirms that **6n'**s ESI-MS [(M+H)⁺]: theoretical m/z: 1149.5; measured value: 1149.6

### Embodiment 14 (not of the invention)

### Synthesis of compound 6p

The synthesis method of compound **6p** was the same with that in Embodiment 1; the product was carried out by one-step catalytic coupling reaction to get product **6p** wherein compounds **SM-3p** (0.32mmol) and **SM-4p** (0.32mmol) were used in place of compounds **SM-3a** and **SM-4i**, a yellow solid product **6p** (0.20g) obtained, yield: 50%
¹H NMR (500 MHz, CDCl₃) of product **6p**: δ 7.83 (brs, 2H), 7.51-7.63 (m, 6H), 6.71 (s, 2H), 6.64 (s, 2H), 5.97 (s, 4H), 5.48-5.54 (m, 4H), 5.17 (m, 2H), 4.55-4.66 (m, 8H), 4.14-4.22 (m, 4H), 3.59-3.84 (m, 2H), 2.58 (m, 2H), 1.69-2.05 (m, 6H), 1.26-1.36 (m, 20H), 0.84-0.90 (m, 12H). MS analysis confirms that **6p'**s ESI-MS [(M+H)⁺]: theoretical m/z: 1233.6; measured value: 1233.6

### Embodiment 15 (not of the invention)

### Synthesis of compound 6q

The synthesis method of compound **6q** was the same with that in Embodiment 1; the product was carried out by one-step catalytic coupling reaction to get product **6q** wherein compounds **SM-3r** (0.16mmol) and **SM-4r** (0.16mmol) were used in place of compounds **SM-3a** and **SM-4i**, a yellow solid product **6q** (0.02g) obtained, yield: 10%
¹H NMR (500 MHz, CDC13) of product **6q**: δ 7.83-7.84 (m, 2H), 7.52-7.63 (m, 6H), 6.72 (s, 2H), 6.65 (s, 2H), 5.97 (s, 4H), 5.43-5.53 (m, 4H), 5.21 (m, 2H), 4.57-4.77 (m, 8H), 4.29 (m, 4H), 3.80-3.82 (m, 2H), 3.49 (m, 2H), 2.57 (m, 2H), 1.88-1.91 (m, 2H), 1.59-1.70 (m, 16H), 1.12-1.33 (m, 6H), 0.81-0.85 (m, 12H) MS analysis confirms that **6q'**s ESI-MS [(M+H)⁺]: theoretical m/z: 1257.6; measured value: 1257.7

### Embodiment 16 (not of the invention)

### Synthesis of compound 6r

The synthesis method of compound **6r** was the same with that in Embodiment 1; the product was carried out by one-step catalytic coupling reaction to get product **6r** wherein compounds **SM-3g** (0.09mmol) and **SM-4r** (0.09mmol) were used in place of compounds **SM-3a** and **SM-4i**, a yellow solid product **6r** (0.044g) obtained, yield: 47.8%
¹H NMR (500 MHz, CDCl₃) of product **6r**: δ 7.85-7.84 (m, 2H), 7.60 (m, 6H), 6.72 (s, 1H), 6.66 (s, 1H), 5.97 (s, 2H), 5.54 (m, 2H), 5.10-5.31 (m, 5H), 4.57-4.78 (m, 4H), 4.22-4.34 (m, 3H), 3.86 (m, 2H), 3.68 (m, 1H), 3.15-3.46 (m, 1H), 2.58 (m, 1H), 2.36 (m, 1H), 2.22-2.24 (m, 2H), 1.99-2.11 (m, 5H), 1.15-1.50 (m, 18H), 0.74-0.90 (m, 12H) MS analysis confirms that **6r'**s ESI-MS [(M+H)⁺]: theoretical m/z: 1052.5; measured value: 1052.6

### Embodiment 17 (not of the invention)

### Synthesis of compound 6s

The synthesis method of compound **6s** was the same with that in Embodiment 1; the product was carried out by one-step catalytic coupling reaction to get product **6s** wherein compounds **SM-3a** (0.17mmol) and **SM-4s** (0.17mmol) were used in place of compounds **SM-3a** and **SM-4i**, a yellow solid product **6s** (0.05g) obtained, yield: 31%
¹H NMR (500 MHz, CDCl₃) of product **6s**: δ 7.85-7.84 (m, 2H), 7.60 (m, 6H), 6.72 (s, 1H), 6.66 (s, 1H), 5.97 (s, 2H), 5.54 (m, 2H), 5.10-5.31 (m, 5H), 4.57-4.78 (m, 4H), 4.22-4.34 (m, 3H), 3.86 (m, 2H), 3.68 (m, 1H), 3.15-3.46 (m, 1H), 2.58 (m, 1H), 2.36 (m, 1H), 2.22-2.24 (m, 2H), 1.99-2.11 (m, 5H), 1.15-1.50 (m, 18H), 0.74-0.90 (m, 12H) MS analysis confirms that **6s**'s ESI-MS [(M+H)⁺]: theoretical m/z: 918.4; measured value: 918.5

### Embodiment 18 (not of the invention)

### Synthesis of compound 6t

The synthesis method of compound **6t** was the same with that in Embodiment 1; the product was carried out by one-step catalytic coupling reaction to get product **6t** wherein compounds **SM-3c** (0.38mmol) and **SM-4t** (0.38mmol) were used in place of compounds **SM-3a** and **SM-4i,** a yellow solid product **6t** (0.25g) obtained, yield: 65%
¹H NMR (500 MHz, CDCl₃) of product **6t**: δ 7.72-7.82 (m, 2H), 7.59 (s, 4H), 6.95-7.07 (m, 3H), 5.48-5.55 (m, 3H), 5.13-5.30 (m, 4H), 4.71-4.81 (m, 4H), 4.20-4.32 (m, 4H), 3.84-3.47 (m, 5H), 2.59-2.59 (m, 1H), 1.89-2.34 (m, 5H), 1.26 (s, 18H), 0.85-0.88 (m, 12H). MS analysis confirms that **6t'**s ESI-MS [(M+H)⁺]: theoretical m/z: 1002.5; measured value: 1002.6

### Embodiment 19 (not of the invention)

### Synthesis of compound 6u

The synthesis method of compound **6u** was the same with that in Embodiment 1; the product was carried out by one-step catalytic coupling reaction to get product **6u** wherein compounds **SM-3a** (0.15mmol) and **SM-4t** (0.15mmol) were used in place of compounds **SM-3a** and **SM-4i,** a yellow solid product **6u** (0.051g) obtained, yield: 35%
¹H NMR (500 MHz, CDCl₃) of product **6u**: δ 7.58 (s, 4H), 7.21-7.23 (m, 1H), 6.95-7.06 (m, 3H), 6.80-6.82 (m, 1H), 5.46-5.53 (m, 3H), 5.23-5.30 (m, 3H), 4.71-4.80 (m, 3H), 4.32-4.33 (m, 1H), 4.19-4.20 (m, 1H), 3.82-3.85 (m, 1H), 3.65-3.74 (m, 4H), 2.94-2.96 (m, 1H), 2.88-2.89 (m, 1H), 2.62 (s, 4H), 2.33-2.34 (m, 1H), 2.18-2.22 (m, 2H), 1.89-2.10 (m, 4H), 1.25-1.31 (m, 9H), 0.83-0.8 (m, 12H). MS analysis confirms that **6u'**s ESI-MS [(M+H)⁺]: theoretical m/z: 960.5; measured value: 960.6

### Embodiment 20 (not of the invention)

### Synthesis of compound 6v

The synthesis method of compound **6v** was the same with that in Embodiment 1; the product was carried out by one-step catalytic coupling reaction to get product **6v** wherein compounds **SM-3g** (0.09mmol) and **SM-4u** (0.09mmol) were used in place of compounds **SM-3a** and **SM-4i,** a yellow solid product **6v** (0.034g) obtained, yield: 37%
¹H NMR (500 MHz, CDCl₃) of product **6v**: δ 7.77-7.82 (m, 3H), 7.54-7.62 (m, 5H), 6.95-7.08 (m, 3H), 6.02-6.05 (m, 1H), 5.83-5.85 (m, 1H), 5.52 (s, 1H), 5.39-5.44 (m, 2H), 5.30-5.32 (m, 1H), 5.22-5.24 (m, 1H), 5.06-5.08 (m, 1H), 4, 68-4.86 (m, 5H), 4.42-4.44 (m, 1H), 4.32-4.36 (m, 1H), 4.24-4.25 (m, 2H), 3.97-4.00 (m, 1H), 3.88-3.91 (m, 1H), 2.66-2.68 (m, 1H), 2.42-2.45 (m, 1H), 2.31-2.34 (m, 1H), 2.19-2.30 (m, 2 H), 2.12-2.18 (m, 1H), 1.63-1.84 (m, 16H), 1.24-1.26 (m, 2H), 1.09-1.16 (m, 4H), 0.86-0.96 (m, 6H). MS analysis confirms that **6v'**s ESI-MS [(M+H)⁺]: theoretical m/z: 1026.5; measured value: 1026.6

### Embodiment 21 (not of the invention)

### Synthesis of compound 6w

The synthesis method of compound **6w** was the same with that in Embodiment 1; the product was carried out by one-step catalytic coupling reaction to get product **6w** wherein compounds **SM-3v** (0.19mmol) and **SM-4a** (0.19mmol) were used in place of compounds **SM-3a** and **SM-4i,** a yellow solid product **6w** (0.07g) obtained, yield: 38%
¹H NMR (500 MHz, CDCl₃) of product **6w**: δ 8.11 (m, 1H), 8.01-8.00 (m, 1H), δ7.84-7.79 (m, 2H), 7.64-7.45 (m, 10H), 7.21-7.13 (m, 3H), 5.61-5.58 (m, 1H), 5.53-5.51 (m, 1H), 5.45-5.43 (m, 1H), 5.27-5.25 (m, 1H), 4.51-4.48 (m, 1H), 4.35-4.27 (m, 2H), 4.13-4.09 (m, 1H), 3.85-3.84(m, 1H), 3.67 (s, 3H), 3.40 (s, 3H), 2.20-2.96 (m, 8H), 0.89-0.83 (m, 12H). MS analysis confirms that **6w'**s ESI-MS [(M+H)⁺]: theoretical m/z: 965.4; measured value: 965.5

### Embodiment 22 (not of the invention)

### Synthesis of compound 6x

The synthesis method of compound **6x** was the same with that in Embodiment 1; the product was carried out by one-step catalytic coupling reaction to get product **6x** wherein compounds **SM-3w** (0.47mmol) and **SM-4a** (0.47mmol) were used in place of compounds **SM-3a** and **SM-4i,** a yellow solid product **6x** (0.16g) obtained, yield: 35%
¹H NMR (500 MHz, CDC13) of product **6x**: δ 8.04-8.02 (m, 1H), δ7.90-7.88 (m, 1H), 7.68-7.52 (m, 11H), 7.36-7.32 (m, 2H), 7.22-7.24 (m, 2H), 5.55-5.48 (m, 3H), 5.28 (m, 2H), 4.42-4.34 (m, 2H), 3.88-3.86(m, 2H), 3.71 (s, 6H), 2.40-2.01 (m, 8H), 0.92-0.89 (m, 12H) MS analysis confirms that **6x'**s ESI-MS [(M+H)⁺]: theoretical m/z: 965.4; measured value: 965.5

### Embodiment 23 (not of the invention)

### Synthesis of compound 6y

The synthesis method of compound **6y** was the same with that in Embodiment 1; the product was carried out by one-step catalytic coupling reaction to get product **6y** wherein compounds **SM-3x** (0.51mmol) and **SM-4a** (0.51mmol) were used in place of compounds **SM-3a** and **SM-4i,** a yellow solid product **6y** (0.07g) obtained, yield: 19%
¹H NMR (500 MHz, CDC13) of product **6y**: δ 7.68-7.47 (m, 7H), 7.33-7.18 (m, 3H), 5.54-5.53 (m, 1H), 5.35-5.25 (m, 2H), 4.35-4.30 (m, 1H), 3.87-3.85 (m, 1H), 3.76-3.69 (m, 6H), 3.30(m, 1H), 2.91 (m, 1H), 2.38-2.35 (m, 2H), 2.34-1.92 (m, 7H), 1.38-1.20 (m, 12H), 0.95-0.85 (m, 6H). MS analysis confirms that **6y'**s ESI-MS [(M+H)⁺]: theoretical m/z: 723.4; measured value: 723.5

### Embodiment 24 (not of the invention)

### Synthesis of compound 6z

The synthesis method of compound **6z** was the same with that in Embodiment 1; the product was carried out by one-step catalytic coupling reaction to get product **6z** wherein compounds **SM-3y** (0.54mmol) and **SM-4a** (0.54mmol) were used in place of compounds **SM-3a** and **SM-4i**, a yellow solid product **6z** (0.204g) obtained, yield: 50%
¹H NMR (500 MHz, CDCl₃) of product **6z**: δ 7.76-7.56 (m, 7H), 7.34-7.21 (m, 3H), 5.51-5.26 (m, 3H), 4.34-4.33 (m, 1H), 3.84-3.60 (m, 7H), 3.51 (m, 1H), 2.76-2.74 (m, 1H), 2.40-2.33 (m, 2H), 2.38-1.95 (m, 13H), 1.26-1.23 (m, 4H), 0.93-0.86 (m, 6H). MS analysis confirms that **6z'**s ESI-MS [(M+H)⁺]: theoretical m/z: 751.4; measured value: 751.5

### Embodiment 25 (not of the invention)

### Synthesis of compound 6aa

The synthesis method of compound **6aa** was the same with that in Embodiment 1; the product was carried out by one-step catalytic coupling reaction to get product **6aa** wherein compounds **SM-3z** (0.54mmol) and **SM-4a** (0.54mmol) were used in place of compounds **SM-3a** and **SM-4i,** a yellow solid product **6aa** (0.142g) obtained, yield: 34%
¹H NMR (500 MHz, CDCl₃) of product **6aa**: δ 7.82-7.49 (m, 6H), 7.34-7.19 (m, 4H), 5.54-5.49 (m, 1H), 5.36-5.27 (m, 1H), 4.37-4.28 (m, 1H), 3.57-3.55 (m, 6H), 2.98 (m, 1H), 2.34-2.33 (m, 2H), 2.27-1.57 (m, 12H), 1.44-1.21 (m, 8H), 0.94-0.87 (m, 6H). MS analysis confirms that **6aa'**s ESI-MS [(M+H)⁺]: theoretical m/z: 765.4; measured value: 765.5

### Embodiment 26 (not of the invention)

### Synthesis of compound 6ab

The synthesis method of compound **6ab** was the same with that in Embodiment 1; the product was carried out by one-step catalytic coupling reaction to get product **6ab** wherein compounds **SM-3aa** (7.36mmol) and **SM-4a** (7.36mmol) were used in place of compounds **SM-3a** and **SM-4i**, a yellow solid product **6ab** (3.6g) obtained, yield: 65%
¹H NMR (500 MHz, CDCl₃) of product **6ab**: δ 7.85-7.76 (m, 2H), 7.67-7.56 (m, 5H), 7.40-7.37 (m, 2H), 7.22-7.16 (m, 1H), 5.51-5.45 (m, 2H), 5.40-5.30 (m, 2H), 4.45-4.36 (m, 2H), 3.88-3.86(m, 2H), 3.71 (s, 6H), 2.87-2.85 (m, 1H), 2.51-1.74 (m, 11H), 1.10-0.80 (m, 12H). MS analysis confirms that **6ab'**s ESI-MS [(M+H)⁺]: theoretical m/z: 753.4; measured value: 753.5

### Embodiment 27 (not of the invention)

### Synthesis of compound 6ac

The synthesis method of compound **6ac** was the same with that in Embodiment 1; the product was carried out by one-step catalytic coupling reaction to get product **6ac** wherein compounds **SM-3aa** (0.19mmol) and **SM-4n** (0.19mmol) were used in place of compounds **SM-3a** and **SM-4i**, a yellow solid product **6ac** (0.08g) obtained, yield: 42%
¹H NMR (500 MHz, CDCl₃) of product **6ac**: δ 7.81-7.45 (m, 8H), 7.37-7.22 (m, 4H), 6.72-6.62 (m, 2H), 5.97-5.93 (m, 2H), 5.55-5.35 (m, 3H), 4.71-4.57 (m, 4H), 4.26-4.12 (m, 2H), 3.77-3.70 (m, 3H), 3.51-3.43 (m, 3H), 2.83 (m, 1H), 2.57-2.47 (m, 2H), 2.07-1.77 (m, 9H), 1.12-1.11 (m, 6H), 0.84-0.82 (m, 6H). MS analysis confirms that **6ac'**s ESI-MS [(M+H)⁺]: theoretical m/z: 958.4; measured value: 958.5

### Embodiment 28 (not of the invention)

### Synthesis of compound 6ad

The synthesis method of compound **6ad** was the same with that in Embodiment 1; the product was carried out by one-step catalytic coupling reaction to get product **6ad** wherein compounds **SM-3aa** (0.20mmol) and **SM-4i** (0.20mmol) were used in place of compounds **SM-3a** and **SM-4i,** a yellow solid product **6ad** (0.064g) obtained, yield: 33%
¹H NMR (500 MHz, CDCl₃) of product **6ad**: δ 7.80-7.46 (m, 8H), 7.37-7.22 (m, 4H), 6.78-6.66 (m, 2H), 5.98-5.97 (m, 2H), 5.56-5.34 (m, 3H), 4.75-4.59 (m, 4H), 4.25-4.17 (m, 2H), 3.86-3.64 (m, 3H), 3.49-3.46 (m, 3H), 2.82 (m, 1H), 2.58-2.47 (m, 2H), 2.08-1.76 (m, 9H), 1.12-1.11 (m, 6H), 0.86-0.84 (m, 6H). MS analysis confirms that **6ad'**s ESI-MS [(M+H)⁺]: theoretical m/z: 958.4; measured value: 958.5

### Embodiment 29 (not of the invention)

### Synthesis of compound 6ae

The synthesis method of compound **6ae** was the same with that in Embodiment 1; the product was carried out by one-step catalytic coupling reaction to get product **6ae** wherein compounds **SM-3a** (0.13mmol) and **SM-4ac** (0.13mmol) were used in place of compounds **SM-3a** and **SM-4i,** a yellow solid product **6ae** (0.021g) obtained, yield: 17%
¹H NMR (500 MHz, CDCl₃) of product **6ae**: δ 7.81-7.55 (m, 8H), 7.34-7.22 (m, 4H), 6.80-6.69 (m, 2H), 5.99-5.97 (m, 1H), 5.57-5.56 (m, 1H), 5.32-5.17 (m, 2H), 4.93-4.72 (m, 4H), 4.35-4.25 (m, 2H), 3.74-3.69 (m, 6H), 2.96 (m, 1H), 2.37-2.36 (m, 1H), 2.24-1.76 (m, 8H), 1.16-0.79 (m, 12H). MS analysis confirms that **6ae'**s ESI-MS [(M+H)⁺]: theoretical m/z: 929.4; measured value: 929.5

### Embodiment 30 (not of the invention)

### Synthesis of compound 6af

The synthesis method of compound **6af** was the same with that in Embodiment 1; the product was carried out by one-step catalytic coupling reaction to get product **6af** wherein compounds **SM-3a** (0.16mmol) and **SM-4ad** (0.16mmol) were used in place of compounds **SM-3a** and **SM-4i,** a yellow solid product **6af** (0.015g) obtained, yield: 10%
¹H NMR (500 MHz, CDCl₃) of product **6af**: δ 7.77-7.54 (m, 8H), 7.28-7.22 (m, 2H), 6.73-6.68 (m, 2H), 6.00-5.98 (m, 2H), 5.61-5.46 (m, 2H), 5.35-5.22 (m, 2H), 4.85-4.75 (m, 4H), 4.365-4.10 (m, 2H), 3.72-3.70 (m, 6H), 2.95 (m, 1H), 2.39 (m, 1H), 2.03-1.81 (m, 8H), 1.10-0.90 (m, 12H). MS analysis confirms that **6af'**s ESI-MS [(M+H)⁺]: theoretical m/z: 929.4; measured value: 929.5

### Embodiment 31 (not of the invention)

### Synthesis of compound 6ag

The synthesis method of compound **6ag** was the same with that in Embodiment 1; the product was carried out by one-step catalytic coupling reaction to get product **6ag** wherein compounds **SM-3ab** (0.24mmol) and **SM-4a** (0.24mmol) were used in place of compounds **SM-3a** and **SM-4i**, a yellow solid product **6ag** (0.15g) obtained, yield: 65%
¹H NMR (500 MHz, CDCl₃) of product **6ag**: δ 7.83-7.53 (m, 7H), 7.47-7.19 (m, 3H), 5.50-5.48 (m, 1H), 5.27-5.26 (m, 1H), 5.08-5.03 (m, 1H), 4.54-4.48 (m, 1H), 4.40-4.33 (m, 1H), 4.01-3.82 (m, 3H), 3.70 (m, 6H), 2.95-2.90 (m, 1H), 2.38-2.37 (m, 1H), 2.23-1.83 (m, 8H), 1.27-1.11 (m, 6H), 0.97-0.86 (m, 6H). MS analysis confirms that **6ag'**s ESI-MS [(M+H)⁺]: theoretical m/z: 755.4; measured value: 755.5

### Embodiment 32 (not of the invention)

### Synthesis of compound 6ah

The synthesis method of compound **6ah** was the same with that in Embodiment 1; the product was carried out by one-step catalytic coupling reaction to get product **6ah** wherein compounds **SM-3ab** (0.24mmol) and **SM-4n** (0.24mmol) were used in place of compounds **SM-3a** and **SM-4i**, a yellow solid product **6ah** (0.033g) obtained, yield: 14.2%
¹H NMR (500 MHz, CDC13) of product **6ah:** δ 7.80-7.59 (m, 8H), 7.27 (m, 2H), 6.71-6.65 (m, 2H), 5.96 (s, 2H), 5.46-5.38 (m, 3H), 5.08-5.03 (m, 1H), 4.68-4.53 (m, 5H), 3.79-3.70 (m, 3H), 3.57-3.50 (m, 3H), 2.91-2.84 (m, 2H), 2.15-1.88 (m, 10H), 1.26-1.11 (m, 6H), 0.93-0.86 (m, 6H). MS analysis confirms that **6ah'**s ESI-MS [(M+H)⁺]: theoretical m/z: 960.4; measured value: 960.5

### Embodiment 33 (not of the invention)

### Synthesis of compound 6ai

The synthesis method of compound **6ai** was the same with that in Embodiment 1; the product was carried out by one-step catalytic coupling reaction to get product **6ai** wherein compounds **SM-3ab** (0.03mmol) and **SM-4i** (0.03mmol) were used in place of compounds **SM-3a** and **SM-4i,** a yellow solid product **6ai** (0.075g) obtained, yield: 25%
¹H NMR (500 MHz, CDCl₃) of product **6ai**: δ 7.80-7.58 (m, 8H), 7.28-7.23 (m, 2H), 6.78-6.66 (m, 2H), 5.98-5.97 (m, 2H), 5.47-5.37 (m, 3H), 5.08-5.04 (m, 1H), 4.75-4.53 (m, 5H), 4.24-4.21 (m, 2H), 3.79-3.65 (m, 3H), 3.57-3.49 (m, 3H), 2.92 (m, 1H), 2.57 (m, 1H), 2.15-1.73 (m, 8H), 1.28-1.11 (m, 6H), 0.83-0.75 (m, 6H). MS analysis confirms that **6ai'**s ESI-MS [(M+H)⁺]: theoretical m/z: 960.4; measured value: 960.5

### Embodiment 34 (not of the invention)

### Synthesis of compound 6aj

The synthesis method of compound **6aj** was the same with that in Embodiment 1; the product was carried out by one-step catalytic coupling reaction to get product **6aj** wherein compounds **SM-3ab** (0.36mmol) and **SM-4aa** (0.36mmol) were used in place of compounds **SM-3a** and **SM-4i,** a yellow solid product **6aj** (0.15g) obtained, yield: 43%
¹H NMR (500 MHz, CDCl₃) of product **6aj**: δ 7.85-7.39 (m, 8H), 5.58-5.54 (m, 1H), 5.41-5.35 (m, 1H), 5.09-5.05 (m, 1H), 4.60 (m, 1H), 4.54-4.40 (m, 2H), 4.31-4.30 (m, 1H), 4.20-4.18 (m, 1H), 4.02 (m, 1H), 3.80 (m, 3H), 3.72-3.43 (m, 3H), 3.04-3.03 (m, 2H), 2.98-2.84 (m, 2H), 2.45 (m, 1H), 2.30 (m, 1H), 1.76-1.62 (m, 2H), 1.49-1.33 (m, 2H), 1.15-1.12 (m, 6H), 0.95-0.87 (m, 6H). MS analysis confirms that **6aj'**s ESI-MS [(M+H)⁺]: theoretical m/z: 769.4; measured value: 769.5

### Embodiment 35 (not of the invention)

### Synthesis of compound 6ak

The synthesis method of compound **6ak** was the same with that in Embodiment 1; the product was carried out by one-step catalytic coupling reaction to get product **6ak**, wherein compounds **SM-3ae** (0.2mmol) and **SM-4n** (0.2mmol) were used in place of compounds **SM-3a** and **SM-4i,** a yellow solid product **6ak** was obtained (yield: 51%).
MS analysis confirms that **6ak's** ESI-MS [(M+H)⁺]: theoretical m/z: 970.4; measured value: 970.6.

### Embodiment 36 (not of the invention)

### Synthesis of compound 6am

The synthesis method of compound **6am** was the same with that in Embodiment 1; the product was carried out by one-step catalytic coupling reaction to get product **6am**, wherein compounds **SM-3ae** (0.2mmol) and **SM-4ad** (0.2mmol) were used in place of compounds **SM-3a** and **SM-4i,** a yellow solid product **6am** was obtained (yield: 53%).
MS analysis confirms that **6am's** ESI-MS [(M+H)⁺]: theoretical m/z: 955.4; measured value: 955.6.

### Embodiment 37 (not of the invention)

### Synthesis of compound 6an

The synthesis method of compound **6an** was the same with that in Embodiment 1; the product was carried out by one-step catalytic coupling reaction to get product **6an**, wherein compounds **SM-3ae** (0.2mmol) and **SM-4i** (0.2mmol) were used in place of compounds **SM-3a** and **SM-4i,** a yellow solid product **6an** was obtained (yield: 52%).
MS analysis confirms that **6an's** ESI-MS [(M+H)⁺]: theoretical m/z: 970.4; measured value: 970.6.

### Embodiment 38 (not of the invention)

### Synthesis of compound 6ap

The synthesis method of compound **6ap** was the same with that in Embodiment 1; the product was carried out by one-step catalytic coupling reaction to get product **6ap**, wherein compounds **SM-3ae** (0.2mmol) and **SM-4ac** (0.2mmol) were used in place of compounds **SM-3a** and **SM-4i,** a yellow solid product **6ap** was obtained (yield: 53%).
MS analysis confirms that **6ap's** ESI-MS [(M+H)⁺]: theoretical m/z: 955.4; measured value: 955.6.

### Embodiment 39 (not of the invention)

### Synthesis of compound 6aq

The synthesis method of compound **6aq** was the same with that in Embodiment 1; the product was carried out by one-step catalytic coupling reaction to get product **6aq**, wherein compounds **SM-3af** (0.2mmol) and **SM-4a** (0.2mmol) were used in place of compounds **SM-3a** and **SM-4i,** a yellow solid product **6aq** was obtained (yield: 53%).
MS analysis confirms that **6aq's** ESI-MS [(M+H)⁺]: theoretical m/z: 885.4; measured value: 885.5.

### Embodiment 40 (not of the invention)

### Synthesis of compound 6ar

The synthesis method of compound **6ar** was the same with that in Embodiment 1; the product was carried out by one-step catalytic coupling reaction to get product **6ar**, wherein compounds **SM-3ag** (0.2mmol) and **SM-4a** (0.2mmol) were used in place of compounds **SM-3a** and **SM-4i**, a yellow solid product **6ar** was obtained (yield: 55%).
MS analysis confirms that **6ar's** ESI-MS [(M+H)⁺]: theoretical m/z: 903.4; measured value: 903.5.

### Embodiment 41 (not of the invention)

### Synthesis of compound 6as

The synthesis method of compound **6as** was the same with that in Embodiment 1; the product was carried out by one-step catalytic coupling reaction to get product **6as**, wherein compounds **SM-3ah** (0.2mmol) and **SM-4a** (0.2mmol) were used in place of compounds **SM-3a** and **SM-4i,** a yellow solid product **6as** was obtained (yield: 54%).
MS analysis confirms that **6as's** ESI-MS [(M+H)⁺]: theoretical m/z: 903.4; measured value: 903.5.

### Embodiment 42 (not of the invention)

### Synthesis of compound 6at

The synthesis method of compound **6at** was the same with that in Embodiment 1; the product was carried out by one-step catalytic coupling reaction to get product **6at**, wherein compounds **SM-3ai** (0.2mmol) and **SM-4a** (0.2mmol) were used in place of compounds **SM-3a** and **SM-4i,** a yellow solid product **6at** was obtained (yield: 51%).
MS analysis confirms that **6at's** ESI-MS [(M+H)⁺]: theoretical m/z: 919.4; measured value: 919.5.

### Embodiment 43 (not of the invention)

### Synthesis of compound 6au

The synthesis method of compound **6au** was the same with that in Embodiment 1; the product was carried out by one-step catalytic coupling reaction to get product **6au**, wherein compounds **SM-3aj** (0.2mmol) and **SM-4a** (0.2mmol) were used in place of compounds **SM-3a** and **SM-4i,** a yellow solid product **6au** was obtained (yield: 52%). MS analysis confirms that **6au's** ESI-MS [(M+H)⁺]: theoretical m/z: 919.4; measured value: 919.5.

### Embodiment 44 (not of the invention)

### Synthesis of compound 6av

The synthesis method of compound **6av** was the same with that in Embodiment 1; the product was carried out by one-step catalytic coupling reaction to get product **6av**, wherein compounds **SM-3am** (0.2mmol) and **SM-4a** (0.2mmol) were used in place of compounds **SM-3a** and **SM-4i**, a yellow solid product **6av** was obtained (yield: 63%).
¹H NMR (500 MHz, CDCl₃) of product **6av**: δ 7.54-7.80 (m, 9H), 7.17-7.22 (m, 3H), 6.76-6.85 (m, 3H), 5.60-5.72 (m, 2H), 5.19-5.44 (m, 4H), 4.82-4.92 (m, 5H), 3.97-4.34 (m, 4H), 3.79-3.82 (m, 3H), 3.68-3.73 (m, 6H), 2.95 (m, 1H), 2.37 (m, 1H), 2.20-2.21 (m, 1H), 1.98-2.11 (m, 4H), 0.88-0.95 (m, 12H). MS analysis confirms that **6av'**s ESI-MS [(M+H)⁺]: theoretical m/z: 915.4; measured value: 915.5

### Embodiment 45 (not of the invention)

### Synthesis of compound 6aw

The synthesis method of compound **6aw** was the same with that in Embodiment 1; the product was carried out by one-step catalytic coupling reaction to get product **6aw**, wherein compounds **SM-3ak** (0.2mmol) and **SM-4a** (0.2mmol) were used in place of compounds **SM-3a** and **SM-4i**, a yellow solid product **6aw** was obtained (yield: 61%).
¹H NMR (500 MHz, CDCl₃) of product **6aw:** δ 7.48-7.80 (m, 9H), 7.16-7.25 (m, 4H), 6.83-6.84 (m, 1H), 6.72-6.73 (m, 1H), 5.70-5.78 (m, 2H), 5.22-5.41 (m, 4H), 4.74-4.98 (m, 5H), 4.28-4.30 (m, 2H), 4.01-4.13 (m, 2H), 3.81 (s, 3H), 3.64-3.66 (m, 6H), 2.92 (m, 1H), 2.38 (m, 1H), 2.17-2.18 (m, 1H), 1.94-2.07 (m, 4H), 0.85-0.91 (m, 12H). MS analysis confirms that **6aw'**s ESI-MS [(M+H)⁺]: theoretical m/z: 915.4; measured value: 915.5

### Embodiment 46 (not of the invention)

### Synthesis of compound 6ax

The synthesis method of compound **6ax** was the same with that in Embodiment 1; the product was carried out by one-step catalytic coupling reaction to get product **6ax**, wherein compounds **SM-3an** (0.2mmol) and **SM-4a** (0.2mmol) were used in place of compounds **SM-3a** and **SM-4i**, a yellow solid product **6ax** was obtained (yield: 54%).
¹H NMR (500 MHz, CDC13) of product **6ax**: δ 7.54-7.84 (m, 9H), 7.23 (s, 1H), 7.20 (s, 1H), 6.79 (s, 2H), 6.74 (s, 1H), 6.81-6.87 (m, 2H), 5.58-5.70 (m, 2H), 5.46 (m, 1H), 5.19-5.34 (m, 3H), 4.72-4.92 (m, 5H), 3.97-4.35 (m, 4H), 3.86-3.89 (m, 6H), 3.69-3.74 (m, 6H), 2.96 (m, 1H), 2.38 (m, 1H), 2.22 (m, 1H), 1.99-2.12 (m, 4H), 0.89-0.96 (m, 12H). MS analysis confirms that **6ax'**s ESI-MS [(M+H)⁺]: theoretical m/z: 945.4; measured value: 945.6

### Embodiment 47 (not of the invention)

### Synthesis of compound 6ay

The synthesis method of compound **6ay** was the same with that in Embodiment 1; the product was carried out by one-step catalytic coupling reaction to get product **6ay** wherein compounds **SM-3ac** (0.2mmol) and **SM-4ag** (0.2mmol) were used in place of compounds **SM-3a** and **SM-4i**, a yellow solid product **6ay** was obtained (yield: 53%).
MS analysis confirms that **6ay'**s ESI-MS [(M+H)⁺]: theoretical m/z: 963.4; measured value: 963.5.

### Embodiment 48 (not of the invention)

### Synthesis of compound 6az

The synthesis method of compound **6az** was the same with that in Embodiment 1; the product was carried out by one-step catalytic coupling reaction to get product **6az** wherein compounds **SM-3n** (0.2mmol) and **SM-4ae** (0.2mmol) were used in place of compounds **SM-3a** and **SM-4i**, a yellow solid product **6az** was obtained (yield: 56%).
MS analysis confirms that **6az's** ESI-MS [(M+H)⁺]: theoretical m/z: 956.4; measured value: 956.5.

### Embodiment 49 (not of the invention)

### Synthesis of compound 6ba

The synthesis method of compound **6ba** was the same with that in Embodiment 1; the product was carried out by one-step catalytic coupling reaction to get product **6ba** wherein compounds **SM-3a** (0.55mmol) and **SM-4b** (0.55mmol) were used in place of compounds **SM-3a** and **SM-4i,** a yellow solid product 6ba (0.13g) obtained, yield: 32%
¹H NMR (500 MHz, CDCl₃) of product **6ba**: δ 7.50-7.63 (m, 6H), 7.16-7.23 (m, 2H), 6.26 (s, 1H), 6.06-6.08 (m, 1H), 5.98 (s, 1H), 5.58-5.59 (m, 2H), 5.24-5.30 (m, 1H), 4.72-4.75 (m, 1H), 4.47-4.49 (m, 1H), 4.28-4.36 (m, 2H), 3.83-3.88 (m, 1H), 3.70 (s, 6H), 2.93-2.94 (m, 1H), 2.34-2.38 (m, 1H), 2.16-2.24 (m, 1H), 1.98-2.11 (m, 4H), 0.83-0.91 (m, 12H). MS analysis confirms that 6ba's ESI-MS [(M+H)⁺]: theoretical m/z: 737.4; measured value: 737.5

### Embodiment 50 (not of the invention)

### Synthesis of compound 6bb

The synthesis method of compound **6bb** was the same with that in Embodiment 1; the product was carried out by one-step catalytic coupling reaction to get product **6bb** wherein compounds **SM-3e** (0.057mmol) and **SM-4f** (0.057mmol) were used in place of compounds **SM-3a** and **SM-4i,** a yellow solid product **6bb** (0.013g) obtained, yield: 27.5%
¹H NMR (500 MHz, CDCl₃) of product **6bb**: δ 7.57-7.72 (m, 4H), 7.16-7.23 (m, 2H), 6.29 (s, 1H), 6.00-6.07 (m, 2H), 5.24-5.36 (m, 3H), 4.75, 4.76 (d, 1H), 4.45-4.57 (m, 2H), 4.27-4.36 (m, 2H), 3.88 (s, 1H), 3.67-3.68 (m, 1H), 2.20-2.34 (m, 2H), 1.99-2.09 (m, 2H), 1.46 (s, 18H), 0.93 (m, 18H). MS analysis confirms that **6bb'**s ESI-MS [(M+H)⁺]: theoretical m/z: 849.5; measured value: 849.6

### Embodiment 51 (not of the invention)

### Synthesis of compound 6bc

The synthesis method of compound **6bc** was the same with that in Embodiment 1; the product was carried out by one-step catalytic coupling reaction to get product **6bc** wherein compounds **SM-3a** (0.31mmol) and **SM-4d** (0.31mmol) were used in place of compounds **SM-3a** and **SM-4i,** a yellow solid product **6bc** (0.048g) obtained, yield: 20%
¹H NMR (500 MHz, CDCl₃) of product **6bc**: δ 7.46-7.54 (m, 4H), 7.15-7.24 (m, 2H), 6.29 (s, 1H), 6.07-6.08 (m, 1H), 6.00 (s, 1H), 5.50-5.52 (m, 1H), 5.23-5.27 (m, 2H), 4.69-4.72 (m, 1H), 4.25-4.47 (m, 3H), 3.83-3.86 (m, 1H), 3.70 (s, 3H), 2.34-2.38 (m, 1H), 1.95-2.23 (m, 5H), 1.46 (s, 6H), 0.88-0.93 (m, 12H). MS analysis confirms that **6bc's** ESI-MS [(M+H)⁺]: theoretical m/z: 779.4; measured value: 779.5

### Embodiment 52 (not of the invention)

### Synthesis of compound 6bd

The synthesis method of compound **6bd** was the same with that in Embodiment 1; the product was carried out by one-step catalytic coupling reaction to get product **6bd** wherein compounds **SM-3b** (0.32mmol) and **SM-4b** (0.32mmol) were used in place of compounds **SM-3a** and **SM-4i,** a yellow solid product **6bd** (0.08g) obtained, yield: 33.7%
¹H NMR (500 MHz, CDCl₃) of product **6bd**: δ 7.77-7.80 (m, 2H), 7.56-7.60 (m, 4H), 7.20-7.23 (m, 2H), 6.30-6.33 (m, 2H), 6.08-6.09 (m, 2H), 5.99 (s, 2H), 5.34-5.39 (m, 2H), 4.72-4.74 (m, 2H), 4.42-4.45 (m, 2H), 4.27-4.30 (m, 2H), 3.71 (s, 6H), 1.96-2.01 (m, 2H), 1.25-1.34 (m, 6H), 0.87-0.90 (m, 6H). MS analysis confirms that **6bd'**s ESI-MS [(M+H)⁺]: theoretical m/z: 735.4; measured value: 735.4

### Embodiment 53 (not of the invention)

### Synthesis of compound 6be

The synthesis method of compound **6be** was the same with that in Embodiment 1; the product was carried out by one-step catalytic coupling reaction to get product **6be** wherein compounds **SM-3d** (0.29mmol) and **SM-4d** (0.29mmol) were used in place of compounds **SM-3a** and **SM-4i,** a yellow solid product **6be** (0.10g) obtained, yield: 42% ¹H NMR (500 MHz, CDCl₃) of product **6be**: δ 7.70-7.76 (m, 2H), 7.47-7.60 (m, 4H), 7.21-7.25 (m, 2H), 6.28-6.32 (m, 2H), 6.07-6.08 (m, 2H), 6.01 (s, 2H), 5.21-5.23 (m, 2H), 4.69-4.72 (m, 2H), 4.44-4.47 (m, 2H), 4.25-4.29 (m, 2H), 1.94-1.99 (m, 2H), 1.46 (s, 18H), 0.82-0.89 (m, 12H). MS analysis confirms that **6be'**s ESI-MS [(M+H)⁺]: theoretical m/z: 819.5; measured value: 819.5

### Embodiment 54 (not of the invention)

### Synthesis of compound 6bf

The synthesis method of compound **6bf** was the same with that in Embodiment 1; the product was carried out by one-step catalytic coupling reaction to get product **6bf** wherein compounds **SM-3h** (0.11mmol) and **SM-4h** (0.11mmol) were used in place of compounds **SM-3a** and **SM-4i,** a yellow solid product **6bf** (0.031g) obtained, yield: 33.6%
¹H NMR (500 MHz, CDCl₃) of product **6bf**: δ 7.47-7.63 (m, 6H), 7.15-7.23 (m, 2H), 7.21-7.24 (m, 2H), 6.07-6.08 (m, 2H), 6.00 (s, 2H), 5.30-5.32 (m, 2H), 5.08-5.09 (m, 2H), 4.73-4.76 (m, 2H), 4.48-4.51 (m, 2H), 4.27-4.30 (m, 2H), 1.94-2.00 (m, 2H), 1.83-1.86 (m, 4H), 1.71 (s, 8H), 1.58 (s, 4H), 0.90-0.91 (m, 12H). MS analysis confirms that **6bf'**s ESI-MS [(M+H)⁺]: theoretical m/z: 843.5; measured value: 843.6

### Embodiment 55 (not of the invention)

### Synthesis of compound 6bg

The synthesis method of compound **6bg** was the same with that in Embodiment 1; the product was carried out by one-step catalytic coupling reaction to get product **6bg** wherein compounds **SM-3g** (0.11mmol) and **SM-4h** (0.11mmol) were used in place of compounds **SM-3a** and **SM-4i,** a yellow solid product **6bg** (0.014g) obtained, yield: 15%
¹H NMR (CD₃OD, 400 MHz) of product **6bg**: δ 7.38-7.34 (m, 1H), 7.00-6.96 (m, 2H), 6.11-6.03 (m, 1H), 5.43-5.39 (m, 1H), 5.29-5.27 (m, 1H), 4.65-4.64 (m, 2H), 4.62 (s, 2H), 4.57 (s, 2H). MS analysis confirms that **6bg'**s ESI-MS [(M+H)⁺]: theoretical m/z: 845.5; measured value: 843.6

### Embodiment 56 (not of the invention)

### Synthesis of compound 6bh

The synthesis method of compound **6bh** was the same with that in Embodiment 1; the product was carried out by one-step catalytic coupling reaction to get product **6bh** wherein compounds **SM-3x** (0.4mmol) and **SM-4b** (0.4mmol) were used in place of compounds **SM-3a** and **SM-4i,** a yellow solid product **6bh** (0.165g) obtained, yield: 57.5%
¹H NMR (500 MHz, CDCl₃) of product **6bh**: δ 7.66-7.52 (m, 8H), 7.20 (m, 2H), 6.23 (m, 1H), 6.23 (m, 1H), 6.06-6.05 (m, 1H), 5.98 (m, 1H), 5.73 (m, 1H), 5.53-5.52 (m, 1H), 5.35 (m, 1H), 4.74-4.71 (m, 1H), 4.49-4.47 (m, 1H), 4.29-4.26 (m, 1H), 3.77-3.69 (m, 6H), 2.33-2.32 (m, 1H), 2.09-1.95 (m, 4H), 1.32-1.24 (m, 4H), 0.91-0.80 (m, 6H). MS analysis confirms that **6bh'**s ESI-MS [(M+H)⁺]: theoretical m/z: 721.3; measured value: 721.5

### Embodiment 57 (not of the invention)

### Synthesis of compound 6bi

The synthesis method of compound **6bi** was the same with that in Embodiment 1; the product was carried out by one-step catalytic coupling reaction to get product **6bi** wherein compounds **SM-3y** (0.41mmol) and **SM-4b** (0.41mmol) were used in place of compounds **SM-3a** and **SM-4i,** a yellow solid product **6bi** (0.13g) obtained, yield: 42.5%
¹H NMR (500 MHz, CDCl₃) of product **6bi**: δ 7.76-7.55 (m, 8H), 7.26-7.23 (m, 2H), 6.29-6.28 (m, 1H), 6.08-6.07 (m, 1H), 5.99 (m, 1H), 5.51-5.49 (m, 1H), 5.37 (m, 1H), 4.75-4.72 (m, 1H), 4.47-4.44 (m, 1H), 4.30-4.27 (m, 1H), 3.72-3.70 (m, 6H), 2.77-2.74 (m, 1H), 2.39-2.34 (m, 1H), 2.15-1.73 (m, 10H), 1.26 (m, 1H), 0.90-0.85 (m, 6H). MS analysis confirms that **6bi'**s ESI-MS [(M+H)⁺]: theoretical m/z: 749.4; measured value: 749.5

### Embodiment 58 (not of the invention)

### Synthesis of compound 6bj

The synthesis method of compound **6bj** was the same with that in Embodiment 1; the product was carried out by one-step catalytic coupling reaction to get product **6bj** wherein compounds **SM-3z** (0.39mmol) and **SM-4b** (0.39mmol) were used in place of compounds **SM-3a** and **SM-4i,** a yellow solid product **6bj** (0.10g) obtained, yield: 33.5%
¹H NMR (500 MHz, CDCl₃) of product **6bj**: δ 7.75-7.46 (m, 6H), 7.35-7.24 (m, 4H), 6.08-5.99 (m, 1H), 5.52-5.48 (m, 1H), 4.75-4.72 (m, 1H), 4.47-4.44 (m, 1H), 4.30-4.28 (m, 1H), 3.76-3.58 (m, 6H), 2.39 (m, 2H), 2.14-1.55 (m, 11H), 1.26 (m, 6H), 0.94-0.88 (m, 6H). 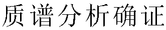 , **6bj** 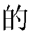 SI-MS [(M+H)⁺]: m/z 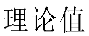 763.4, 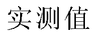 763.5. MS analysis confirms that **6bj'**s ESI-MS [(M+H)⁺]: theoretical m/z: 763.4; measured value: 763.5

### Embodiment 59 (not of the invention)

### Synthesis of compound 6bk

The synthesis method of compound **6bk** was the same with that in Embodiment 1; the product was carried out by one-step catalytic coupling reaction to get product **6bk** wherein compounds **SM-3aa** (0.22mmol) and **SM-4b** (0.22mmol) were used in place of compounds **SM-3a** and **SM-4i,** a yellow solid product **6bk** (0.10g) obtained, yield: 61%
¹H NMR (500 MHz, CDCl₃) of product **6bk**: δ 7.81-7.52 (m, 6H), 7.38-7.20 (m, 4H), 6.09 (m, 1H), 6.0 (m, 1H), 5.43 (m, 1H), 4.73-4.70 (m, 1H), 4.48-4.43 (m, 1H), 4.32-4.29 (m, 1H), 3.70-3.63 (m, 6H), 2.85-2.83 (m, 1H), 2.09-1.48 (m, 11H), 1.11 (m, 6H), 0.92-0.85 (m, 6H). MS analysis confirms that **6bk'**s ESI-MS [(M+H)⁺]: theoretical m/z: 751.4; measured value: 751.5

### Embodiment 60 (not of the invention)

### Synthesis of compound 6bm

The synthesis method of compound **6bm** was the same with that in Embodiment 1; the product was carried out by one-step catalytic coupling reaction to get product **6bm** wherein compounds **SM-3ab** (5.83mmol) and **SM-4b** (5.83mmol) were used in place of compounds **SM-3a** and **SM-4i,** a yellow solid product **6bm** (3.0g) obtained, yield: 54%
¹H NMR (500 MHz, CDCl₃) of product **6bm**: δ 7.76-7.42 (m, 9H), 7.28-7.21 (m, 1H), 6.24 (m, 1H), 6.10-6.09 (m, 1H), 5.99 (m, 1H), 5.45-5.46 (m, 1H), 5.13-5.04 (m, 1H), 4.74-4.71 (m, 1H), 4.53-4.52 (m, 2H), 4.41-4.28 (m, 2H), 4.14-4.00 (m, 2H), 3.70 (m, 6H), 2.94 (m, 1H), 2.11-1.99 (m, 3H), 1.27-1.12 (m, 6H), 0.95-0.87 (m, 6H). MS analysis confirms that **6bm'**s ESI-MS [(M+H)⁺]: theoretical m/z: 753.4; measured value: 753.5

### Embodiment 61 (not of the invention)

### Synthesis of compound 6bn

The synthesis method of compound **6bn** was the same with that in Embodiment 1; the product was carried out by one-step catalytic coupling reaction to get product **6bn** wherein compounds **SM-3n** (0.2mmol) and **SM-4af** (0.2mmol) were used in place of compounds **SM-3a** and **SM-4i,** a yellow solid product **6bn** was obtained (yield: 61%).
MS analysis confirms that **6bn's** ESI-MS [(M+H)⁺]: theoretical m/z: 958.4; measured value: 958.5.

### Embodiment 62 (not of the invention)

### Synthesis of compound 6bp

The synthesis method of compound **6bp** was the same with that in Embodiment 1; the product was carried out by one-step catalytic coupling reaction to get product **6bp** wherein compounds **SM-3ap** (0.2mmol) and **SM-4n** (0.2mmol) were used in place of compounds **SM-3a** and **SM-4i,** a yellow solid product **6bp** was obtained (yield: 56%).
MS analysis confirms that **6bp's** ESI-MS [(M+H)⁺]: theoretical m/z: 992.4; measured value: 992.5.

### Embodiment 63 (not of the invention)

### Synthesis of compound 6bq

The synthesis method of compound **6bq** was the same with that in Embodiment 1; the product was carried out by one-step catalytic coupling reaction to get product **6bq** wherein compounds **SM-3aq** (0.2mmol) and **SM-4bj** (0.2mmol) were used in place of compounds **SM-3a** and **SM-4i,** a yellow solid product **6bq** was obtained (yield: 53%).
MS analysis confirms that **6bq's** ESI-MS [(M+H)⁺]: theoretical m/z: 835.4; measured value: 835.5.

### Embodiment 64 (not of the invention)

### Synthesis of compound 6br

The synthesis method of compound **6br** was the same with that in Embodiment 1; the product was carried out by one-step catalytic coupling reaction to get product **6br** wherein compounds **SM-3ap** (0.2mmol) and **SM-4n** (0.2mmol) were used in place of compounds **SM-3a** and **SM-4i,** a yellow solid product **6br** was obtained (yield: 52%).
MS analysis confirms that **6br's** ESI-MS [(M+H)⁺]: theoretical m/z: 1042.4; measured value: 1042.5.

### Embodiment 65 (not of the invention)

### Synthesis of compound 6bs

The synthesis method of compound **6bs** was the same with that in Embodiment 1; the product was carried out by one-step catalytic coupling reaction to get product **6bs** wherein compounds **SM-3ar** (0.2mmol) and **SM-4bj** (0.2mmol) were used in place of compounds **SM-3a** and **SM-4i,** a yellow solid product **6bs** was obtained (yield: 54%).
MS analysis confirms that **6bs's** ESI-MS [(M+H)⁺]: theoretical m/z: 1027.4; measured value: 1027.5.

### Embodiment 66 (not of the invention)

### Synthesis of compound 6bt

The synthesis method of compound **6bt** was the same with that in Embodiment 1; the product was carried out by one-step catalytic coupling reaction to get product **6bt** wherein compounds **SM-3as** (0.2mmol) and **SM-4bi** (0.2mmol) were used in place of compounds **SM-3a** and **SM-4i,** a yellow solid product **6bt** was obtained (yield: 52%).
MS analysis confirms that **6bt'**s ESI-MS [(M+H)⁺]: theoretical m/z: 968.4; measured value: 968.5.

### Embodiment 67 (not of the invention)

### Synthesis of compound 6bu

The synthesis method of compound **6bu** was the same with that in Embodiment 1; the product was carried out by one-step catalytic coupling reaction to get product **6bu** wherein compounds **SM-3at** (0.2mmol) and **SM-4ad** (0.2mmol) were used in place of compounds **SM-3a** and **SM-4i,** a yellow solid product **6bu** was obtained (yield: 56%).
MS analysis confirms that **6bu'**s ESI-MS [(M+H)⁺]: theoretical m/z: 979.4; measured value: 979.5.

### Embodiment 68 (not of the invention)

### Synthesis of compound 6bv

The synthesis method of compound **6bv** was the same with that in Embodiment 1; the product was carried out by one-step catalytic coupling reaction to get product **6bv** wherein compounds **SM-3au** (0.2mmol) and **SM-4a** (0.2mmol) were used in place of compounds **SM-3a** and **SM-4i,** a yellow solid product **6bv** was obtained (yield: 53%).
MS analysis confirms that **6bv'**s ESI-MS [(M+H)⁺]: theoretical m/z: 991.3; measured value: 991.4.

### Embodiment 69 (not of the invention)

### Synthesis of compound 6bw

The synthesis method of compound **6bw** was the same with that in Embodiment 1; the product was carried out by one-step catalytic coupling reaction to get product **6bw** wherein compounds **SM-3av** (0.2mmol) and **SM-4ad** (0.2mmol) were used in place of compounds **SM-3a** and **SM-4i,** a yellow solid product **6bw** was obtained (yield: 52%).
MS analysis confirms that **6bw's** ESI-MS [(M+H)⁺]: theoretical m/z: 1025.3; measured value: 1025.4.

### Embodiment 70 (according to the invention)

### Synthesis of compound 6bx

The synthesis method of compound **6bx** was the same with that in Embodiment 1; the product was carried out by one-step catalytic coupling reaction to get product **6bx** wherein compounds **SM-3ay** (0.2mmol) and **SM-4a** (0.2mmol) were used in place of compounds **SM-3a** and **SM-4i,** a yellow solid product **6bx** was obtained (yield: 54%).
MS analysis confirms that **6bx's** ESI-MS [(M+H)⁺]: theoretical m/z: 771.4; measured value: 771.4.

### Embodiment 71 (according to the invention)

### Synthesis of compound 6by

The synthesis method of compound **6by** was the same with that in Embodiment 1; the product was carried out by one-step catalytic coupling reaction to get product **6by** wherein compounds **SM-3b** (0.2mmol) and **SM-4ag** (0.2mmol) were used in place of compounds **SM-3a** and **SM-4i,** a yellow solid product **6by** was obtained (yield: 56%).
MS analysis confirms that **6by's** ESI-MS [(M+H)⁺]: theoretical m/z: 771.4; measured value: 771.4.

### Embodiment 72 (not of the invention)

### Synthesis of compound 6bz

The synthesis method of compound **6bz** was the same with that in Embodiment 1; the product was carried out by one-step catalytic coupling reaction to get product **6bz** wherein compounds **SM-3ax** (0.2mmol) and **SM-4ah** (0.2mmol) were used in place of compounds **SM-3a** and **SM-4i,** a yellow solid product **6bz** was obtained (yield: 61%).
MS analysis confirms that **6bz'**s ESI-MS [(M+H)⁺]: theoretical m/z: 805.3; measured value: 805.4.

### Embodiment 73 (not of the invention)

### Synthesis of compound 6ca

The synthesis method of compound **6ca** was the same with that in Embodiment 1; the product was carried out by one-step catalytic coupling reaction to get product **6ca** wherein compounds **SM-3ay** (0.2mmol) and **SM-4ah** (0.2mmol) were used in place of compounds **SM-3a** and **SM-4i,** a yellow solid product **6ca** was obtained (yield: 53%).
MS analysis confirms that **6ca's** ESI-MS [(M+H)⁺]: theoretical m/z: 803.3; measured value: 803.4.

### Embodiment 74 (not of the invention)

### Synthesis of compound 6cb

The synthesis method of compound **6cb** was the same with that in Embodiment 1; the product was carried out by one-step catalytic coupling reaction to get product **6cb** wherein compounds **SM-3ba** (0.2mmol) and **SM-4ah** (0.2mmol) were used in place of compounds **SM-3a** and **SM-4i,** a yellow solid product **6cb** was obtained (yield: 53%).
MS analysis confirms that **6cb's** ESI-MS [(M+H)⁺]: theoretical m/z: 833.3; measured value: 833.4.

### Embodiment 75 (not of the invention)

### Synthesis of compound 6cc

The synthesis method of compound **6cc** was the same with that in Embodiment 1; the product was carried out by one-step catalytic coupling reaction to get product **6cc** wherein compounds **SM-3av** (0.2mmol) and **SM-4ah** (0.2mmol) were used in place of compounds **SM-3a** and **SM-4i,** a yellow solid product **6cc** was obtained (yield: 58%).
MS analysis confirms that **6cc's** ESI-MS [(M+H)⁺]: theoretical m/z: 867.3; measured value: 867.3.

### Embodiment 76 (not of the invention)

### Synthesis of compound 6cd

The synthesis method of compound **6cd** was the same with that in Embodiment 1; the product was carried out by one-step catalytic coupling reaction to get product **6cd** wherein compounds **SM-3aw** (0.2mmol) and **SM-4ah** (0.2mmol) were used in place of compounds **SM-3a** and **SM-4i,** a yellow solid product **6cd** was obtained (yield: 54%).
MS analysis confirms that **6cd's** ESI-MS [(M+H)⁺]: theoretical m/z: 865.2; measured value: 865.3.

### Embodiment 77 (not of the invention)

### Synthesis of compound 6ce

The synthesis method of compound **6ce** was the same with that in Embodiment 1; the product was carried out by one-step catalytic coupling reaction to get product **6ce** wherein compounds **SM-3bb** (0.2mmol) and **SM-4ai** (0.2mmol) were used in place of compounds **SM-3a** and **SM-4i,** a yellow solid product **6ce** was obtained (yield: 57%).
MS analysis confirms that **6ce's** ESI-MS [(M+H)⁺]: theoretical m/z: 831.4; measured value: 831.5.

### Embodiment 78 (not of the invention)

### Synthesis of compound 6cf

The synthesis method of compound **6cf** was the same with that in Embodiment 1; the product was carried out by one-step catalytic coupling reaction to get product **6cf** wherein compounds **SM-3bd** (0.2mmol) and **SM-4aj** (0.2mmol) were used in place of compounds **SM-3a** and **SM-4i,** a yellow solid product **6cf** was obtained (yield: 56%).
MS analysis confirms that **6cf's** ESI-MS [(M+H)⁺]: theoretical m/z: 803.3; measured value: 803.4.

### Embodiment 79 (not of the invention)

### Synthesis of compound 6cg

The synthesis method of compound **6cg** was the same with that in Embodiment 1; the product was carried out by one-step catalytic coupling reaction to get product **6cg** wherein compounds **SM-3bg** (0.2mmol) and **SM-4ak** (0.2mmol) were used in place of compounds **SM-3a** and **SM-4i,** a yellow solid product **6cg** was obtained (yield: 52%).
MS analysis confirms that **6cg's** ESI-MS [(M+H)⁺]: theoretical m/z: 763.4; measured value: 763.5

### Embodiment 80 (not of the invention)

### Synthesis of compound 6ch

The synthesis method of compound **6ch** was the same with that in Embodiment 1; the product was carried out by one-step catalytic coupling reaction to get product **6ch** wherein compounds **SM-3bi** (0.2mmol) and **SM-4am** (0.2mmol) were used in place of compounds **SM-3a** and **SM-4i,** a yellow solid product **6ch** was obtained (yield: 53%).
MS analysis confirms that **6ch's** ESI-MS [(M+H)⁺]: theoretical m/z: 735.4; measured value: 735.5

### Embodiment 81 (not of the invention)

### Synthesis of compound 6ci

The synthesis method of compound **6ci** was the same with that in Embodiment 1; the product was carried out by one-step catalytic coupling reaction to get product **6ci** wherein compounds **SM-3bg** (0.2mmol) and **SM-4a** (0.2mmol) were used in place of compounds **SM-3a** and **SM-4i,** a yellow solid product **6ci** was obtained (yield: 53%).
MS analysis confirms that **6ci's** ESI-MS [(M+H)⁺]: theoretical m/z: 750.4; measured value: 750.5

### Embodiment 82 (not of the invention)

### Synthesis of compound 6cj

The synthesis method of compound **6cj** was the same with that in Embodiment 1; the product was carried out by one-step catalytic coupling reaction to get product **6cj** wherein compounds **SM-3bi** (0.2mmol) and **SM-4a** (0.2mmol) were used in place of compounds **SM-3a** and **SM-4i,** a yellow solid product **6cj** was obtained (yield: 59%).
MS analysis confirms that **6cj's** ESI-MS [(M+H)⁺]: theoretical m/z: 736.4; measured value: 736.5

### Embodiment 83 (not of the invention)

### Synthesis of compound 6ck

The synthesis method of compound **6ck** was the same with that in Embodiment 1; the product was carried out by one-step catalytic coupling reaction to get product **6ck** wherein compounds **SM-3bi** (0.2mmol) and **SM-4am** (0.2mmol) were used in place of compounds **SM-3a** and **SM-4i,** a yellow solid product **6ck** was obtained (yield: 53%).
MS analysis confirms that **6ck's** ESI-MS [(M+H)⁺]: theoretical m/z: 735.4; measured value: 735.5

### Embodiment 84 (not of the invention)

### Synthesis of compound 6cm

The synthesis method of compound **6cm** was the same with that in Embodiment 1; the product was carried out by one-step catalytic coupling reaction to get product **6cm** wherein compounds **SM-3a** (0.2mmol) and **SM-4an** (0.2mmol) were used in place of compounds **SM-3a** and **SM-4i,** a yellow solid product **6cm** (110mg) obtained, yield: 54%
¹H NMR (500 MHz, CDCl₃) of product **6cm:** δ 7.74-7.80 (m, 1H), 7.53-7.62 (m, 8H), 7.26-7.28 (m, 3H), 7.18-7.22 (m, 3H), 5.56-5.67 (m, 2H), 5.44 (m, 1H), 4.74-4.94 (m, 5H), 4.34 (m, 1H), 4.23 (m, 1H), 4.08 (m, 1H), 3.85 (m, 1H), 3.67-3.73 (m, 6H), 2.92 (m, 1H), 2.37 (m, 1H), 2.22 (m, 1H), 2.00-2.11 (m, 4H), 0.90-0.91 (m, 12H). MS analysis confirms that **6cm'**s ESI-MS [(M+H)⁺]: theoretical m/z: 919.4; measured value: 919.5

### Embodiment 85 (not of the invention)

### Synthesis of compound 6cq

The synthesis method of compound **6cq** was the same with that in Embodiment 1; the product was carried out by one-step catalytic coupling reaction to get product **6cq** wherein compounds **SM-3a** (0.2mmol) and **SM-4ar** (0.2mmol) were used in place of compounds **SM-3a** and **SM-4i,** a yellow solid product **6cq** (83mg) obtained, yield: 43%.
¹H NMR (500 MHz, CDCl₃) of product **6cq:** δ 7.46-7.75 (m, 9H), 7.12-7.30 (m, 3H), 6.81-6.87 (m, 2H), 5.64-5.74 (m, 2H), 5.17-5.41 (m, 4H), 4.56-4.93 (m, 5H), 3.94-4.30 (m, 4H), 3.81-3.85 (m, 6H), 3.63-3.65 (m, 6H), 2.83 (m, 1H), 2.33 (m, 1H), 2.17 (m, 1H), 1.96-2.07 (m, 4H), 0.86-0.89 (m, 12H). MS analysis confirms that **6cq'**s ESI-MS [(M+H)⁺]: theoretical m/z: 945.5; measured value: 945.7

### Embodiment 86 (not of the invention)

### Synthesis of compound 6cu

The synthesis method of compound **6cu** was the same with that in Embodiment 1; the system was carried out by one-step catalytic coupling reaction to get a product, which was then removed Boc, neutralized and purified to get compound **6cu,** wherein compounds **SM-3a** (0.2mmol) and **SM-4av** (0.2mmol) were used in place of compounds **SM-3a** and **SM-4i** to get a yellow solid Boc protected product(310mg), yield: 25%. 10mL of 3N HCl/Et₂O was added, the system was allowed to react at room temperature until the reactants have reacted completely, adjusted pH to alkaline, subjected to purification with preparation TLC to get a yellow solid **6cu** (55mg), yield of the above-mentioned 2-stepreaction: 37%.
H NMR (500 MHz, CDCl₃) of product **6cu:** δ 7.50-7.78 (m, 9H), 7.02-7.35 (m, 3H), 5.67 (m, 2H), 5.13-5.26 (m, 2H), 4.69-4.75 (m, 2H), 4.35-4.41 (m, 2H), 4.13-4.14 (m, 1H), 3.88 (m, 1H), 3.71 (s, 6H), 3.35 (m, 1H), 2.18-2.39 (m, 2H), 2.00-2.11 (m, 4H), 0.91 (s, 12H). MS analysis confirms **6cu**'s ESI-MS [(M+H)⁺]: theoretical m/z: 740.4; measured value: 740.5.

### Embodiment 87 (not of the invention)

### Synthesis of compound 6cv

The synthesis method of compound **6cv** was the same with that in Embodiment 1, the system was then carried out by one-step catalytic coupling reaction to get a product, which was removed Boc, neutralized, purified to get **6cv,** wherein compounds **SM-3b** (0.2mmol) and **SM-4av** (0.2mmol) were used in place of compounds **SM-3a** and **SM-4i to** get a yellow solid Boc protected product(110mg), which was added 10mL of 3N HCl/Et₂O, allowed to react at room temperature until reactants have gone, adjusted pH to alkaline, subjected to purification with preparation TLC to get a yellow solid **6cv** (47mg); yield of the above-mentioned 2-step reaction: 32%.
MS analysis confirms **6cv's** ESI-MS [(M+H)⁺]: theoretical m/z: 738.4; measured value: 738.5.

### Embodiment 88 (not of the invention)

### Synthesis of compound 6cw

The synthesis method of compound **6cw** was the same with that in Embodiment 1; the product was carried out by one-step catalytic coupling reaction to get product **6cw** wherein compounds **SM-3b** (0.2mmol) and **SM-4av** (0.2mmol) were used in place of compounds **SM-3a** and **SM-4i,** a yellow solid product **6cw** was obtained (yield: 35%).
MS analysis confirms that **6cw's** ESI-MS [(M+H)⁺]: theoretical m/z: 838.4; measured value: 838.6

### Embodiment 89 (not of the invention)

### Synthesis of compound 6cx

The synthesis method of compound **6cx** was the same with that in Embodiment 1, the system was then carried out by one-step catalytic coupling reaction to get a Boc protected product, then the Boc was removed to get **6cx,** wherein compounds **SM-3b** (0.2mmol) and **SM-4aw** (0.2mmol) were used in place of compounds **SM-3a** and **SM-4i** to get 13mg of a yellow solid product, yield: 10%. 10mg of the product was taken, added 10mL of 3N HCl/ether, allowed to react thoroughly at room temperature under stirring. The reaction liquid was concentrated to get a yellow solid **6cx,** yield: 32%.
MS analysis confirms **6cx's** ESI-MS [(M+H)⁺]: theoretical m/z: 778.5; measured value: 778.6.

### Embodiment 90 (not of the invention)

### Synthesis of compound 6cy

The synthesis method of compound **6cy** was the same with that in Embodiment 1; the product was carried out by one-step catalytic coupling reaction to get product **6cy,** wherein compounds **SM-3b** (0.2mmol) and **SM-4aw** (0.2mmol) were used in place of compounds **SM-3a** and **SM-4i,** a yellow solid product **6cy** (33mg) was obtained, yield: 23%.
MS analysis confirms that **6cy**'s ESI-MS [(M+H)⁺]:: theoretical m/z: 878.5; measured value: 878.6.

### Embodiment 91 (not of the invention)

### Synthesis of compound 6cz

The synthesis method of compound **6cz** was the same with that in Embodiment 1; the product was carried out by one-step catalytic coupling reaction to get product **6cz,** wherein compounds **SM-3bj** (0.2mmol) and **SM-4b** (0.2mmol) were used in place of compounds **SM-3a** and **SM-4i,** a yellow solid product **6cz** was obtained, yield: 29%.
¹H NMR (500 MHz, CDC13) of product **6cz:** δ 7.62-7.78 (m, 10H), 5.98-6.09 (m, 2H), 5.43-5.59 (m, 2H), 4.49-4.60 (m, 4H), 3.70-3.75 (m, 8H), 3.01 (s, 3H), 2.78 (m, 1H), 0.89-0.91 (m, 12H). MS analysis confirms that **6cz**'s ESI-MS [(M+H)⁺]:: theoretical m/z: 816.3; measured value: 816.5.

### Embodiment 92 (not of the invention)

### Synthesis of compound 6da

The synthesis method of compound **6da** was the same with that in Embodiment 1; the product was carried out by one-step catalytic coupling reaction to get product **6da,** wherein compounds **SM-3bk** (0.2mmol) and **SM-4b** (0.2mmol) were used in place of compounds **SM-3a** and **SM-4i,** a yellow solid product **6da** was obtained, yield: 32%.
MS analysis confirms that **6da**'s ESI-MS [(M+H)⁺]:: theoretical m/z: 842.4; measured value: 842.5.

### Embodiment 93 (not of the invention)

### Synthesis of compound 6db

The synthesis method of compound **6db** was the same with that in Embodiment 1; the product was carried out by one-step catalytic coupling reaction to get product **6db,** wherein compounds **SM-3bm** (0.2mmol) and **SM-4b** (0.2mmol) were used in place of compounds **SM-3a** and **SM-4i,** a yellow solid product **6db** was obtained, yield: 22%.
MS analysis confirms that **6db'**s ESI-MS [(M+H)⁺]:: theoretical m/z: 796.4; measured value: 796.6.

### Embodiment 94 (not of the invention)

### Synthesis of compound 6dc

The synthesis method of compound **6dc** was the same with that in Embodiment 1; the product was carried out by one-step catalytic coupling reaction to get product **6dc,** wherein compounds **SM-3bn** (0.2mmol) and **SM-4b** (0.2mmol) were used in place of compounds **SM-3a** and **SM-4i,** a yellow solid product **6dc** was obtained, yield: 33%.
MS analysis confirms that **6dc**'s ESI-MS [(M+H)⁺]:: theoretical m/z: 824.4; measured value: 824.5.

### Embodiment 95 (not of the invention)

### Synthesis of compound 6dd

The synthesis method of compound **6dd** was the same with that in Embodiment 1; the product was carried out by one-step catalytic coupling reaction to get product **6dd,** wherein compounds **SM-3bp** (0.2mmol) and **SM-4b** (0.2mmol) were used in place of compounds **SM-3a** and **SM-4i,** a yellow solid product **6dd** (20mg) was obtained, yield: 28%.
MS analysis confirms that **6dd**'s ESI-MS [(M+H)⁺]:: theoretical m/z: 844.4; measured value: 844.5.

### Embodiment 96 (not of the invention)

### Synthesis of compound 6de

The synthesis method of compound **6de** was the same with that in Embodiment 1; the product was carried out by one-step catalytic coupling reaction to get product **6de,** wherein compounds **SM-3bf** (0.2mmol) and **SM-4a** (0.2mmol) were used in place of compounds **SM-3a** and **SM-4i,** a yellow solid product **6de** was obtained, yield: 35%.
¹H NMR (500 MHz, CDC13) of product **6de:** δ 7.81 (m, 1H), 7.53-7.59 (m, 8H), 7.34 (s, 1H), 7.24 (s, 1H), 7.19 (s, 1H), 5.55-5.56 (d, *J* = 8.5 Hz, 1H), 5.10-5.12 (d, *J* = 8.5 Hz, 1H), 4.48-4.51 (t, *J* = 7.5 Hz, 1H), 4.33-4.36 (m, 1H), 3.97 (m, 1H), 3.85 (m, 1H), 3.70 (s, 3H), 3.45 (m, 1H), 3.14 (m, 1H), 2.95 (s, 6H). 2.34-2.39 (m, 2H), 2.19-2.24 (m, 2H), 1.97-2.10 (m, 6H), 0.86-0.91 (m, 12H). MS analysis confirms that **6de'**s ESI-MS [(M+H)⁺]:: theoretical m/z: 752.4; measured value: 752.5.

### Embodiment 97 (not of the invention)

### Synthesis of compound 6df

The synthesis method of compound **6df** was the same with that in Embodiment 1; the product was carried out by one-step catalytic coupling reaction to get product **6df,** wherein compounds **SM-3bf** (0.2mmol) and **SM-4b** (0.2mmol) were used in place of compounds **SM-3a** and **SM-4i,** a yellow solid product **6df** was obtained, yield: 35%.
¹H NMR (500 MHz, CDC13) of product **6df:** δ 7.82-7.86 (m, 1H), 7.54-7.68 (m, 8H), 7.34 (s, 1H), 7.19-7.23 (m, 2H), 6.24-6.28 (m, 1H), 5.98-6.08 (m, 2H), 5.44-5.53 (m, 1H), 5.26 (m, 1H), 5.08-5.09 (m, 1H), 4.71 (m, 1H), 4.49-4.51 (m, 1H), 4.28-4.34 (m, 1H), 3.94-3.95 (m, 1H), 3.70 (s, 3H), 3.43 (m, 1H), 3.15 (m, 1H), 2.92 (s, 6H), 1.97-2.20 (m, 6H), 1.05-1.10 (m, 6H), 0.88 (s, 6H). MS analysis confirms that **6df'**s ESI-MS [(M+H)⁺]:: theoretical m/z: 750.4; measured value: 750.5.

### Embodiment 98 (not of the invention)

### Synthesis of compound 6dg

The synthesis method of compound **6dg** was the same with that in Embodiment 1; the product was carried out by one-step catalytic coupling reaction to get product **6dg,** wherein compounds **SM-3b** (0.2mmol) and **SM-4ax** (0.2mmol) were used in place of compounds **SM-3a** and **SM-4i,** a yellow solid product **6dg** was obtained, yield: 36%. ¹H NMR (500 MHz, CDC13) of product **6dg:** δ 7.10-7.71 (m, 17H), 5.97-6.15 (m, 3H), 5.41-5.55 (m, 3H), 4.73 (m, 1H), 4.48-4.55 (m, 1H), 4.26 (m, 1H), 4.03 (m, 1H), 3.69 (s, 3H), 3.30 (m, 1H), 2.72 (m, 1H), 2.44 (s, 3H), 1.97-2.27 (m, 6H), 0.88-0.99 (m, 6H).
MS analysis confirms that **6dg'**s ESI-MS [(M+H)⁺]:: theoretical m/z: 770.4; measured value: 770.5.

### Embodiment 99 (not of the invention)

### Synthesis of compound 6dh

The synthesis method of compound **6dh** was the same with that in Embodiment 1; the product was carried out by one-step catalytic coupling reaction to get product **6dh,** wherein compounds **SM-3bq** (0.2mmol) and **SM-4a** (0.2mmol) were used in place of compounds **SM-3a** and **SM-4i,** a yellow solid product **6dh** was obtained, yield: 22%.
MS analysis confirms that **6dh'**s ESI-MS [(M+H)⁺]:: theoretical m/z: 763.4; measured value: 763.5.

### Embodiment 100 (not of the invention)

### Synthesis of compound 6di

The synthesis method of compound **6di** was the same with that in Embodiment 1; the product was carried out by one-step catalytic coupling reaction to get product **6di,** wherein compounds **SM-3br** (0.2mmol) and **SM-4a** (0.2mmol) were used in place of compounds **SM-3a** and **SM-4i,** a yellow solid product **6di** was obtained, yield: 38%.
MS analysis confirms that **6di'**s ESI-MS [(M+H)⁺]:: theoretical m/z: 777.4; measured value: 777.4.

### Embodiment 101 (not of the invention)

### Synthesis of compound 6dj

The synthesis method of compound **6dj** was the same with that in Embodiment 1; the product was carried out by one-step catalytic coupling reaction to get product **6dj,** wherein compounds **SM-3bs** (0.2mmol) and **SM-4a** (0.2mmol) were used in place of compounds **SM-3a** and **SM-4i,** a yellow solid product **6dj** was obtained, yield: 46%.
¹H NMR (500 MHz, CDCl₃) of product **6dj:** δ 7.59-7.47 (m, 10H), 6.26 (m, 1H), 6.08 (m, 1H), 5.99 (m, 1H), 5.26 (s, 1H), 4.77 (m, 1H), 4.54 (m, 1H), 4.35 (m, 1H), 4.28 (m, 1H), 3.87 (m, 1H), 3.73 (s, 6H), 2.39 (m, 2H), 2.21-1.69 (m, 14H), 1.26 (d, 6H). MS analysis confirms that **6dj'**s ESI-MS [(M+H)⁺]:: theoretical m/z: 777.4; measured value: 777.5.

### Embodiment 102 (not of the invention)

### Synthesis of compound 6dk

The synthesis method of compound **6dk** was the same with that in Embodiment 1; the product was carried out by one-step catalytic coupling reaction to get product **6dk,** wherein compounds **SM-3br** (0.2mmol) and **SM-4ay** (0.2mmol) were used in place of compounds **SM-3a** and **SM-4i,** a yellow solid product **6dk** was obtained, yield: 36%.
MS analysis confirms that **6dk'**s ESI-MS [(M+H)⁺]:: theoretical m/z: 817.4; measured value: 817.6.

### Embodiment 103 (not of the invention)

### Synthesis of compound 6dm

The synthesis method of compound **6dm** is the same with that in Embodiment 1; the product was carried out by one-step catalytic coupling reaction to get product **6dm,** wherein compounds **SM-3br** (0.2mmol) and **SM-4az** (0.2mmol) were used in place of compounds **SM-3a** and **SM-4i,** a yellow solid product **6dm** was obtained, yield: 38%.
MS analysis confirms that **6dm'**s ESI-MS [(M+H)⁺]:: theoretical m/z: 815.4; measured value: 815.5.

### Embodiment 104 (not of the invention)

### Synthesis of compound 6dn

The synthesis method of compound **6dn** was the same with that in Embodiment 1; the product was carried out by one-step catalytic coupling reaction to get product **6dn,** wherein compounds **SM-3bq** (0.2mmol) and **SM-4ay** (0.2mmol) were used in place of compounds **SM-3a** and **SM-4i,** a yellow solid product **6dn** was obtained, yield: 30%.
MS analysis confirms that **6dn'**s ESI-MS [(M+H)⁺]:: theoretical m/z: 803.4; measured value: 803.5.

### Embodiment 105 (not of the invention)

### Synthesis of compound 6dp

The synthesis method of compound **6dp** was the same with that in Embodiment 1; the product was carried out by one-step catalytic coupling reaction to get product **6dp,** wherein compounds **SM-3bq** (0.2mmol) and **SM-4ba** (0.2mmol) were used in place of compounds **SM-3a** and **SM-4i,** a yellow solid product **6dp** was obtained, yield: 28%.
¹H NMR (500 MHz, CDCl₃) of product **6dp:** δ 8.02 (s, 1H), 7.85-7.55 (m, 9H), 6.34 (m, 1H), 6.09 (m, 1H), 5.99 (m, 1H), 5.42 (m, 1H), 5.36 (m, 1H), 4.81 (m, 1H), 4.44 (m, 1H), 4.38 (m, 1H), 3.90 (m, 1H), 3.71 (s, 6H), 3.50 (m, 1H), 2.34-2.01 (m, 16H). MS analysis confirms that **6dp'**s ESI-MS [(M+H)⁺]:: theoretical m/z: 789.4; measured value: 789.5.

### Embodiment 106 (not of the invention)

### Synthesis of compound 6dq

The synthesis method of compound **6dq** was the same with that in Embodiment 1; the product was carried out by one-step catalytic coupling reaction to get product **6dq,** wherein compounds **SM-3bt** (0.2mmol) and **SM-4b** (0.2mmol) were used in place of compounds **SM-3a** and **SM-4i,** a yellow solid product **6dq** was obtained, yield: 36%.
¹H NMR (500 MHz, CDCl₃) of product **6dq:** δ 7.53-7.22 (m, 8H), 6.21 (m, 1H), 6.10 (m, 1H), 6.03 (m, 1H), 5.46 (m, 1H), 5.39 (m, 1H), 4.74 (m, 1H), 4.60 (m, 1H), 4.32 (m, 1H), 4.21 (m, 1H), 3.99 (m, 1H), 3.86 (m, 1H), 3.72 (s, 3H), 3.69 (s, 3H), 2.62 (m, 1H), 2.44 (m, 1H), 2.06-1.72 (m, 6H), 1.26 (d, 12H). MS analysis confirms that **6dq**'s ESI-MS [(M+H)⁺]:: theoretical m/z: 753.4; measured value: 753.5.

### Embodiment 107 (not of the invention)

### Synthesis of compound 6dr

The synthesis method of compound **6dr** was the same with that in Embodiment 1; the product was carried out by one-step catalytic coupling reaction to get product **6dr,** wherein compounds **SM-3bu** (0.2mmol) and **SM-4b** (0.2mmol) were used in place of compounds **SM-3a** and **SM-4i,** a yellow solid product **6dr** was obtained, yield: 33%.
MS analysis confirms that **6dr'**s ESI-MS [(M+H)⁺]:: theoretical m/z: 753.4; measured value: 753.5.

### Embodiment 108 (not of the invention)

### Synthesis of compound 6ds

The synthesis method of compound **6ds** was the same with that in Embodiment 1; the product was carried out by one-step catalytic coupling reaction to get product **6ds,** wherein compounds **SM-3bv** (0.2mmol) and **SM-4a** (0.2mmol) were used in place of compounds **SM-3a** and **SM-4i,** a yellow solid product **6ds** was obtained, yield: 38%.
MS analysis confirms that **6ds'**s ESI-MS [(M+H)⁺]:: theoretical m/z: 797.4; measured value: 797.5.

### Embodiment 109 (not of the invention)

### Synthesis of compound 6dt

The synthesis method of compound **6dt** was the same with that in Embodiment 1; the product was carried out by one-step catalytic coupling reaction to get product **6dt,** wherein compounds **SM-3bv** (0.2mmol) and **SM-4b** (0.2mmol) were used in place of compounds **SM-3a** and **SM-4i,** a yellow solid product **6dt** was obtained, yield: 41%.
MS analysis confirms that **6dt'**s ESI-MS [(M+H)⁺]:: theoretical m/z: 795.4; measured value: 795.5.

### Embodiment 110 (not of the invention)

### Synthesis of compound 6du

The synthesis method of compound **6du** was the same with that in Embodiment 1; the product was carried out by one-step catalytic coupling reaction to get product **6du,** wherein compounds **SM-3bw** (0.2mmol) and **SM-4b** (0.2mmol) were used in place of compounds **SM-3a** and **SM-4i,** a yellow solid product **6du** was obtained, yield: 39%.
¹H NMR (500 MHz, CDCl₃) of product **6du:** δ 7.16-7.82 (m, 15H), 5.98-6.26 (m, 3H), 5.35-5.53 (m, 1H), 4.71-4.74 (m, 1H), 4.48-4.51 (m, 1H), 3.91-4.04 (m, 6H), 3.62-3.69 (m, 8H), 2.47-2.38 (m, 1H), 2.04-2.08 (m, 1H), 1.69-2.00 (m, 1H), 1.05-0.87 (m, 6H).
MS analysis confirms that **6du**'s ESI-MS [(M+H)⁺]:: theoretical m/z: 829.4; measured value: 829.5.

### Embodiment 111 (not of the invention)

### Synthesis of compound 6dv

The synthesis method of compound **6dv** was the same with that in Embodiment 1; the product was carried out by one-step catalytic coupling reaction to get product **6dv,** wherein compounds **SM-3bw** (0.2mmol) and **SM-4ah** (0.2mmol) were used in place of compounds **SM-3a** and **SM-4i,** a yellow solid product **6dv** was obtained, yield: 34%.
MS analysis confirms that **6dv'**s ESI-MS [(M+H)⁺]:: theoretical m/z: 863.3; measured value: 863.5.

### Embodiment 112 (not of the invention)

### Synthesis of compound 6dw

The synthesis method of compound **6dw** was the same with that in Embodiment 1; the product was carried out by one-step catalytic coupling reaction to get product **6dw,** wherein compounds **SM-3bv** (0.2mmol) and **SM-4ah** (0.2mmol) were used in place of compounds **SM-3a** and **SM-4i,** a yellow solid product **6dw** was obtained, yield: 37%.
MS analysis confirms that **6dw'**s ESI-MS [(M+H)⁺]:: theoretical m/z: 829.4; measured value: 829.4.

### Embodiment 113 (according to the invention)

### Synthesis of compound 6dy

The synthesis method of compound **6dy** was the same with that in Embodiment 1; the product was carried out by one-step catalytic coupling reaction to get product **6dy,** wherein compounds **SM-3ay** (0.2mmol) and **SM-4a** (0.2mmol) were used in place of compounds **SM-3a** and **SM-4i,** a yellow solid product **6dy** was obtained, yield: 42%.
¹H NMR (500 MHz, CDCl₃) of product **6dy:** δ 7.65-7.18 (m, 15H), 6.23 (m, 1H), 6.01 (m, 1H), 5.89 (m, 1H), 5.50 (m, 1H), 5.39 (m, 1H), 5.25 (m, 1H), 4.52 (m, 1H), 4.34 (m, 1H), 4.12 (m, 1H), 3.84 (m, 1H), 3.67 (s, 3H), 3.61 (s, 3H), 2.34-1.83 (m, 6H), 1.23 (d, 6H). MS analysis confirms that **6dy**'s ESI-MS [(M+H)⁺]:: theoretical m/z: 771.4; measured value: 771.4.

### Embodiment 114 (not of the invention)

### Synthesis of compound 6dz

The synthesis method of compound **6dz** was the same with that in Embodiment 1; the product was carried out by one-step catalytic coupling reaction to get product **6dz,** wherein compounds **SM-3ck** (0.2mmol) and **SM-4bf** (0.2mmol) were used in place of compounds **SM-3a** and **SM-4i,** a yellow solid product **6dz** was obtained, yield: 38%.
¹H NMR (500 MHz, CDCl₃) of product **6dz:** δ 7.71-7.40 (m, 20H), 6.28 (m, 1H), 6.04 (m, 1H), 5.99 (m, 1H), 5.50 (m, 1H), 5.39 (m, 1H), 4.54 (m, 1H), 4.12 (m, 1H), 3.98 (m, 1H), 3.68 (s, 3H), 3.65 (s, 3H), 2.23-1.82 (m, 6H). MS analysis confirms that **6dz**'s ESI-MS [(M+H)⁺]:: theoretical m/z: 805.3; measured value: 805.5.

### Embodiment 115 (according to the invention)

### Synthesis of compound 6ea

The synthesis method of compound **6ea** was the same with that in Embodiment 1; the product was carried out by one-step catalytic coupling reaction to get product **6ea,** wherein compounds **SM-3ay** (0.2mmol) and **SM-4b** (0.2mmol) were used in place of compounds **SM-3a** and **SM-4i,** a yellow solid product **6ea** was obtained, yield: 33%.
¹H NMR (500 MHz, CDCl₃) of product **6ea:** δ 7.67-7.20 (m, 13H), 6.26 (s, 1H), 6.15 (s, 1H), 6.08 (m, 1H), 5.99 (m, 1H), 5.59 (m, 1H), 5.46 (m, 1H), 5.31 (m, 1H), 4.76 (m, 1H), 4.48 (m, 1H), 4.30 (m, 1H), 3.70 (s, 3H), 3.65 (s, 3H), 3.22 (m, 1H), 2.24-1.92 (m, 6H), 1.26 (d, 6H). MS analysis confirms that **6ea**'s ESI-MS [(M+H)⁺]:: theoretical m/z: 771.4; measured value: 771.5.

### Embodiment 116 (not of the invention)

### Synthesis of compound 6eb

The synthesis method of compound **6eb** was the same with that in Embodiment 1; the product was carried out by one-step catalytic coupling reaction to get product **6eb,** wherein compounds **SM-3bx** (0.2mmol) and **SM-4b** (0.2mmol) were used in place of compounds **SM-3a** and **SM-4i,** a yellow solid product **6eb** was obtained, yield: 37%.
MS analysis confirms that **6eb'**s ESI-MS [(M+H)⁺]:: theoretical m/z: 737.4; measured value: 737.4.

### Embodiment 117 (not of the invention)

### Synthesis of compound 6ec

The synthesis method of compound **6ec** was the same with that in Embodiment 1; the product was carried out by one-step catalytic coupling reaction to get product **6ec,** wherein compounds **SM-3by** (0.2mmol) and **SM-4b** (0.2mmol) were used in place of compounds **SM-3a** and **SM-4i,** a yellow solid product **6ec** was obtained, yield: 43%.

MS analysis confirms that **6ec**'s ESI-MS [(M+H)⁺]: theoretical m/z: 737.4; measured value: 737.5.

### Embodiment 118 (not of the invention)

### Synthesis of compound 6ee

The synthesis method of compound **6ee** was the same with that in Embodiment 1; the product was carried out by one-step catalytic coupling reaction to get product **6ee,** wherein compounds **SM-3at** (0.2mmol) and **SM-4ad** (0.2mmol) were used in place of compounds **SM-3a** and **SM-4i,** a yellow solid product **6ee** was obtained, yield: 52%.
MS analysis confirms that **6ee**'s ESI-MS [(M+H)⁺]: theoretical m/z: 979.4; measured value: 979.5.

### Embodiment 119 (not of the invention)

### Synthesis of compound 6ef

The synthesis method of compound **6ef** was the same with that in Embodiment 1; the product was carried out by one-step catalytic coupling reaction to get product **6ef,** wherein compounds **SM-3at** (0.2mmol) and **SM-4b** (0.2mmol) were used in place of compounds **SM-3a** and **SM-4i,** a yellow solid product **6ef** was obtained, yield: 51%.
MS analysis confirms that **6ef'**s ESI-MS [(M+H)⁺]: theoretical m/z: 787.4; measured value: 787.5.

### Embodiment 120 (not of the invention)

### Synthesis of compound 6eg

The synthesis method of compound **6eg** was the same with that in Embodiment 1; the product was carried out by one-step catalytic coupling reaction to get product **6eg,** wherein compounds **SM-3bz** (0.2mmol) and **SM-4a** (0.2mmol) were used in place of compounds **SM-3a** and **SM-4i,** a yellow solid product **6eg** was obtained, yield: 58%.
MS analysis confirms that **6eg'**s ESI-MS [(M+H)⁺]: theoretical m/z: 787.4; measured value: 787.5.

### Embodiment 121 (not of the invention)

### Synthesis of compound 6eh

The synthesis method of compound **6eh** was the same with that in Embodiment 1; the product was carried out by one-step catalytic coupling reaction to get product **6eh,** wherein compounds **SM-3cm** (0.2mmol) and **SM-4b** (0.2mmol) were used in place of compounds **SM-3a** and **SM-4i,** a yellow solid product **6eh** was obtained, yield: 53%.
MS analysis confirms that **6eh'**s ESI-MS [(M+H)⁺]: theoretical m/z: 833.3; measured value: 833.4.

### Embodiment 122 (not of the invention)

### Synthesis of compound 6ei

The synthesis method of compound **6ei** was the same with that in Embodiment 1; the product was carried out by one-step catalytic coupling reaction to get product **6ei,** wherein compounds **SM-3cp** (0.2mmol) and **SM-4a** (0.2mmol) were used in place of compounds **SM-3a** and **SM-4i,** a yellow solid product **6ei** was obtained, yield: 47%.
MS analysis confirms that **6ei'**s ESI-MS [(M+H)⁺]: theoretical m/z: 833.3; measured value: 833.4.

### Embodiment 123 (not of the invention)

### Synthesis of compound 6ej

The synthesis method of compound **6ej** was the same with that in Embodiment 1; the product was carried out by one-step catalytic coupling reaction to get product **6ej,** wherein compounds **SM-3ci** (0.2mmol) and **SM-4bd** (0.2mmol) were used in place of compounds **SM-3a** and **SM-4i,** a yellow solid product **6ej** was obtained, yield: 43%.
MS analysis confirms that **6ej'**s ESI-MS [(M+H)⁺]: theoretical m/z: 880.4; measured value: 880.5.

### Embodiment 124 (not of the invention)

### Synthesis of compound 6ek

The synthesis method of compound **6ek** was the same with that in Embodiment 1; the product was carried out by one-step catalytic coupling reaction to get product **6ek,** wherein compounds **SM-3cq** (0.2mmol) and **SM-4b** (0.2mmol) were used in place of compounds **SM-3a** and **SM-4i,** a yellow solid product **6ek** (81mg) was obtained, yield: 46%.
MS analysis confirms that **6ek'**s ESI-MS [(M+H)⁺]: theoretical m/z: 881.5; measured value: 881.5.

### Embodiment 125 (not of the invention)

### Synthesis of compound 6em

The synthesis method of compound **6em** was the same with that in Embodiment 1; the product was carried out by one-step catalytic coupling reaction to get product **6em,** wherein compounds **SM-3cq** (0.2mmol) and **SM-4bf** (0.2mmol) were used in place of compounds **SM-3a** and **SM-4i,** a yellow solid product **6em** (75mg) was obtained, yield: 41%.
MS analysis confirms that **6em**'s ESI-MS [(M+H)⁺]: theoretical m/z: 915.5; measured value: 915.6.

### Embodiment 126 (not of the invention)

### Synthesis of compound 6en

The synthesis method of compound **6en** was the same with that in Embodiment 1; the product was carried out by one-step catalytic coupling reaction to get product **6en,** wherein compounds **SM-3cr** (0.2mmol) and **SM-4bg** (0.2mmol) were used in place of compounds **SM-3a** and **SM-4i,** a yellow solid product **6en** (94mg) was obtained, yield: 54%.
MS analysis confirms that **6en**'s ESI-MS [(M+H)⁺]: theoretical m/z: 869.5; measured value: 869.5.

### Embodiment 127 (not of the invention)

### Synthesis of compound 6ep

The synthesis method of compound **6ep** was the same with that in Embodiment 1; the product was carried out by one-step catalytic coupling reaction to get product **6ep,** wherein compounds **SM-3cr** (0.2mmol) and **SM-4bh** (0.2mmol) were used in place of compounds **SM-3a** and **SM-4i,** a yellow solid product **6ep** (71mg) was obtained, yield: 39%.
MS analysis confirms that **6ep'**s ESI-MS [(M+H)⁺]: theoretical m/z: 903.5; measured value: 903.5.

### Embodiment 128 (not of the invention)

### Synthesis of compound 6fa

The synthesis method of compound **6fa** was the same with that in Embodiment 1; the product was carried out by one-step catalytic coupling reaction to get product **6fa,** wherein compounds **SM-3cb** (0.2mmol) and **SM-4b** (0.2mmol) were used in place of compounds **SM-3a** and **SM-4i,** a yellow solid product **6fa** (69mg) was obtained, yield: 43%.
MS analysis confirms that **6fa**'s ESI-MS [(M+H)⁺]: theoretical m/z: 865.3; measured value: 865.3.

### Embodiment 129 (not of the invention)

### Synthesis of compound 6fb

The synthesis method of compound **6fb** was the same with that in Embodiment 1; the product was carried out by one-step catalytic coupling reaction to get product **6fb,** wherein compounds **SM-3cn** (0.2mmol) and **SM-4b** (0.2mmol) were used in place of compounds **SM-3a** and **SM-4i,** a yellow solid product **6fb** (32mg) was obtained, yield: 20%.
¹H NMR (500 MHz, CDCl₃) of product **6fb:** δ 7.39-7.10 (m, 8H), 6.09 (s, 1H), 5.99 (s, 1H), 5.49 (s, 1H), 5.25 (s, 1H), 4.74 (m, 1H), 4.38 (m, 1H), 4.32 (m, 1H), 3.91 (s, 1H), 3.71 (s, 6H), 2.38 (m, 2H), 2.19 (m, 2H), 2.09-2.07 (m, 4H), 1.28 (s, 6H), 1.27 (s, 6H). MS analysis confirms that **6fb'**s ESI-MS [(M+H)⁺]: theoretical m/z: 799.3; measured value: 799.3.

### Embodiment 130 (not of the invention)

### Synthesis of compound 6fc

The synthesis method of compound **6fc** was the same with that in Embodiment 1; the product was carried out by one-step catalytic coupling reaction to get product **6fc,** wherein compounds **SM-3cm** (0.2mmol) and **SM-4bf** (0.2mmol) were used in place of compounds **SM-3a** and **SM-4i,** a yellow solid product **6fc** (46mg) was obtained, yield: 26%.
¹H NMR (500 MHz, CDCl₃) of product **6fc:** δ 7.92-7.27 (m, 18H), 6.24 (s, 1H), 6.19 (s, 1H), 5.97 (s, 1H), 5.89 (s, 1H), 5.48 (m, 1H), 5.28 (s, 1H), 4.55 (m, 1H), 4.10 (s, 1H), 3.76 (s, 6H), 2.32-2.03 (m, 6H). MS analysis confirms that **6fc'**s ESI-MS [(M+H)⁺]: theoretical m/z: 867.3; measured value: 867.3.

### Embodiment 131 (not of the invention)

### Synthesis of compound 6fd

The synthesis method of compound **6fd** was the same with that in Embodiment 1; the product was carried out by one-step catalytic coupling reaction to get product **6fd,** wherein compounds **SM-3cm** (0.2mmol) and **SM-4b** (0.2mmol) were used in place of compounds **SM-3a** and **SM-4i,** a yellow solid product **6fd** (59mg) was obtained, yield: 25%.
¹H NMR (500 MHz, CDCl₃) of product **6fd:** δ 7.46-7.41 (m, 13H), 6.20 (s, 1H), 6.09 (s, 1H), 5.99 (s, 1H), 5.51 (m, 1H), 5.31 (m, 1H), 4.78 (m, 1H), 4.57 (m, 1H), 4.31 (m, 1H), 3.70 (s, 6H), 3.24 (m, 1H), 2.24-1.92 (m, 6H), 1.28 (s, 6H). MS analysis confirms that **6fd'**s ESI-MS [(M+H)⁺]: theoretical m/z: 833.3; measured value: 833.3.

### Embodiment 132 (not of the invention)

### Synthesis of compound 6fe

The synthesis method of compound **6fe** was the same with that in Embodiment 1; the product was carried out by one-step catalytic coupling reaction to get product **6fe,** wherein compounds **SM-3cn** (0.2mmol) and **SM-4bf** (0.2mmol) were used in place of compounds **SM-3a** and **SM-4i,** a yellow solid product **6fd** (53mg) was obtained, yield: 35%.
¹H NMR (500 MHz, CDCl₃) of product **6fe:** δ 7.47-7.32 (m, 13H), 6.16 (s, 1H), 5.98 (s, 1H), 5.92 (s, 1H), 5.53 (m, 1H), 5.47 (m, 1H), 5.23 (m, 1H), 4.61 (m, 1H), 4.37 (m, 1H), 3.88 (m, 1H), 3.74 (s, 6H), 2.34-2.03 (m, 6H), 1.27 (s, 6H). MS analysis confirms that **6fe**'s ESI-MS [(M+H)⁺]:: theoretical m/z: 833.3; measured value: 833.3.

### Embodiment 133 (not of the invention)

### Synthesis of compound 6ff

The synthesis method of compound **6ff** was the same with that in Embodiment 1; the product was carried out by one-step catalytic coupling reaction to get product **6ff,** wherein compounds **SM-3cp** (0.2mmol) and **SM-4ag** (0.2mmol) were used in place of compounds **SM-3a** and **SM-4i,** a yellow solid product **6ff** (40mg) was obtained, yield: 26%.
¹H NMR (500 MHz, CDCl₃) of product **6ff:** δ 7.46-7.31 (m, 18H), 6.27 (s, 1H), 6.12 (s, 1H), 5.99 (s, 1H), 5.90 (s, 1H), 5.52 (m, 1H), 5.33 (s, 1H), 4.53 (m, 1H), 4.11 (s, 1H), 3.69 (s, 6H), 2.34-1.99 (m, 6H). MS analysis confirms that **6ff'**s ESI-MS [(M+H)⁺]:: theoretical m/z: 867.3; measured value: 867.3.

### Embodiment 134 (not of the invention)

### Synthesis of compound 6fg

The synthesis method of compound **6fg** was the same with that in Embodiment 1; the product was carried out by one-step catalytic coupling reaction to get product **6fg,** wherein compounds **SM-3cp** (0.2mmol) and **SM-4a** (0.2mmol) were used in place of compounds **SM-3a** and **SM-4i,** a yellow solid product **6fg** (81mg) was obtained, yield: 48%.
¹H NMR (500 MHz, CDCl₃) of product **6fg:** δ 7.47-7.40 (m, 13H), 6.24 (s, 1H), 5.97 (s, 1H), 5.90 (s, 1H), 5.58 (m, 1H), 5.27 (s, 1H), 4.62 (m, 1H), 4.36 (m, 1H), 4.12 (m, 1H), 3.88 (m, 1H), 3.73 (s, 6H), 2.18-2.02 (m, 6H), 1.26 (s, 6H). MS analysis confirms that **6fg'**s ESI-MS [(M+H)⁺]:: theoretical m/z: 833.3; measured value: 833.3.

### Embodiment 135 (not of the invention)

### Synthesis of compound 6fh

The synthesis method of compound **6fh** was the same with that in Embodiment 1; the product was carried out by one-step catalytic coupling reaction to get product **6fh,** wherein compounds **SM-3cb** (0.2mmol) and **SM-4a** (0.2mmol) were used in place of compounds **SM-3a** and **SM-4i,** a yellow solid product **6fh** (63mg) was obtained, yield: 38%.
¹H NMR (500 MHz, CDCl₃) of product **6fh:** δ 7.55-7.13 (m, 8H), 6.07 (s, 1H), 5.98 (s, 1H), 5.57 (s, 1H), 5.28 (s, 1H), 4.79 (m, 1H), 4.60 (m, 1H), 4.39 (m, 1H), 4.33 (s, 1H), 3.73 (s, 6H), 2.39 (m, 1H), 2.25 (m, 1H), 2.11-2.07 (m, 6H), 1.07 (s, 6H), 0.94 (s, 6H). MS analysis confirms that **6fh'**s ESI-MS [(M+H)⁺]:: theoretical m/z: 799.3; measured value: 799.3.

### Embodiment 136 (not of the invention)

### Synthesis of compound 6fi

The synthesis method of compound **6fi** was the same with that in Embodiment 1; the product was carried out by one-step catalytic coupling reaction to get product **6fi,** wherein compounds **SM-3cb** (0.2mmol) and **SM-4ag** (0.2mmol) were used in place of compounds **SM-3a** and **SM-4i,** a yellow solid product **6fi** (59mg) was obtained, yield: 35%.
¹H NMR (500 MHz, CDCl₃) of product **6fi:** δ 7.45-7.39 (m, 13H), 6.18 (s, 1H), 6.06 (s, 1H), 5.95 (s, 1H), 5.61 (m, 1H), 5.30 (m, 1H), 4.77 (m, 1H), 4.56 (m, 1H), 4.30 (m, 1H), 3.70 (s, 3H), 3.63 (s, 3H), 3.22 (s, 1H), 2.25-1.91 (m, 6H), 1.26 (s, 6H). MS analysis confirms that **6fi'**s ESI-MS [(M+H)⁺]:: theoretical m/z: 833.3; measured value: 833.3.

### Embodiment 137 (not of the invention)

### Synthesis of compound 6fj

The synthesis method of compound **6fj** was the same with that in Embodiment 1; the product was carried out by one-step catalytic coupling reaction to get product **6fj,** wherein compounds **SM-3cc** (0.2mmol) and **SM-4b** (0.2mmol) were used in place of compounds **SM-3a** and **SM-4i,** a yellow solid product **6fj** (61mg) was obtained, yield: 37%.
MS analysis confirms that **6fj'**s ESI-MS [(M+H)⁺]:: theoretical m/z: 799.3; measured value: 799.4.

### Embodiment 138 (not of the invention)

### Synthesis of compound 6fk

The synthesis method of compound **6fk** was the same with that in Embodiment 1; the product was carried out by one-step catalytic coupling reaction to get product **6fk,** wherein compounds **SM-3cc** (0.2mmol) and **SM-4bf** (0.2mmol) were used in place of compounds **SM-3a** and **SM-4i,** a yellow solid product **6fk** (59mg) was obtained, yield: 35%.
MS analysis confirms that **6fk'**s ESI-MS [(M+H)⁺]:: theoretical m/z: 833.3; measured value: 833.4.

### Embodiment 139 (not of the invention)

### Synthesis of compound 6fm

The synthesis method of compound **6fm** was the same with that in Embodiment 1; the product was carried out by one-step catalytic coupling reaction to get product **6fm,** wherein compounds **SM-3cd** (0.2mmol) and **SM-4bf** (0.2mmol) were used in place of compounds **SM-3a** and **SM-4i,** a yellow solid product **6fm** (57mg) was obtained, yield: 32%.
MS analysis confirms that **6fm'**s ESI-MS [(M+H)⁺]:: theoretical m/z: 867.3; measured value: 867.5.

### Embodiment 140 (not of the invention)

### Synthesis of compound 6fn

The synthesis method of compound **6fn** was the same with that in Embodiment 1; the product was carried out by one-step catalytic coupling reaction to get product **6fn,** wherein compounds **SM-3cd** (0.2mmol) and **SM-4b** (0.2mmol) were used in place of compounds **SM-3a** and **SM-4i,** a yellow solid product **6fn** (59mg) was obtained, yield: 35%.
¹H NMR (500 MHz, CDCl₃) of product **6fn:** δ 7.20-7.66 (m, 13H), 5.99-6.26 (m, 3H), 5.56-5.58 (m, 1H), 5.31-5.32 (m, 1H), 4.73-4.76 (m, 2H), 4.49-4.51 (m, 1H), 3.79-3.82 (m, 2H), 3.68-3.71 (m, 5H), 3.54 (s, 3H), 1.93-2.04 (m, 5H), 0.90-0.91 (m, 6H). MS analysis confirms that **6fn'**s ESI-MS [(M+H)⁺]:: theoretical m/z: 833.3; measured value: 833.5.

### Embodiment 141 (not of the invention)

### Synthesis of compound 6fp

The synthesis method of compound **6fp** was the same with that in Embodiment 1; the product was carried out by one-step catalytic coupling reaction to get product **6fp,** wherein compounds **SM-3ce** (0.2mmol) and **SM-4bf** (0.2mmol) were used in place of compounds **SM-3a** and **SM-4i,** a yellow solid product **6fp** (60mg) was obtained, yield: 37%.
MS analysis confirms that **6fp**'s ESI-MS [(M+H)⁺]:: theoretical m/z: 817.3; measured value: 817.3.

### Embodiment 142 (not of the invention)

### Synthesis of compound 6fq

The synthesis method of compound **6fq** was the same with that in Embodiment 1; the product was carried out by one-step catalytic coupling reaction to get product **6fq,** wherein compounds **SM-3cf** (0.2mmol) and **SM-4b** (0.2mmol) were used in place of compounds **SM-3a** and **SM-4i,** a yellow solid product **6fq** (52mg) was obtained, yield: 32%.
MS analysis confirms that **6fq'**s ESI-MS [(M+H)⁺]:: theoretical m/z: 817.3; measured value: 817.3.

### Embodiment 143 (not of the invention)

### Synthesis of compound 6fr

The synthesis method of compound **6fr** was the same with that in Embodiment 1; the product was carried out by one-step catalytic coupling reaction to get product **6fr,** wherein compounds **SM-3cf** (0.2mmol) and **SM-4bf** (0.2mmol) were used in place of compounds **SM-3a** and **SM-4i,** a yellow solid product **6fr** (55mg) was obtained, yield: 32%.
MS analysis confirms that **6fr'**s ESI-MS [(M+H)⁺]:: theoretical m/z: 851.3; measured value: 851.3.

### Embodiment 144 (not of the invention)

### Synthesis of compound 6fs

The synthesis method of compound **6fs** was the same with that in Embodiment 1; the product was carried out by one-step catalytic coupling reaction to get product **6fs,** wherein compounds **SM-3cg** (0.2mmol) and **SM-4bf** (0.2mmol) were used in place of compounds **SM-3a** and **SM-4i,** a yellow solid product **6fs** (67mg) was obtained, yield: 39%.
MS analysis confirms that **6fs'**s ESI-MS [(M+H)⁺]: theoretical m/z: 850.3; measured value: 850.5.

### Embodiment 145 (not of the invention)

### Synthesis of compound 6ft

The synthesis method of compound **6ft** was the same with that in Embodiment 1; the product was carried out by one-step catalytic coupling reaction to get product **6ft,** wherein compounds **SM-3ch** (0.2mmol) and **SM-4ag** (0.2mmol) were used in place of compounds **SM-3a** and **SM-4i,** a yellow solid product **6ft** (82mg) was obtained, yield: 48%.
MS analysis confirms that **6ft'**s ESI-MS [(M+H)⁺]: theoretical m/z: 833.3; measured value: 833.5.

### Embodiment 146 (not of the invention)

### Synthesis of compound 6fu

The synthesis method of compound **6fu** was the same with that in Embodiment 1; the product was carried out by one-step catalytic coupling reaction to get product **6fu,** wherein compounds **SM-3cs** (0.25mmol) and **SM-4bn** (0.25mmol) were used in place of compounds **SM-3a** and **SM-4i,** a yellow solid product **6fu** (57mg) was obtained, yield: 25%.
MS analysis confirms that **6fu**'s ESI-MS [(M+H)⁺]: theoretical m/z: 901.3; measured value: 901.4.

### Embodiment 147 (not of the invention)

### Synthesis of compound 6fv

The synthesis method of compound **6fv** was the same with that in Embodiment 1; the product was carried out by one-step catalytic coupling reaction to get product **6fv,** wherein compounds **SM-3cu** (0.25mmol) and **SM-4bp** (0.25mmol) were used in place of compounds **SM-3a** and **SM-4i,** a yellow solid product **6fv** (48mg) was obtained, yield: 21%.
MS analysis confirms that **6fv'**s ESI-MS [(M+H)⁺]: theoretical m/z: 901.3; measured value: 901.4.

### Embodiment 148 (not of the invention)

### Synthesis of compound 6fw

The synthesis method of compound **6fw** was the same with that in Embodiment 1; the product was carried out by one-step catalytic coupling reaction to get product **6fw,** wherein compounds **SM-3cu** (0.25mmol) and **SM-4bq** (0.25mmol) were used in place of compounds **SM-3a** and **SM-4i,** a yellow solid product **6fw** (72mg) was obtained, yield: 32%.
MS analysis confirms that **6fw'**s ESI-MS [(M+H)⁺]: theoretical m/z: 897.3; measured value: 897.4.

### Embodiment 149 (not of the invention)

### Synthesis of compound 6fx

The synthesis method of compound **6fx** was the same with that in Embodiment 1; the product was carried out by one-step catalytic coupling reaction to get product **6fx,** wherein compounds **SM-3cs** (0.25mmol) and **SM-4br** (0.25mmol) were used in place of compounds **SM-3a** and **SM-4i,** a yellow solid product **6fx** (81mg) was obtained, yield: 36%.
MS analysis confirms that **6fx'**s ESI-MS [(M+H)⁺]: theoretical m/z: 941.3; measured value: 941.4.

### Embodiment 150 (not of the invention)

### Synthesis of compound 6fy

The synthesis method of compound **6fy** was the same with that in Embodiment 1; the product was carried out by one-step catalytic coupling reaction to get product **6fy,** wherein compounds **SM-3cs** (1.0mmol) and **SM-4ag** (1.0mmol) were used in place of compounds **SM-3a** and **SM-4i,** a yellow solid product **6fy** (374mg) was obtained, yield: 43%.
¹H NMR (500 MHz, CDCl₃) of product **6fy:** δ 7.19-7.84 (m, 15H), 5.92-6.17 (m, 3H), 5.55-5.68 (m, 2H), 4.54-4.68 (m, 1H), 4.39-4.41 (m, 1H), 4.12-4.15 (m, 1H), 3.95-3.98 (m, 1H), 3.63-3.71 (m, 7H), 2.81 (m, 1H), 2.42-2.43 (m, 1H), 2.28-2.31 (m, 1H), 2.10-2.16 (m, 2H), 0.93-0.97 (m, 6H). MS analysis confirms that **6fy'**s ESI-MS [(M+H)⁺]:: theoretical m/z: 883.3; measured value: 883.4.

### Embodiment 151 (not of the invention)

### Synthesis of compound 6fz

The synthesis method of compound **6fz** was the same with that in Embodiment 1; the product was carried out by one-step catalytic coupling reaction to get product **6fz,** wherein compounds **SM-3cs** (0.35mmol) and **SM-4a** (0.35mmol) were used in place of compounds **SM-3a** and **SM-4i,** a yellow solid product **6fz** (200mg) was obtained.
¹H NMR (500 MHz, CDCl₃) of product **6fz:** δ 7.33-7.86 (m, 10H), 6.08-6.27 (m, 3H), 5.57-5.61 (m, 1H), 5.27 (m, 1H), 4.81-4.83 (m, 1H), 4.56-4.59 (m, 1H), 4.20-4.38 (m, 2H), 3.66-3.89 (m, 7H), 2.39 (m, 1H), 2.26 (m, 1H), 2.02-2.06 (m, 4H), 1.05-1.10 (m, 3H), 0.84-0.96 (m, 9H). MS analysis confirms that **6fz'**s ESI-MS [(M+H)⁺]: theoretical m/z: 849.3; measured value: 849.4.

### Embodiment 152 (not of the invention)

### Synthesis of compound 6ga

The synthesis method of compound **6ga** was the same with that in Embodiment 1; the product was carried out by one-step catalytic coupling reaction to get product **6ga,** wherein compounds **SM-3ct** (0.35mmol) and **SM-4a** (0.35mmol) were used in place of compounds **SM-3a** and **SM-4i,** a yellow solid product **6ga** (190mg) was obtained.
¹H NMR (500 MHz, CDCl₃) of product **6ga:** δ 7.16-7.86 (m, 15H), 5.97-6.26 (m, 3H), 5.47-5.62 (m, 2H), 5.26-5.29 (m, 1H), 4.58-4.62 (m, 1H), 4.36 (m, 1H), 4.03-4.22 (m, 1H), 3.64-3.89 (m, 7H), 2.36-2.40 (m, 1H), 2.21-2.24 (m, 1H), 2.02-2.11 (m, 3H), 0.83-0.91 (m, 6H). MS analysis confirms that **6ga'**s ESI-MS [(M+H)⁺]:: theoretical m/z: 883.3; measured value: 883.4.

### Embodiment 153 (not of the invention)

### Synthesis of compound 6gb

The synthesis method of compound **6gb** was the same with that in Embodiment 1; the product was carried out by one-step catalytic coupling reaction to get product **6gb,** wherein compounds **SM-3ct** (0.44mmol) and **SM-4ag** (0.44mmol) were used in place of compounds **SM-3a** and **SM-4i,** a yellow solid product **6gb** (180mg) was obtained.
¹H NMR (500 MHz, CDCl₃) of product **6gb:** δ 7.13-7.97 (m, 20H), 5.95-6.26 (m, 4H), 5.33-5.53 (m, 3H), 4.55-4.63 (m, 1H), 4.01-4.21 (m, 1H), 3.64-3.79 (m, 7H), 2.21-2.26 (m, 1H), 1.90-2.04 (m, 3H). MS analysis confirms that **6gb'**s ESI-MS [(M+H)⁺]: theoretical m/z: 917.3; measured value: 917.4.

### Embodiment 154 (not of the invention)

### Synthesis of compound 6gc

The synthesis method of compound **6gc** was the same with that in Embodiment 1; the product was carried out by one-step catalytic coupling reaction to get product **6gc,** wherein compounds **SM-3cu** (12.5mmol) and **SM-4bf** (12.5mmol) were used in place of compounds **SM-3a** and **SM-4i,** a yellow solid product **6gc** (4.3g) was obtained, yield: 39%.
¹H NMR (500 MHz, CDCl₃) of product **6gc:** δ 7.61-7.26 (m, 10H), 6.09 (m, 1H), 6.01 (m, 1H), 5.54 (m, 1H), 5.46 (m, 1H), 4.77 (m, 1H), 4.56 (m, 1H), 4.39 (m, 1H), 4.33 (m, 1H), 3.93 (m, 1H), 3.71 (d, 6H), 2.92 (m, 1H), 2.42-2.04 (m, 7H), 1.26 (d, 12H). MS analysis confirms that **6gc'**s ESI-MS [(M+H)⁺]:: theoretical m/z: 883.3; measured value: 883.4.

### Embodiment 155 (not of the invention)

### Synthesis of compound 6gd

The synthesis method of compound **6gd** was the same with that in Embodiment 1; the product was carried out by one-step catalytic coupling reaction to get product **6gd,** wherein compounds **SM-3cu** (0.44mmol) and **SM-4b** (0.44mmol) were used in place of compounds **SM-3a** and **SM-4i,** a yellow solid product **6gd** (100mg) was obtained, yield: 26%.
¹H NMR (500 MHz, CDCl₃) of product **6gd:** δ 7.61-7.26 (m, 10H), 6.09 (m, 1H), 6.01 (m, 1H), 5.54 (m, 1H), 5.46 (m, 1H), 4.77 (m, 1H), 4.56 (m, 1H), 4.39 (m, 1H), 4.33 (m, 1H), 3.93 (m, 1H), 3.71 (d, 6H), 2.92 (m, 1H), 2.42-2.04 (m, 7H), 1.26 (d, 12H). MS analysis confirms that **6gd'**s ESI-MS [(M+H)⁺]:: theoretical m/z: 849.3; measured value: 849.4.

### Embodiment 156 (not of the invention)

### Synthesis of compound 6ge

The synthesis method of compound **6ge** was the same with that in Embodiment 1; the product was carried out by one-step catalytic coupling reaction to get product **6ge,** wherein compounds **SM-3cv** (0.37mmol) and **SM-4b** (0.37mmol) were used in place of compounds **SM-3a** and **SM-4i,** a yellow solid product **6ge** (40mg) was obtained, yield: 12%.
¹H NMR (500 MHz, CDCl₃) of product **6ge:** δ 7.71-7.24 (m, 15H), 6.09 (m, 2H), 5.53 (m, 2H), 4.76 (d, 1H), 4.53 (s, 1H), 4.31 (m, 1H), 4.13 (m, 1H), 3.74 (d, 6H), 3.32 (m, 1H), 2.89 (m, 1H), 2.31-1.99 (m, 6H), 1.28 (d, 6H). MS analysis confirms that **6ge**'s ESI-MS [(M+H)⁺]: theoretical m/z: 883.3; measured value: 883.4.

### Embodiment 157 (not of the invention)

### Synthesis of compound 6gf

The synthesis method of compound **6gf** was the same with that in Embodiment 1; the product was carried out by one-step catalytic coupling reaction to get product **6gf,** wherein compounds **SM-3cv** (0.39mmol) and **SM-4bf** (0.39mmol) were used in place of compounds **SM-3a** and **SM-4i,** a yellow solid product **6gf** (50mg) was obtained, yield: 14%.
MS analysis confirms that **6gf'**s ESI-MS [(M+H)⁺]:: theoretical m/z: 917.3; measured value: 917.4.

### Embodiment 158 (not of the invention)

### Synthesis of compound 6gg

The synthesis method of compound **6gg** was the same with that in Embodiment 1; the product was carried out by one-step catalytic coupling reaction to get product **6gg,** wherein compounds **SM-3cm** (0.39mmol) and **SM-4bg** (0.39mmol) were used in place of compounds **SM-3a** and **SM-4i,** a yellow solid product **6gg** (31mg) was obtained, yield: 16%.
¹H NMR (500 MHz, CDCl₃) of product **6gg:** δ 7.26-7.47 (m, 11H), 6.10-6.33 (m, 3H), 5.27-5.44 (m, 2H), 4.78-4.81 (m, 1H), 4.46-4.58 (m, 1H), 4.19-4.31 (m, 1H), 3.66-3.77 (m, 7H), 3.21-3.23 (m, 1H), 2.81-2.94 (m, 1H), 2.20-2.23 (m, 1H), 2.07 (m, 1H), 1.88-1.91 (m, 2H), 0.84-0.89 (m, 6H). MS analysis confirms that **6gg'**s ESI-MS [(M+H)⁺]: theoretical m/z: 807.3; measured value: 807.4.

### Embodiment 159 (not of the invention)

### Synthesis of compound 6gh

The synthesis method of compound **6gh** was the same with that in Embodiment 1; the product was carried out by one-step catalytic coupling reaction to get product **6gg,** wherein compounds **SM-3cn** (0.86mmol) and **SM-4bg** (0.86mmol) were used in place of compounds **SM-3a** and **SM-4i,** a yellow solid product **6gh** (190mg) was obtained, yield: 29%.
¹H NMR (500 MHz, CDCl₃) of product **6gh:** δ 7.14-7.70 (m, 6H), 6.06-6.23 (m, 3H), 5.56-5.58 (m, 1H), 5.23 (m, 1H), 4.80 (m, 1H), 4.55-4.61 (m, 1H), 4.18-4.38 (m, 2H), 3.60-3.87 (m, 7H), 2.34-2.37 (m, 1H), 2.18-2.21 (m, 1H), 2.00-2.10 (m, 4H), 0.88-0.92 (m, 12H). MS analysis confirms that **6gh'**s ESI-MS [(M+H)⁺]: theoretical m/z: 773.3; measured value: 773.4.

### Embodiment 160 (not of the invention)

### Synthesis of compound 6gi

The synthesis method of compound **6gi** was the same with that in Embodiment 1; the product was carried out by one-step catalytic coupling reaction to get product **6gi,** wherein compounds **SM-3cn** (0.80mmol) and **SM-4bh** (0.80mmol) were used in place of compounds **SM-3a** and **SM-4i,** a yellow solid product **6gi** (260mg) was obtained, yield: 42%.
¹H NMR (500 MHz, CDCl₃) of product **6gi:** δ 7.00-7.54 (m, 11H), 5.97-6.28 (m, 3H), 5.45-5.55 (m, 2H), 5.24 (m, 1H), 4.57-4.60 (m, 1H), 4.35 (m, 1H), 3.64-3.88 (m, 7H), 3.53-3.55 (m, 1H), 2.88-2.92 (m, 1H), 2.34-2.35 (m, 1H), 2.19-2.22 (m, 1H), 2.04-2.09 (m, 2H), 0.88-0.96 (m, 6H). MS analysis confirms that **6gi'**s ESI-MS [(M+H)⁺]:: theoretical m/z: 807.3; measured value: 807.3.

### Embodiment 161 (not of the invention)

### Synthesis of compound 6gj

The synthesis method of compound **6gj** was the same with that in Embodiment 1; the product was carried out by one-step catalytic coupling reaction to get product **6gj,** wherein compounds **SM-3ca** (0.31mmol) and **SM-4bs** (0.31mmol) were used in place of compounds **SM-3a** and **SM-4i,** a yellow solid product **6gj** (35mg) was obtained, yield: 14%.
MS analysis confirms that **6gj'**s ESI-MS [(M+H)⁺]: theoretical m/z: 807.3; measured value: 807.4.

### Embodiment 162 (not of the invention)

### Synthesis of compound 6gk

The synthesis method of compound **6gk** was the same with that in Embodiment 1; the product was carried out by one-step catalytic coupling reaction to get product **6gk,** wherein compounds **SM-3cw** (0.31mmol) and **SM-4bs** (0.31mmol) were used in place of compounds **SM-3a** and **SM-4i,** a yellow solid product **6gk** (30mg) was obtained, yield: 11%.
MS analysis confirms that **6gk'**s ESI-MS [(M+H)⁺]:: theoretical m/z: 841.3; measured value: 841.4.

### Embodiment 163 (not of the invention)

### Synthesis of compound 6gm

The synthesis method of compound **6gm** was the same with that in Embodiment 1; the product was carried out by one-step catalytic coupling reaction to get product **6gm,** wherein compounds **SM-3ca** (0.33mmol) and **SM-4bt** (0.33mmol) were used in place of compounds **SM-3a** and **SM-4i,** a yellow solid product **6gm** (50mg) was obtained, yield: 20%.
MS analysis confirms that **6gm'**s ESI-MS [(M+H)⁺]: theoretical m/z: 773.3; measured value: 773.4.

### Embodiment 164 (not of the invention)

### Synthesis of compound 6gn

The synthesis method of compound **6gn** was the same with that in Embodiment 1; the product was carried out by one-step catalytic coupling reaction to get product **6gn,** wherein compounds **SM-3cw** (0.25mmol) and **SM-4bt** (0.25mmol) were used in place of compounds **SM-3a** and **SM-4i,** a yellow solid product **6gn** (29mg) was obtained, yield: 14%.
MS analysis confirms that **6gn'**s ESI-MS [(M+H)⁺]: theoretical m/z: 807.3; measured value: 807.4.

### Embodiment 165 (not of the invention)

### Synthesis of compound 6gp

The synthesis method of compound **6gp** was the same with that in Embodiment 1; the product was carried out by one-step catalytic coupling reaction to get product **6gp,** wherein compounds **SM-3cw** (0.25mmol) and **SM-4bt** (0.25mmol) were used in place of compounds **SM-3a** and **SM-4i,** a yellow solid product **6gp** (32mg) was obtained, yield: 13%.
MS analysis confirms that **6gp'**s ESI-MS [(M+H)⁺]: theoretical m/z: 933.3; measured value: 933.4.

### Embodiment 166 (not of the invention)

### Synthesis of compound 6gq

The synthesis method of compound **6gq** was the same with that in Embodiment 1; the product was carried out by one-step catalytic coupling reaction to get product **6gq,** wherein compounds **SM-3cu** (0.25mmol) and **SM-4bw** (0.25mmol) were used in place of compounds **SM-3a** and **SM-4i,** a yellow solid product **6gq** (37mg) was obtained, yield: 15%.
MS analysis confirms that **6gq'**s ESI-MS [(M+H)⁺]:: theoretical m/z: 933.3; measured value: 933.4.

### Embodiment 167 (not of the invention)

### Synthesis of compound Ref-3

The synthesis method of compound **Ref-3** was the same with that in Embodiment 1; the product was carried out by one-step catalytic coupling reaction to get product **Ref-3,** wherein compounds **SM-3cm** (0.2mmol) and **SM-4ag** (0.2mmol) were used in place of compounds **SM-3a** and **SM-4i,** a yellow solid product **Ref-3** (69mg) was obtained, yield: 40%.
¹H NMR (500 MHz, CDCl₃) of product **Ref-3:** δ 10.53 (s, 1H), 7.75-7.14 (m, 17H), 6.13 (m, 2H), 5.46 (m, 2H), 5.31 (m, 2H), 3.80 (m, 6H), 3.23 (m, 2H), 2.91 (m, 2H), 2.23-1.67 (m, 12H). MS analysis confirms that **Ref-3**'s ESI-MS [(M+H)⁺]:: theoretical m/z: 869.3; measured value: 869.3.

### Embodiment of treatment effect

The autonomous replication level of HCV in liver cells in vitro is very low, and the only animal that can be infected by HCV is chimpanzee, therefore there is no appropriate animal model currently available for preclinical pharmacodynamic study. Some researchers have transplanted human liver tissue infected with HCV in vitro into immune deficient mice to establish an in vivo mice model, but these mice are hard to raise and the model is unstable and short of normal immunologic reaction. Moreover, the model has significant difference with the pathogenic process of hepatitis C, therefore, it has not been used for animal model to assess the action of anti-HCV medicine yet. The progress of the development of anti-viral drugs for the treatment of HCV infection had been very slow because the pathogenic mechanism and life cycle of HCV were unclear before 1999 due to the lack of cell culture system for effective multiplication of HCV. But researchers managed to achieve a breakthrough in 1999 after numerous attempts. An effective cell culture model-replicon system in which HCV is capable of autonomous replication in transfected human hepatic carcinoma cell line Huh-7 had been established in that year on the basis of subgenomic HCV RNA built with genetic engineering.
The above-mentioned cell culture model-replicon system which has been widely accepted in the industry is used in this invention for ex vivo experiments and subsequent assessment of drugs based on the experiment results. The resulted ex vivo experiment results for HCV NS5, a target site of action of anti-HCV drugs include:
1) inhibitory action(IC₅₀) of the compounds on the activity of HCV NS5 replicase;
2) inhibitory action(EC₅₀) of the compounds on HCV NS5 replicon;
Currently available foreign preclinical and clinical studies suggest the ex vivo experiment results are consistent with relevant in vivo activity tests.
The therapeutic effect of the compounds of this invention on HCV infection can be preliminarily assessed through the following preclinical in vitro inhibitory action test and can be further confirmed in clinical trials. Other methods used in the invention are also quite familiar to professionals in this field with ordinary technical knowledge.

### Method for testing the anti-viral activity(EC₅₀) in HCV NS5A replicon system:

The viral replication level in infected cells was determined by testing Renilla luciferase with the newly established dual-reporter gene replicon system. There was a good linear relation between the expression level of the reporter gene and the HCV RNA replication level and viral protein expression level. The anti-viral activity was determined for 3 replicates in 3 replicate cells at five(5) 1:2 diluted concentration gradients, with 1 to 2 positive controls. The EC₅₀ of the compounds was calculated in the end.
The compounds of the invention, or their stereoisomers, tautomers, esterified or amidated prodrugs, or pharmaceutically acceptable salts and mixtures have been subjected to test to determined their therapeutic effects in the treatment of HCV infection. The results show they have significant HCV NS5 inhibitory effect. Moreover, the results of **6a-6ep(Ia), 6fa-6gq(Ib)** and reference compounds **Ref-1(BMS-790052), Ref-2(GS5885), Ref-3** in HCV-NS5 inhibition test reveal that the cyclopentanyl/hexamethyleneamino radical containing linear polypeptide compounds **6a-6ep, 6fa-6gq** and reference compounds **Ref-1, Ref-2, Ref-3** have good HCV inhibitory results. The detailed testing results of the HCV-NS5A inhibitory activity of compounds **6a-6ep** and reference compounds **Ref-1, Ref-2, Ref-3** are listed in Table 1 as shown below; in the Table, the results are marked with an **"A"** when the inhibitory activity(EC₅₀) is ≥ 50nM, or with a **"B"** when the inhibitory activity(EC₅₀) is in the range of 1.0-49.9nM, or with a "C" when the inhibitory activity(EC₅₀) is in the range of 0.001-0.999nM. The replicons GT-1a, GT-1b, GT-2a, GT-3a, GT-4a, GT-5a and GT-6a used for the test are routine replicons commercially available in this field. The detailed testing data of HCV NS5A replicon inhibition test are as shown in Table 1 and Table 2.

**Table 1: HCV NS5A replicon inhibitory activity test results of compounds (6a-6gq) of the invention**

| No. | Compound NO. | NS5A Inhibitory Activity (EC₅₀ of GT-1a subtype inhibition) | No. | Compound NO. | NS5A Inhibitory Activity (EC₅₀ of GT-1a subtype inhibition) |
|---|---|---|---|---|---|
| 1 | 6a | C | 86 | 6cu | C |
| 2 | 6b | A | 87 | 6cv | C |
| 3 | 6c | A | 88 | 6cw | C |
| 4 | 6d | B | 89 | 6cx | C |
| 5 | 6e | A | 90 | 6cy | C |
| 6 | 6f | A | 91 | 6cz | C |
| 7 | 6g | A | 92 | 6da | C |
| 8 | 6h | A | 93 | 6db | C |
| 9 | 6i | C | 94 | 6dc | C |
| 10 | 6j | A | 95 | 6dd | C |
| 11 | 6k | A | 96 | 6de | C |
| 12 | 6m | B | 97 | 6df | C |
| 13 | 6n | A | 98 | 6dg | C |
| 14 | 6p | A | 99 | 6dh | C |
| 15 | 6q | A | 100 | 6di | C |
| 16 | 6r | A | 101 | 6dj | B |
| 17 | 6s | C | 102 | 6dk | C |
| 18 | 6t | A | 103 | 6dm | C |
| 19 | 6u | B | 104 | 6dn | C |
| 20 | 6v | A | 105 | 6dp | C |
| 21 | 6w | A | 106 | 6dq | C |
| 22 | 6x | C | 107 | 6dr | C |
| 23 | 6y | C | 108 | 6ds | C |
| 24 | 6z | C | 109 | 6dt | C |
| 25 | 6aa | C | 110 | 6du | C |
| 26 | 6ab | C | 111 | 6dv | C |
| 27 | 6ac | C | 112 | 6dw | C |
| 28 | 6ad | C | 113 | 6dy | C |
| 29 | 6ae | C | 114 | 6dz | C |
| 30 | 6af | C | 115 | 6ea | C |
| 31 | 6ag | C | 116 | 6eb | C |
| 32 | 6ah | C | 117 | 6ec | C |
| 33 | 6ai | C | 118 | 6ed | C |
| 34 | 6aj | C | 119 | 6ee | C |
| 35 | 6ak | C | 120 | 6ef | C |
| 36 | 6am | C | 121 | 6eg | C |
| 37 | 6an | C | 122 | 6eh | C |
| 38 | 6ap | C | 123 | 6ei | C |
| 39 | 6aq | C | 124 | 6ej | C |
| 40 | 6ar | C | 125 | 6ek | B |
| 41 | 6as | C | 126 | 6em | B |
| 42 | 6at | C | 127 | 6en | B |
| 43 | 6au | C | 128 | 6ep | B |
| 44 | 6av | C | 129 | 6fa | C |
| 45 | 6aw | C | 130 | 6fb | C |
| 46 | 6ax | C | 131 | 6fc | C |
| 47 | 6ay | C | 132 | 6fd | C |
| 48 | 6az | C | 133 | 6fe | C |
| 49 | 6ba | C | 134 | 6ff | C |
| 50 | 6bb | A | 135 | 6fg | C |
| 51 | 6bc | B | 136 | 6fh | C |
| 52 | 6bd | C | 137 | 6fi | C |
| 53 | 6be | C | 138 | 6fj | C |
| 54 | 6bf | A | 139 | 6fk | C |
| 55 | 6bg | A | 140 | 6fm | C |
| 56 | 6bh | C | 141 | 6fn | C |
| 57 | 6bi | C | 142 | 6fp | C |
| 58 | 6bj | C | 143 | 6fq | C |
| 59 | 6bk | C | 144 | 6fr | C |
| 60 | 6bm | C | 145 | 6fs | C |
| 61 | 6bn | C | 146 | 6ft | C |
| 62 | 6bp | C | 147 | 6fu | C |
| 63 | 6bq | C | 148 | 6fv | C |
| 64 | 6br | C | 149 | 6fw | C |
| 65 | 6bs | A | 150 | 6fx | C |
| 66 | 6bt | C | 151 | 6fy | C |
| 67 | 6bu | C | 152 | 6fz | C |
| 68 | 6bv | C | 153 | 6ga | C |
| 69 | 6bw | C | 154 | 6gb | C |
| 70 | 6bx | C | 155 | 6gc | C |
| 71 | 6by | C | 156 | 6ge | C |
| 72 | 6bz | C | 157 | 6gf | C |
| 73 | 6ca | C | 158 | 6gg | C |
| 74 | 6cb | A | 159 | 6gh | C |
| 75 | 6cc | A | 160 | 6gi | C |
| 76 | 6cd | A | 161 | 6gi | C |
| 77 | 6ce | C | 162 | 6gk | C |
| 78 | 6cf | C | 163 | 6gm | C |
| 79 | 6cg | C | 164 | 6gn | C |
| 80 | 6ch | C | 165 | 6gp | C |
| 81 | 6ci | C | 166 | 6gq | C |
| 82 | 6cj | C | 167 | Ref-1 | C |
| 83 | 6ck | C | 168 | Ref-2 | C |
| 84 | 6cm | C | 169 | Ref-3 | C |
| 85 | 6cq | B | 170 | Ref-4 | C |

**Table 2: The highly-effective HCV NS5A replicon inhibiting compounds of this invention and their activity test results**

| No. | Compound NO. | Inhibitory activity against various HCV-NS5A subtypes(EC₅₀, GT-1a to GT-6a) | | | | | | |
|---|---|---|---|---|---|---|---|---|
| | | GT-1a | GT-1b | GT-2a | GT-3a | GT-4a | GT-5a | GT-6a |
| 1 | 6ba | 39 | 12 | 16 | 173 | 10 | 23 | 221 |
| 2 | 6dy | 11 | 14 | 6 | 15 | 8 | 23 | 24 |
| 3 | 6fc | 21 | 11 | 5 | 15 | 8 | 23 | 111 |
| 4 | 6fd | 20 | 5 | 7 | 26 | 5 | 13 | 55 |
| 5 | 6fg | 9 | 13 | 8 | 24 | 12 | 29 | 23 |
| 6 | 6fz | 7 | N/A | N/A | N/A | N/A | N/A | N/A |
| 7 | 6gc | 11 | N/A | N/A | N/A | N/A | N/A | N/A |
| 8 | 6gd | 4 | N/A | N/A | N/A | N/A | N/A | N/A |
| 9 | Ref-1 | 52 | 21 | 29 | 218 | 11 | 36 | 118 |
| 10 | Ref-2 | 88 | N/A | N/A | N/A | N/A | N/A | N/A |
| 11 | Ref-3 | 51 | N/A | N/A | N/A | N/A | N/A | N/A |
| 12 | Ref-4 | 22 | N/A | N/A | N/A | N/A | N/A | N/A |

| | | | | | | | | |
|---|---|---|---|---|---|---|---|---|
| N/A: Not Available. | | | | | | | | |

The results in Table 2 suggest that compounds **6a-6ep(Ia)** and **6fa-6gq(Ib)** of this invention have excellent HCV NS5A inhibitory activity and are among the novel HCV NS5A inhibitors that have good activity; some compounds (e.g. compounds **6dy, 6fg, 6fz, 6gc** and **6gd** in Table 2), in particular, have inhibitory activity significantly superior to that of **Ref-1**(BMS-790052), **Ref-2(GS5885), Ref-3** and **Ref-4** (Idenix compound IDX-719), therefore some novel compounds (e.g. **6dy, 6fg, 6fz, 6gc** and **6gd)** designed and prepared in this invention are of value that deserves further test and promotion.

### MTD screening test

**Method:** ICR mice, 10 mice/group, 5 males and 5 females. A treatment group and a control group are set up for every drugs, respectively, mice in control group were administered 0.5%CMC-Na solution, i.e. the solvent of the drugs. The mice were fasted overnight but granted free access to water before the administration. Their body weight before the administration of drugs is in the range of 18.8∼24.1 g. The mice were administered the drugs by peroral lavage at the dosage of 40 mL/kg. The mice were closely observed 3 hours after the administration and twice a day afterwards for 7 days, one in the morning and one in the afternoon. At the end of the observation period, 2 mice (1 male and 1 female) were randomly selected from each treatment group and subjected to histopathological examination of part of their tissues and organs.
In order to test the toxicity of some of the novel heterocyclic compounds 6a-6ep(Ia), 6fa-6gq(Ib) of this invention and some highly active compounds in reference compounds Ref-1, Ref-2, Ref-3 (e.g.: 6ba, 6bx, 6by, 6bz, 6dy, 6fb, 6fc, 6fd, 6fg, 6ft), healthy mice of body weight 18-22g were subjected to administration of the drugs by lavage at daily single dosage of 2000mg/kg for 5 consecutive days and close observations for 7 consecutive days for assessment of the acute toxicity of the trial drugs on body based on the mice's toxic reaction (Acute Toxicity Study, MTD). The results show the overall toxicity of the compound group of the invention is very low (LD50 > 10000), most mice (80%-100%) survive the administration. Two third of the novel heterocyclic compounds that have good HCV inhibiting activity (EC50: <0.05nM) after administration of the drugs by lavage at the dosage of 2000mg/kg yield a survival rate of 100%. The experiment results show the compounds of the invention have good therapeutic effect in the treatment of HCV infection and demonstrate significant inhibitory effect against HCV NS5A. Two third of the novel compounds that show high HCV inhibiting activity have very low overall toxicity (survive rate of mice after administration: 100%). It is believed that these compounds are generally nontoxic.

## Claims

1. A chemical compound represented by Formula **Ia** selected from the group consisting of:
| | |
|---|---|
| **Ia-70** | 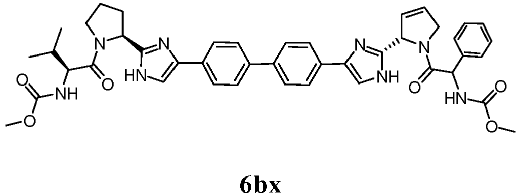 |
| **Ia-71** | 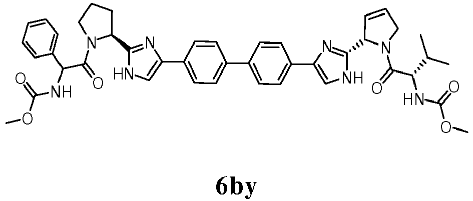 |
| **Ia-113** | 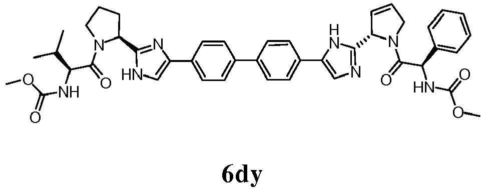 |
| **Ia-115** | 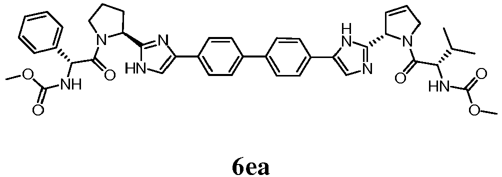 |
its stereoisomers, tautomers, pharmaceutically acceptable salts, or its isotopic substitutions in which the hydrogen, oxygen or nitrogen atom is replaced by its corresponding isotope.

2. A chemical compound as represented by Formula **Ia** in claim 1, its stereoisomers, tautomers, pharmaceutically acceptable salts for use in the preparation of HCV inhibitor drugs.

3. A pharmaceutical composition comprising the chemical compounds as represented by Formula **Ia** in claim 1 or 2, their stereoisomers, tautomers, pharmaceutically acceptable salts, and pharmaceutical acceptable excipient.

4. A pharmaceutical composition according to claim 3, which includes: Immunomodulators, hepatitis C virus (HCV) NS3/4A inhibitors, HCV-NS5B inhibitors, HCV inhibitors of the nucleoside and non-nucleoside and nucleoside derivatives, hepatitis B viral (HBV) inhibitors, antiviral human immunodeficiency virus (HIV) inhibitors, anti-cancer drugs and anti-inflammatory drugs.

5. A pharmaceutical composition according to claim 4, wherein:
- the described immunomodulators are interferon or interferon derivatives;
- the described HBV inhibitors include Lamivudine, Telbivudine, Adefovir Dipivoxil, Emtricitabine, Entecavir, Tenofovir and Telbivudine;
- the described anti-HIV inhibitors include ritonavir and/or ribavirin;
- the described HCV protease inhibitors include VX-950, ZN2007, ABT-450, RG-7227, TMC-435, MK-5172, MK-7009, ACH-1625, GS-9256, TG2349, BMS-650032, IDX320, Yimitasvir phosphate capsule or Seraprevir potassium;
- the described hepatitis C virus polymerase inhibitor comprises GS-5885, TMC647055, ABT-267, BMS-791325, PPI-383 or ALS-002158.

6. A pharmaceutical composition according to claim 5, wherein the described interferon is pegylated interferon.

7. A pharmaceutical composition according to Claim 3-6 for use in preparation of antiviral HCV inhibitors.

## Patentansprüche

1. Chemische Verbindung, dargestellt durch Formel Ia, ausgewählt aus der Gruppe bestehend aus:
| | |
|---|---|
| **Ia-70** | 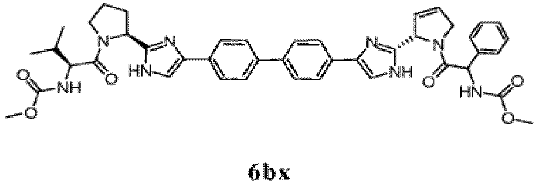 |
| **Ia-71** | 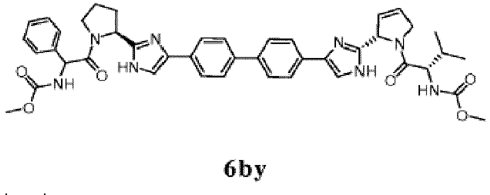 |
| **Ia-113** | 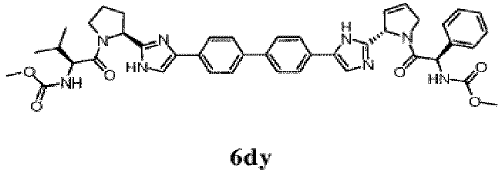 |
| **Ia-115** | 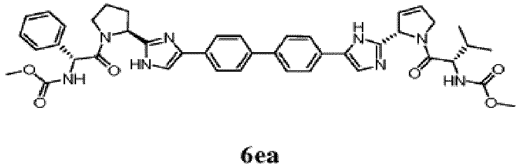 |
deren Stereoisomeren, Tautomeren, pharmazeutisch verträglichen Salzen oder deren Isotopensubstitutionen, bei denen das Wasserstoff-, Sauerstoff- oder Stickstoffatom durch dessen entsprechende Isotope ersetzt wird.

2. Chemische Verbindung, wie sie durch Formel Ia in Anspruch 1 dargestellt ist, deren Stereoisomere, Tautomere, pharmazeutisch verträgliche Salze zur Verwendung bei der Herstellung von HCV-Inhibitor-Arzneimitteln.

3. Pharmazeutische Zusammensetzung, umfassend die chemischen Verbindungen, wie sie durch Formel Ia in Anspruch 1 oder 2 dargestellt sind, deren Stereoisomere, Tautomere, pharmazeutisch verträgliche Salze und einen pharmazeutisch verträglichen Hilfsstoff.

4. Pharmazeutische Zusammensetzung nach Anspruch 3, enthaltend: Immunmodulatoren, Hepatitis-C-Virus (HCV) NS3/4A-Inhibitoren, HCV-NS5B-Inhibitoren, HCV-Inhibitoren des Nukleosids und Nicht-Nukleosids und Nukleosid-Derivate, Hepatitis-B-Virus-(HBV)-Inhibitoren, antivirale Humane Immundefizienz-Virus-(HIV)-Inhibitoren, Anti-Krebsmedikamente und entzündungshemmende Medikamente.

5. Pharmazeutische Zusammensetzung nach Anspruch 4, wobei:
- die beschriebenen Immunmodulatoren Interferon oder Interferonderivate sind;
- die beschriebenen HBV-Inhibitoren Lamivudin, Telbivudin, Adefovir-Dipivoxil, Emtricitabin, Entecavir, Tenofovir und Telbivudin enthalten;
- die beschriebenen Anti-HIV-Inhibitoren Ritonavir und/oder Ribavirin enthalten;
- die beschriebenen HCV-Proteaseinhibitoren VX-950, ZN2007, ABT-450, RG- 7227, TMC-435, MK-5172, MK- 7009, ACH-1625, GS-9256, TG2349, BMS-650032, IDX320, Yimitasvir-Phosphatkapsel oder Seraprevir-Kalium enthalten;
- der beschriebenen Hepatitis-C-Virus-Polymerase-Inhibitor GS-5885, TMC647055, ABT-267, BMS-791325, PPI-383 oder ALS-002158 umfasst.

6. Pharmazeutische Zusammensetzung nach Anspruch 5, wobei das beschriebene Interferon pegyliertes Interferon ist.

7. Pharmazeutische Zusammensetzung nach Anspruch 3-6 zur Verwendung bei der Herstellung von antiviralen HCV-Inhibitoren.

## Revendications

1. Composé chimique représenté par la Formule la sélectionné dans le groupe constitué par :
| | |
|---|---|
| **Ia-70** | 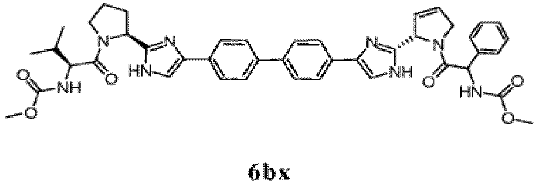 |
| **Ia-71** | 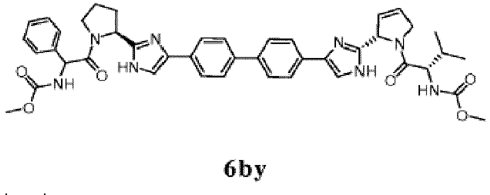 |
| **Ia-113** | 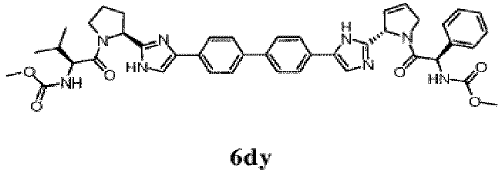 |
| **Ia-115** | 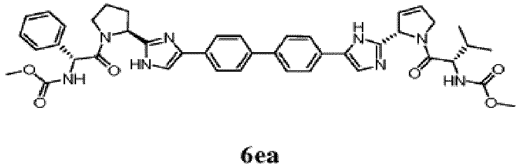 |
ses stéréo-isomères, tautomères, sels pharmaceutiquement acceptables ou ses substitutions isotopiques dans lesquels l'atome d'hydrogène, d'oxygène ou d'azote est remplacé par son isotope correspondant.

2. Composé chimique représenté par la Formule la dans la revendication 1, ses stéréo-isomères, tautomères, sels pharmaceutiquement acceptables pour une utilisation dans la préparation de médicaments inhibiteurs du VHC.

3. Composition pharmaceutique comprenant les composés chimiques tels que représentés par la Formule la dans la revendication 1 ou 2, leurs stéréo-isomères, tautomères, sels pharmaceutiquement acceptables et un excipient pharmaceutiquement acceptable.

4. Composition pharmaceutique selon la revendication 3, qui inclut : des immunomodulateurs, des inhibiteurs NS3/4A du virus de l'hépatite C (VHC), des inhibiteurs du VHC-NS5B, des inhibiteurs du VHC du nucléoside et du non nucléoside et des dérivés nucléosidiques, des inhibiteurs du virus de l'hépatite B (VHB), des inhibiteurs antiviraux du virus de l'immunodéficience humaine (HIV), des médicaments anticancéreux et anti-inflammatoires.

5. Composition pharmaceutique selon la revendication 4, dans laquelle :
- les immunomodulateurs décrits sont des interférons ou des dérivés d'interféron ;
- les inhibiteurs du VHB décrits incluent la Lamivudine, la Telbivudine, l'Adefovir Dipivoxil, l'Emtricitabine, l'Entecavir, le Tenofovir et la Telbivudine ;
- les inhibiteurs anti-HIV décrits incluent le ritonavir et/ou la ribavirine ;
- les inhibiteurs de protéase du VHC décrits incluent les substances VX-950, ZN2007, ABT-450, RG- 7227, TMC-435, MK-5172, MK- 7009, ACH-1625, GS-9256, TG2349, BMS-650032, IDX320, Yimitasvir phosphate capsule ou Seraprevir potassium ;
- l'inhibiteur de la polymérase du virus de l'hépatite C décrit comprend les substances GS-5885, TMC647055, ABT-267, BMS-791325, PPI-383 ou ALS-002158.

6. Composition pharmaceutique selon la revendication 5, dans laquelle l'interféron décrit est l'interféron pégylé.

7. Composition pharmaceutique selon la revendication 3-6 pour l'utilisation dans la préparation d'inhibiteurs antiviraux du VHC.
